# EUROPEAN PATENT APPLICATION

(11) **EP 4 374 927 A2**
(43) Date of publication of application: **29.05.2024**
(21) Application number: 24169240.9
(22) Date of filing: 09.12.2019
(51) Int. Cl.: A61P 35/00

(54) **SAPONIN CONJUGATES**

(30) Priority: 21.12.2018 NL 2022283; 10.07.2019 NL 2023468; 25.07.2019 NL 2023568
(62) Divisional of application: 19817677.8
(71) Applicant: Sapreme Technologies B.V., 3721 MA Bilthoven (NL)
(72) Inventor: POSTEL, Ruben, Utrecht (NL); FUCHS, Hendrik, Berlin (DE)
(74) Representative: Nederlandsch Octrooibureau

(57) **Abstract**

The current invention relates to an effector moiety capable of inducing an intracellular effect when present inside a mammalian cell, the effector moiety comprising a payload conjugated with at least one saponin. The invention also relates to an antibody-drug conjugate comprising the effector moiety according to the invention, or a ligand-drug conjugate comprising the effector moiety of the invention, the effector moiety comprising covalently coupled saponin. The invention also relates to a therapeutic combination comprising: (a) the effector moiety of the invention, comprising at least one saponin, and optionally a pharmaceutically acceptable excipient; and (b) an antibody-drug conjugate or a ligand-drug conjugate, and optionally a pharmaceutically acceptable excipient. Further, the invention relates to a pharmaceutical composition comprising the effector moiety of the invention or the antibody-drug conjugate of the invention or the ligand-drug conjugate of the invention, comprising at least one saponin covalently linked to the effector molecule, and optionally a pharmaceutically acceptable excipient. The invention also relates to the effector moiety of the invention or the antibody-drug conjugate of the invention or the therapeutic combination of the invention or the ligand-drug conjugate of the invention or the pharmaceutical composition of the invention, for use as a medicament. Finally, the invention also relates to the effector moiety of the invention or the antibody-drug conjugate of the invention or the therapeutic combination of the invention or the ligand-drug conjugate of the invention or the pharmaceutical composition of the invention, for use in the treatment or prevention of a cancer or an autoimmune disease.

## Description

### TECHNICAL FIELD

The current invention relates to an effector moiety capable of inducing an intracellular effect when present inside a mammalian cell, the effector moiety conjugated with at least one saponin. The current invention relates to an effector moiety capable of inducing an intracellular effect when present inside a mammalian cell, the effector moiety comprising a payload conjugated with at least one saponin. The invention also relates to an antibody-drug conjugate comprising the effector moiety according to the invention, or a ligand-drug conjugate comprising the effector moiety of the invention, the effector moiety comprising covalently coupled saponin. The invention also relates to a therapeutic combination comprising: (a) the effector moiety of the invention, comprising at least one saponin, and optionally a pharmaceutically acceptable excipient; and (b) an antibody-drug conjugate or a ligand-drug conjugate, and optionally a pharmaceutically acceptable excipient. Further, the invention relates to a pharmaceutical composition comprising the effector moiety of the invention or the antibody-drug conjugate of the invention orthe ligand-drug conjugate of the invention, comprising at least one saponin covalently linked to the effector molecule, and optionally a pharmaceutically acceptable excipient. The invention also relates to the effector moiety of the invention or the antibody-drug conjugate of the invention or the therapeutic combination of the invention or the ligand-drug conjugate of the invention or the pharmaceutical composition of the invention, for use as a medicament. Finally, the invention also relates to the effector moiety of the invention or the antibody-drug conjugate of the invention or the therapeutic combination of the invention or the ligand-drug conjugate of the invention or the pharmaceutical composition of the invention, for use in the treatment or prevention of a cancer or an autoimmune disease.

### BACKGROUND

Molecules with a therapeutic biological activity are in many occasions in theory suitable for application as an effective therapeutic drug for the treatment of a disease such as a cancer in human patients in need thereof. A typical example are small-molecule biologically active moieties. However, many if not all potential drug-like molecules and therapeutics currently used in the clinic suffer from at least one of a plethora of shortcomings and drawbacks. When administered to a human body, therapeutically active molecules may exert off-target effects, in addition to the biologically activity directed to an aspect underlying a to-be-treated disease or health problem. Such off-target effects are undesired and bear a risk for induction of health- or even life-threatening side effects of the administered molecule. It is the occurrence of such adverse events that cause many drug-like compounds and therapeutic moieties to fail phase III clinical trials or even phase IV clinical trials (post-market entry follow-up). Therefore, there is a strong desire to provide drug molecules such as small-molecule therapeutics, wherein the therapeutic effect of the drug molecule should, e.g., (1) be highly specific for a biological factor or biological process driving the disease, (2) be sufficiently safe, (3) be sufficiently efficacious, (4) be sufficiently directed to the diseased cell with little to no off-target activity on non-diseased cells, (5) have a sufficiently timely mode of action (e.g. the administered drug molecule should reach the targeted site in the human patient within a certain time frame and should remain at the targeted site for a certain time frame), and/or (6) have sufficiently long lasting therapeutic activity in the patient's body, amongst others. Unfortunately, to date, 'ideal' therapeutics with many or even all of the beneficial characteristics here above outlined, are not available to the patients, despite already long-lasting and intensive research and despite the impressive progress made in several areas of the individually addressed encountered difficulties and drawbacks.

Chemotherapy is one of the most important therapeutic options for cancer treatment. However, it is often associated with a low therapeutic window because it has no specificity towards cancer cells over dividing cells in healthy tissue. The invention of monoclonal antibodies offered the possibility of exploiting their specific binding properties as a mechanism for the targeted delivery of cytotoxic agents to cancer cells, while sparing normal cells. This can be achieved by chemical conjugation of cytotoxic effectors (also known as payloads or warheads) to antibodies, to create antibody-drug conjugates (ADCs). Typically, very potent payloads such as emtansine (DM1) are used which have a limited therapeutic index (a ratio that compares toxic dose to efficacious dose) in their unconjugated forms. The conjugation of DM1 to trastuzumab (ado-trastuzumab emtansine), also known as Kadcycla, enhances the tolerable dose of DM1 at least two-fold in monkeys. In the past few decades tremendous efforts and investments have been made to develop therapeutic ADCs. However, it remains challenging to bring ADCs into the clinic, despite promising preclinical data. The first ADC approved for clinical use was gemtuzumab ozogamicin (Mylotarg, CD33 targeted, Pfizer/Wyeth) for relapsed acute myelogenous leukemia (AML) in 2000. Mylotarg was however, withdrawn from the market at the request of the Federal Drug Administration (FDA) due to a number of concerns including its safety profile. Patients treated with Mylotarg were more often found to die than patients treated with conventional chemotherapy. Mylotarg was admitted to the market again in 2017 with a lower recommended dose, a different schedule in combination with chemotherapy or on its own, and a new patient population. To date, only five ADCs have been approved for clinical use, and meanwhile clinical development of approximately fifty-five ADCs has been halted. However, interest remains high and approximately eighty ADCs are still in clinical development in nearly six-hundred clinical trials at present.

Despite the potential to use toxic payloads that are normally not tolerated by patients, a low therapeutic index (a ratio that compares toxic dose to efficacious dose) is a major problem accounting for the discontinuance of many ADCs in clinical development, which can be caused by several mechanisms such as off-target toxicity on normal cells, development of resistance against the cytotoxic agents and premature release of drugs in the circulation. A systematic review by the FDA of ADCs found that the toxicity profiles of most ADCs could be categorized according to the payload used, but not the antibody used, suggesting that toxicity is mostly determined by premature release of the payload. Of the approximately fifty-five ADCs that were discontinued, it is estimated that at least twenty-three were due to a poor therapeutic index. For example, development of a trastuzumab tesirine conjugate (ADCT-502, HER-2 targeted, ADC therapeutics) was recently discontinued due to a narrow therapeutic index, possibly due to an on-target, off-tissue effect in pulmonary tissue which expresses considerable levels of HER2. In addition, several ADCs in phase 3 trials have been discontinued due to missing primary endpoint. For example, phase 3 trials of a depatuxizumab mafodotin conjugate (ABT-414, EGFR targeted, AbbVie) tested in patients with newly diagnosed glioblastoma, and a mirvetuximab soravtansine conjugate (IMGN853, folate receptor alpha (FRα) targeted, ImmunoGen) tested in patients with platinum-resistant ovarian cancer, were recently stopped, showing no survival benefit. It is important to note that the clinically used dose of some ADCs may not be sufficient for its full anticancer activity. For example, ado-trastuzumab emtansine has an MTD of 3.6 mg/kg in humans. In preclinical models of breast cancer, ado-trastuzumab emtansine induced tumor regression at dose levels at or above 3 mg/kg, but more potent efficacy was observed at 15 mg/kg. This suggests that at the clinically administered dose, ado-trastuzumab emtansine may not exert its maximal potential anti-tumor effect.

ADCs are mainly composed of an antibody, a cytotoxic moiety such as a payload, and a linker. Several novel strategies have been proposed and carried out in the design and development of new ADCs to overcome the existing problems, targeting each of the components of ADCs. For example, by identification and validation of adequate antigenic targets for the antibody component, by selecting antigens which have high expression levels in tumor and little or no expression in normal tissues, antigens which are present on the cell surface to be accessible to the circulating ADCs, and antigens which allows internalizing of ADCs into the cell after binding; and alternative mechanisms of activity; design and optimize linkers which enhance the solubility and the drug-to-antibody ratio (DAR) of ADCs and overcome resistance induced by proteins that can transport the chemotherapeutic agent out of the cells; enhance the DAR ratio by inclusion of more payloads, select and optimize antibodies to improve antibody homogeneity and developability. In addition to the technological development of ADCs, new clinical and translational strategies are also being deployed to maximize the therapeutic index, such as, change dosing schedules through fractionated dosing; perform biodistribution studies; include biomarkers to optimize patient selection, to capture response signals early and monitor the duration and depth of response, and to inform combination studies.

An example of ADCs with clinical potential are those ADCs such as brentuximab vedotin, inotuzumab ozogamicin, moxetumomab pasudotox, and polatuzumab vedotin, which are evaluated as a treatment option for lymphoid malignancies and multiple myeloma. Polatuzumab vedotin, binding to CD79b on (malignant) B-cells, and pinatuzumab vedotin, binding to CD22, are tested in clinical trials wherein the ADCs each were combined with co-administered rituximab, a monoclonal antibody binding to CD20 and not provided with a payload [B. Yu and D. Liu, Antibody-drug conjugates in clinical trials for lymphoid malignancies and multiple myeloma; Journal of Hematology & Oncology (2019) 12:94]*.* Combinations of monoclonal antibodies such as these examples are yet a further approach and attempt to arrive at the 'magic bullet' which combines many or even all of the aforementioned desired characteristics of ADCs.

Meanwhile in the past few decades, nucleic acid-based therapeutics are under development. Therapeutic nucleic acids can be based on deoxyribonucleic acid (DNA) or ribonucleic acid (RNA), Anti-sense oligonucleotides (ASOs, AONs), and short interfering RNAs (siRNAs), MicroRNAs, and DNA and RNA aptamers, for approaches such as gene therapy, RNA interference (RNAi). Many of them share the same fundamental basis of action by inhibition of either DNA or RNA expression, thereby preventing expression of disease-related abnormal proteins. The largest number of clinical trials is being carried out in the field of gene therapy, with almost 2600 ongoing or completed clinical trials worldwide but with only about 4% entering phase 3. This is followed by clinical trials with ASOs. Similarly to ADCs, despite the large number of techniques being explored, therapeutic nucleic acids share two major issues during clinical development: delivery into cells and off-target effects. For instance, ASOs such as peptide nucleic acid (PNA), phosphoramidate morpholino oligomer (PMO), locked nucleic acid (LNA) and bridged nucleic acid (BNA), are being investigated as an attractive strategy to inhibit specifically target genes and especially those genes that are difficult to target with small molecules inhibitors or neutralizing antibodies. Currently, the efficacy of different ASOs is being studied in many neurodegenerative diseases such as Huntington's disease, Parkinson's disease, Alzheimer's disease, and amyotrophic lateral sclerosis and also in several cancer stages. The application of ASOs as potential therapeutic agents requires safe and effective methods for their delivery to the cytoplasm and/or nucleus of the target cells and tissues. Although the clinical relevance of ASOs has been demonstrated, inefficient cellular uptake, both in vitro and in vivo, limit the efficacy of ASOs and has been a barrier to therapeutic development. Cellular uptake can be < 2% of the dose resulting in too low ASO concentration at the active site for an effective and sustained outcome. This consequently requires an increase of the administered dose which induces off-target effects. Most common side-effects are activation of the complement cascade, the inhibition of the clotting cascade and toll-like receptor mediated stimulation of the immune system.

Chemotherapeutics are most commonly small molecules, however, their efficacy is hampered by the severe off-target side toxicity, as well as their poor solubility, rapid clearance and limited tumor exposure. Scaffold-small-molecule drug conjugates such as polymer-drug conjugates (PDCs) are macromolecular constructs with pharmacologically activity, which comprises one or more molecules of a small-molecule drug bound to a carrier scaffold (e.g. polyethylene glycol (PEG)).

Such conjugate principle has attracted much attention and has been under investigation for several decades. The majority of conjugates of small-molecule drugs under pre-clinical or clinical development are for oncological indications. However, up-to-date only one drug not related to cancer has been approved (Movantik, a PEG oligomer conjugate of opioid antagonist naloxone, AstraZeneca) for opioid-induced constipation in patients with chronic pain in 2014, which is a non-oncology indication. Translating application of drug-scaffold conjugates into treatment of human subjects provides little clinical success so far. For example, PK1 (N-(2-hydroxypropyl)methacrylamide (HPMA) copolymer doxorubicin; development by Pharmacia, Pfizer) showed great anti-cancer activity in both solid tumors and leukemia in murine models, and was under clinical investigation for oncological indications. Despite that it demonstrated significant reduction of nonspecific toxicity and improved pharmacokinetics in man, improvements in anticancer efficacy turned out to be marginal in patients, and as a consequence further development of PK1 was discontinued.

The failure of scaffold-small-molecule drug conjugates is at least partially attributed to its poor accumulation at the tumor site. For example, while in murine models PK1 showed 45-250 times higher accumulation in the tumor than in healthy tissues (liver, kidney, lung, spleen, and heart), accumulation in tumor was only observed in a small subset of patients in the clinical trial.

A potential solution to the aforementioned problems is application of nanoparticle systems for drug delivery such as liposomes. Liposomes are sphere-shaped vesicles consisting of one or more phospholipid bilayers, which are spontaneously formed when phospholipids are dispersed in water. The amphiphilicity characteristics of the phospholipids provide it with the properties of self-assembly, emulsifying and wetting characteristics, and these properties can be employed in the design of new drugs and new drug delivery systems. Drug encapsulated in a liposomal delivery system may convey several advantages over a direct administration of the drug, such as an improvement and control over pharmacokinetics and pharmacodynamics, tissue targeting property, decreased toxicity and enhanced drug activity. An example of such success is liposome-encapsulated form of a small molecule chemotherapy agent doxorubicin (Doxil: a pegylated liposome-encapsulated form of doxorubicin; Myocet: a non-pegylated liposomal doxorubicin), which have been approved for clinical use.

Therefore, a solution still needs to be found that allows for drug therapies such as anti-tumor therapies, applicable for non-systemic use when desired, wherein the drug has for example an acceptable safety profile, little off-target activity, sufficient efficacy, sufficiently low clearance rate from the patient's body, etc.

### SUMMARY

For an embodiment of the present invention, it is a first goal to provide an improved biologically active compound or composition comprising such improved biologically active compound.

It is one of several objectives of embodiments of the current invention to provide a solution to the problem of non-specificity, encountered when administering small-molecule therapeutically active compounds to a human patient in need thereof. It is one of several objectives of embodiments of the current invention to provide a solution to the problem of drugs with non-optimal specificity for a biological factor or biological process driving a disease. It is one of several objectives of embodiments of the current invention to provide a solution to the problem of insufficient safety characteristics of current drugs, when administered to human patients in need thereof. It is one of several objectives of embodiments of the current invention to provide a solution to the problem of current drugs being less efficacious than desired, when administered to human patients in need thereof. It is one of several objectives of embodiments of the current invention to provide a solution to the problem of current drugs being not sufficiently directed to the diseased cell with little to no off-target activity on non-diseased cells, when administered to human patients in need thereof. It is one of several objectives of embodiments of the current invention to provide a solution to the problem that current drugs do not have a sufficiently timely mode of action (e.g. the administered drug molecule should reach the targeted site in the human patient within a certain time frame and should remain at the targeted site for a certain time frame), when administered to human patients in need thereof. It is one of several objectives of embodiments of the current invention to provide a solution to the problem that current drugs have not sufficiently long lasting therapeutic activity in the patient's body, when administered to human patients in need thereof.

At least one of the above objectives of embodiments of the invention is achieved by providing an effector moiety capable of inducing an intracellular effect when present inside a mammalian cell, the effector moiety conjugated with at least one saponin, wherein the at least one saponin is covalently bound to the effector moiety via at least one linker, or is covalently bound directly to said effector moiety.

The present invention will be described with respect to particular embodiments but the invention is not limited thereto but only by the claims. The embodiments of the invention described herein can operate in combination and cooperation, unless specified otherwise.

An aspect of the invention relates to an effector moiety capable of inducing an intracellular effect when present inside a mammalian cell, the effector moiety conjugated with at least one saponin, wherein the at least one saponin is covalently bound to the effector moiety via at least one linker, or is covalently bound directly to said effector moiety.

An aspect of the invention relates to an antibody-drug conjugate comprising the effector moiety according to the invention, or a ligand-drug conjugate comprising the effector moiety of the invention, the effector moiety comprising covalently coupled saponin. The effector moiety of the invention is a conjugate comprising at least one saponin and at least one effector molecule, covalently coupled to each other, either directly, or via at least one linker, optionally comprising a cleavable linker, and optionally via an oligomeric or polymeric scaffold to which the at least one saponin and/or the at least one effector moiety are covalently bound.

An aspect of the invention relates to a therapeutic combination comprising: (a) the effector moiety of the invention, comprising at least one saponin, and optionally a pharmaceutically acceptable excipient; and (b) an antibody-drug conjugate or a ligand-drug conjugate, and optionally a pharmaceutically acceptable excipient.

An aspect of the invention relates to a pharmaceutical composition comprising the effector moiety of the invention or the antibody-drug conjugate of the invention or the ligand-drug conjugate of the invention, comprising at least one saponin covalently linked to the effector molecule, and optionally a pharmaceutically acceptable excipient.

An aspect of the invention relates to the effector moiety of the invention or the antibody-drug conjugate of the invention or the therapeutic combination of the invention or the ligand-drug conjugate of the invention or the pharmaceutical composition of the invention, for use as a medicament.

An aspect of the invention relates to the effector moiety of the invention or the antibody-drug conjugate of the invention or the therapeutic combination of the invention or the ligand-drug conjugate of the invention orthe pharmaceutical composition of the invention, for use in the treatment or prevention of a cancer or an autoimmune disease.

### DEFINITIONS

The term "linker" has its regular scientific meaning, and here refers to a chemical moiety or a linear stretch of amino-acid residues complexed through peptide bonds, which attaches a molecule or an atom to another molecule, e.g. to a ligand or to an effector molecule or to a scaffold. Typically, the linker comprises a chain of atoms linked by chemical bonds. Any linker molecule or linker technology known in the art can be used in the present disclosure. Where indicated, the linker is a linker for covalently binding of molecules through a chemical group on such a molecule suitable for forming a covalent linkage or bond with the linker. The linker may be a non-cleavable linker, e.g., the linker is stable in physiological conditions. The linker may be a cleavable linker, e.g. a linker that is cleavable, in the presence of an enzyme or at a particular pH range or value, or under physiological conditions such as intracellular conditions in the endosomes such as the late endosomes and the lysosomes of mammalian cells such as human cells. Exemplary linkers that can be used in the context of the present disclosure includes, but is not limited to, N-ε-maleimidocaproic acid hydrazide (EMCH), succinimidyl 3-(2-pyridyldithio)propionate or 3-(2-Pyridyldithio)propionic acid N-hydroxysuccinimide ester (SPDP), and 1-[Bis(dimethylamino)methylene]-1H-1,2,3-triazolo[4,5-b]pyridinium 3-oxid hexafluorophosphate (HATU).

The term "tri-functional linker" has its regular scientific meaning, and here refers to a linker which attaches three molecules via a chemical group on each of the three molecules. The skilled person is able to design such tri-functional linkers, based on the present disclosure and the common general knowledge. Such tri-functional linker can exhibit, for instance, a maleimido group that can be used for conjugation to targeting ligands that exhibit thiol groups to perform a thiol-ene reaction. In addition, the tri-functional linker could exhibit a dibenzocyclooctyne (DBCO) group to perform the so-called strain-promoted alkyne-azide cycloaddition (SPAAC, click chemistry) with an azido bearing saponin. Finally, the tri-functional linker could obtain a third functional group such as a trans-cyclooctene (TCO) group to perform the so-called inverse electron demand Diels-Alder (IEDDA) reaction with a tetrazine (Tz) bearing effector molecule. The skilled person will appreciate that the chemical groups of thetri-functional linker can be all three the same, or different, orthe linker may comprise two of the same chemical groups for linking a molecule to the tri-functional linker. The formed bonds between the tri-functional linker can be covalent or non-covalent, and covalent bonds are preferred. The formed bonds between the tri-functional linker and the one or two or three bound molecules via respective chemical groups, can be cleavable (labile) bonds, such as cleavable under acidic conditions inside cells such as endosomes and lysosomes of mammalian cells such as human cells, or can be non-cleavable bonds. Of course, the tri-functional linker may encompass one or two chemical groups for forming covalent bonds while the further two or one chemical group(s), respectively, are/is for forming a non-covalent bond. Of course, the tri-functional linker may encompass one or two chemical groups for forming cleavable bonds while the further two or one chemical group(s), respectively, are/is for forming a non-cleavable bond.

The term "cleavable", such as used in the term "cleavable linker" or "cleavable bond" has its regular scientific meaning, and here refers to being subject to cleavage under acidic conditions, reductive conditions, enzymatic conditions or light-induced conditions. For example, a cleavable linker may be subject to cleavage under acidic conditions, preferably said cleavable linker is subject to cleavage in vivo under acidic conditions as present in endosomes and/or lysosomes of mammalian cells, preferably human cells, preferably at pH 4.0 - 6.5, and more preferably at pH ≤ 5.5. As another example, a cleavable linker may be subject to cleavage by an enzyme, e.g. by cathepsin. Furthermore, an example of a covalent bond cleavable under reductive conditions is a disulphide bond.

The terms "oligomer" and "polymer" in the context of an oligomeric or polymeric scaffold has its regular scientific meaning. A polymer here refers to a substance which has a molecular structure built up chiefly or completely from a large number of equal or similar units bonded together; an oligomer here refers to a polymer whose molecules consist of relatively few repeating units. For example, a structure comprising 5-10 or less equal or similar units, may be called an oligomeric structure, whereas a structure comprising 10-50 monomeric units or more may be called a polymeric structure, whereas a structure of 10 monomeric units may be called either oligomeric or polymeric.

The term "binding site" has its regular scientific meaning, and here refers to a region or an epitope on a molecule, e.g. a protein, DNA or RNA, to which another molecule can bind.

The term "scaffold" has its regular scientific meaning, and here refers to an oligomeric or polymeric template or a carrier or a base (base molecule or base structure), to which one or more molecules, e.g. ligand molecule, saponin of the invention, glycoside, effector molecule, can be covalently bound, either directly, or via a linker, such as a cleavable linker. A scaffold may have a structurally ordered formation such as a polymer, oligomer, dendrimer, dendronized polymer, or dendronized oligomer or have an assembled polymeric structure such as a hydrogel, microgel, nanogel, stabilized polymeric micelle or liposome, but excludes structures that are composed of non-covalent assemblies of monomers such as cholesterol/phospholipid mixtures. A scaffold may comprise a polymeric or oligomeric structure, such as poly- or oligo(amines), e.g., polyethylenimine and poly(amidoamine); or structures such as polyethylene glycol, poly- or oligo(esters), such as poly(lactids), poly(lactams), polylactide-co-glycolide copolymers; or poly(dextrin), poly- or oligosaccharides, such as cyclodextrin or polydextrose; or structures such as natural and/or artificial poly- or oligoamino acids such as poly-lysine or a peptide or a protein, DNA oligo- or polymers, stabilized RNA polymers or PNA (peptide nucleic acid) polymers. Preferably, the polymeric or oligomeric structures are biocompatible, wherein biocompatible means that the polymeric or oligomeric structure does not show substantial acute or chronic toxicity in organisms and can be either excreted as it is or fully degraded to excretable and/or physiological compounds by the body's metabolism.

The term "ligand" has its regular scientific meaning, and here refers to any molecule or molecules which may selectively bind to a target cell-surface molecule or target cell-surface receptor expressed at target cells, e.g. target cancer cells or target auto-immune cells. The ligand may bind to an epitope comprised by receptors or other antigens on the target cells. Preferably, the cell-binding ligands are antibodies.

The term "antibody" as used herein is used in the broadest sense, which may refer to an immunoglobulin (Ig) defined as a protein belonging to the class IgG, IgM, IgE, IgA, or IgD (or any subclass thereof), or a functional binding fragment or binding domain of an immunoglobulin. In the context of the present invention, a "binding fragment" or a "binding domain" of an immunoglobulin is defined as antigen-binding fragment or -domain or other derivative of a parental immunoglobulin that essentially maintains the antigen binding activity of such parental immunoglobulin. Functional fragments and functional domains are antibodies in the sense of the present invention even if their affinity to the antigen is lower than that of the parental immunoglobulin. "Functional fragments and -domains" in accordance with the invention include, but are not limited to, F(ab')2 fragments, Fab' fragments, Fab fragments, scFv, dsFv, single-domain antibody (sdAb), monovalent IgG, scFv-Fc, reduced IgG (rlgG), minibody, diabodies, triabodies, tetrabodies, Fc fusion proteins, nanobodies, variable V domains such as VHH, Vh, and other types of antigen recognizing immunoglobulin fragments and domains. The fragments and domains may be engineered to minimize or completely remove the intermolecular disulphide interactions that occur between the CH1 and CL domains. Functional fragment and -domains offer the advantage of greater tumor penetration because of their smaller size. In addition, the functional fragment or -domain can be more evenly distributed throughout the tumor mass as compared to whole immunoglobulin.

The antibodies (immunoglobulins) of the present invention may be bi- or multifunctional. For example, a bifunctional antibody has one arm having a specificity for one receptor or antigen, while the other arm recognizes a different receptor or antigen. Alternatively, each arm of the bifunctional antibody may have specificity for a different epitope of the same receptor or antigen of the target cell.

The antibodies (immunoglobulins) of the present invention may be, but are not limited to, polyclonal antibodies, monoclonal antibodies, human antibodies, humanized antibodies, chimeric antibodies, resurfaced antibodies, anti-idiotypic antibodies, mouse antibodies, rat antibodies, rat/mouse hybrid antibodies, llama antibodies, llama heavy-chain only antibodies, heavy-chain only antibodies, and veterinary antibodies. Preferably, the antibody (immunoglobulin) of the present invention is a monoclonal antibody. The resurfaced, chimeric, humanized and fully human antibodies are also more preferred because they are less likely to cause immunogenicity in humans. The antibodies of the ADC of the present invention preferably specifically binds to an antigen expressed on the surface of a cancer cell, an autoimmune cell, a diseased cell, an aberrant cell, while leaving any healthy cell essentially unaltered (e.g. by not binding to such normal cell, or by binding to a lesser extent in number and/or affinity to such healthy cell).

Specific antibodies that can be used for the ADCs of the present invention include, but are not limited to, anti-HER2 monoclonal antibody such as trastuzumab and pertuzumab, anti-CD20 monoclonal antibody such as rituximab, ofatumumab, tositumomab and ibritumomab, anti-CA125 monoclonal antibody such as oregovomab, anti-EpCAM (17-1A) monoclonal antibody such as edrecolomab, anti-EGFR monoclonal antibody such as cetuximab, panitumumab and nimotuzumab, anti-CD30 monoclonal antibody such brentuximab, anti-CD33 monoclonal antibody such as gemtuzumab and huMy9-6, anti-vascular integrin alpha-v beta-3 monoclonal antibody such as etaracizumab, anti-CD52 monoclonal antibody such as alemtuzumab, anti-CD22 monoclonal antibody such as epratuzumab, anti-CEA monoclonal antibody such as labetuzumab, anti-CD44v6 monoclonal antibody such as bivatuzumab, anti-FAP monoclonal antibody such as sibrotuzumab, anti-CD19 monoclonal antibody such as huB4, anti-CanAg monoclonal antibody such as huC242, anti-CD56 monoclonal antibody such huN901, anti-CD38 monoclonal antibody such as daratumumab, anti-CA6 monoclonal antibody such as DS6, anti-IGF-IR monoclonal antibody such as cixutumumab and 3B7, anti-integrin monoclonal antibody such as CNTO 95, and anti-syndecan-1 monoclonal antibody such as B-B4. An embodiment is the protein-toxin conjugate of Stemline: ELZONRIS^{™} (tagraxofusp, SL-401) - ELZONRIS is a novel targeted therapy directed to the interleukin-3 (IL-3) receptor-a (CD123), a target present on a wide range of malignancies.

Any other molecules than antibodies that bind to a cell receptor or antigen of a target cell can also be used as the cell-binding ligand for the ligand-drug conjugates of the present invention and the ligands provided with covalently bound saponin according to the invention. These ligands include, but are not limited to, proteins, polypeptides, peptides, small molecules. Examples of these non-antibody ligands are interferons (e.g. IFN-α, IFN-β, and IFN-y), transferrins, lectins, epidermal growth factors (EGF) and EGF-like domains, gastrin-releasing peptides (GRP), platelet-derived growth factors (PDGF), transforming growth factors (TGF), vaccinia growth factor (VGF), insulin and insulin-like growth factors (IGF, e.g. IGF-1 and IGF-2), other suitable hormones such as thyrotropin releasing hormones (TRH), melanocyte-stimulating hormones (MSH), steroid hormones (e.g. estrogen and androgen), somatostatin, lymphokines (e.g. IL-2, IL-3, IL-4, and IL-6), colony-stimulating factors (CSF, e.g. G-CSF, M-CSF and GM-CSF), bombesin, gastrin, Arg-Gly-Asp or RGD, aptamers (e.g. AS-1411, GBI-10, RNA aptamers against HIV glycoprotein), small molecules (e.g. folate, anisamide phenylboronic acid), vitamins (e.g., vitamin D), carbohydrates (e.g. hyaluronic acid, galactose).

An "effector molecule" or "effector moiety" or "payload" has its regular scientific meaning and in the context of this invention is any substance that affects the metabolism of a cell by interaction with an intracellular effector molecule target, wherein this effector molecule target is any molecule or structure inside cells excluding the lumen of compartments and vesicles of the endocytic and recycling pathway but including the membranes of these compartments and vesicles. Said structures inside cells thus include the nucleus, mitochondria, chloroplasts, endoplasmic reticulum, Golgi apparatus, other transport vesicles, the inner part of the plasma membrane and the cytosol.

The effector molecule or -moiety is a pharmaceutically active substance, such as a toxin such as a proteinaceous toxin, a drug, a polypeptide or a polynucleotide. A pharmaceutically active substance in this invention is an effector molecule or -moiety that is used to achieve a beneficial outcome in an organism, preferably a vertebrate, more preferably a mammal such as non-human subjects or a human being/subject. Benefits include diagnosis, prognosis, treatment, cure and prevention (prophylaxis) of diseases and/or symptoms and/or health problems. The pharmaceutically active substance may also lead to undesired and sometimes even harmful side effects (adverse events such as observed during clinical trials). In this case, pros and cons must be weighed to decide whether the pharmaceutically active substance is suitable in the particular case. If the effect of the pharmaceutically active substance inside a cell is predominantly beneficial for the organism as a whole, the cell is called a target cell. If the effect inside a cell is predominantly harmful for the organism as a whole, the cell is called an off-target cell. In artificial systems such as cell cultures and bioreactors, target cells and off-target cells depend on the purpose and are defined by the user. Examples of effector molecules and -moieties are a drug, a toxin, a polypeptide (such as an enzyme), a polynucleotide (including polypeptides and polynucleotides that comprise non-natural amino acids or nucleic acids), and any combination thereof.

An effector molecule or effector moiety that is a drug may include, but not limited to, anti-cancer agents, anti-inflammatory agents, and anti-infective (e.g., anti-fungal, antibacterial, anti-parasitic, antiviral) agents. Preferably, the drug molecule of the present invention is an anti-cancer agent or an anti-auto-immune agent. Suitable anti-cancer agents include, but are not limited to, alkylating agents, antimetabolites, spindle poison plant alkaloids, cytotoxic/antitumor antibiotics, topoisomerase inhibitors, photosensitizers, and kinase inhibitors. Also included in the definition of "anti-cancer agent" are: e.g. (i) anti-hormonal agents that act to regulate or inhibit hormone action on tumors such as anti-estrogens and selective estrogen receptor modulators; (ii) aromatase inhibitors that inhibit the enzyme aromatase, which regulates estrogen production in the adrenal glands; (iii) anti-androgens; (iv) protein kinase inhibitors; (v) lipid kinase inhibitors; (vi) antisense oligonucleotides, particularly those which inhibit expression of genes in signaling pathways implicated in aberrant cell proliferation; (vii) ribozymes such as VEGF expression inhibitors and HER2 expression inhibitors; (viii) vaccines such as gene therapy vaccines; topoisomerase 1 inhibitors; (ix) anti-angiogenic agents; and pharmaceutically acceptable salts, acids, solvates and derivatives of any of the above.

An effector molecule or-moiety that is a toxin may include, but is not limited to, proteinaceous toxins (e.g. bacterial-derived toxins, and plant-derived toxins), toxins targeting tubulin filaments, toxins targeting DNA, toxins targeting RNA. Examples of proteinaceous toxins are saporin, dianthin, ricin, modeccin, abrin, volkensin, viscumin, shiga toxin, shiga-like toxin, pseudomonas exotoxin (PE, also known as exotoxin A), diphtheria toxin (DT), and cholera toxin. Examples of tubulin filaments-targeting toxins are maytansinoids (e.g. DM1 and DM4), auristatins (e.g. Monomethyl auristatin E (MMAE) and Monomethyl auristatin F (MMAF)), toxoids, tubulysins, cryptophycins, rhizoxin. Examples of DNA-targeting toxins are calicheamicins: N-Acetyl- γ-calicheamicin, CC-1065 analogs, duocarmycins, doxorubicin, methotrexate, benzodiazepines, camptothecin analogues, and anthracyclines. Examples of DNA-targeting toxins are amanitins, spliceostatins, and thailanstatins. A toxin, as used in this invention, is defined as a pharmaceutically active substance that is able to kill or inactivate a cell. Preferably, a targeted toxin is a toxin that is only, or at least predominantly, toxic for target cells but not for off-target cells. The net effect of the targeted toxin is preferably beneficial for the organism as a whole.

An effector molecule or -moiety that is a polypeptide may be, e.g., a polypeptide that recover a lost function, such as for instance enzyme replacement, gene regulating functions, or a toxin. Examples of polypeptides as effector molecules are, e.g., Cas9; toxins (e.g. saporin, dianthin, gelonin, (de)bouganin, agrostin, ricin (toxin A chain); pokeweed antiviral protein, apoptin, diphtheria toxin, pseudomonas exotoxin) metabolic enzymes (e.g. argininosuccinate lyase, argininosuccinate synthetase), enzymes of the coagulation cascade, repairing enzymes; enzymes for cell signaling; cell cycle regulation factors; gene regulating factors (transcription factors such as NF-κB or gene repressors such as methionine repressor).

An effector molecule or an effector moiety that is a polynucleotide may, e.g., be a polynucleotide that comprises coding information, such as a gene or an open reading frame encoding a protein. It may also comprise regulatory information, e.g. promotor or regulatory element binding regions, or sequences coding for micro RNAs. Such polynucleotide may comprise natural and artificial nucleic acids. Artificial nucleic acids include, e.g. peptide nucleic acid (PNA), Morpholino and locked nucleic acid (LNA), as well as glycol nucleic acid (GNA) and threose nucleic acid (TNA). Each of these is distinguished from naturally occurring DNA or RNA by changes to the backbone of the molecule. Examples of nucleotides as effector molecules are, but not limited to, e.g., DNA: single stranded DNA (e.g. DNA for adenine phosphoribosyltransferase); linear double stranded DNA (e.g. clotting factor IX gene); circular double stranded DNA (e.g. plasmids); RNA: mRNA (e.g. TAL effector molecule nucleases), tRNA, rRNA, siRNA, miRNA, antisense RNA; anti-sense oligonucleotides (ASOs, AONs e.g. PNA, PMO, LNA and BNA).

The term "proteinaceous", used in e.g. "proteinaceous molecule" and "proteinaceous toxin", are molecules and toxins comprising at least a string of amino acid residues that can be obtained as an expression product from a single mRNA. Such a molecule or toxin may further comprise any post-translational modifications, a carbohydrate such as an N- or O-linked carbohydrate, disulphide bonds, phosphorylations, sulphatations, etc., as a result of any post-translational modification, and/or may further comprise any other modification such as those resulting from chemical modifications (e.g., linking of effector moieties, saponin, scaffolds, ligands, etc., either directly to e.g. an amino-acid side chain, or via at least one linker (covalently) bound to the molecule for chemically modifying the proteinaceous molecule, and chemically bound (covalently) to the proteinaceous molecule). The term "proteinaceous" also encompasses and includes assemblies of such molecules, e.g. homodimers, heterotrimers, heterohexamers or complex assemblies such as ribosomes.

The terms "specific" and "specifically", in the context of for example "specific binding" and "receptor or molecular target specifically present or expressed at the surface of a tumor cell" and the like, have their normal scientific meaning known in the art, and here refer to e.g. a binding interaction of a first molecule with a second molecule which occurs with a higher affinity relative to any putative binding of the first molecule to a further molecule different from the second molecule, or e.g. to the expression or expression to a higher extent when e.g. the number of receptors or molecular targets is considered, of a cell-surface receptor or molecular target on the surface of a first type of cell such as a tumor cell, autoimmune cell, diseased cell, aberrant cell, relative to the extent of expression of the same receptor or molecular target at a second type of cell such as a healthy cell, etc., wherein expression at the second type of cell can be fully absent or very low, relative to any extent of expression on the tumor cell, etc. Furthermore, the term "specific", for example in "specific binding", has its normal scientific meaning known in the art, and here has the meaning of indicating a molecule that can have an interaction with another molecule with higher binding affinity than background interactions between molecules. Similarly, the term "specificity" refers to an interaction, for example, between two molecules or between a cell and a molecule, which has higher binding affinity than background interactions between molecules. Binding molecules such as immunoglobulins bind via their binding site such as immunoglobulin variable regions of the immunoglobulin, to binding sites on molecules, such as epitopes, cell-surface receptors, etc., with a higher binding affinity than background interactions between molecules. In the context of the invention, background interactions are typically interactions with an affinity lower than a K_{D} of 10E-4 M. Similarly, "specific binding domains" are domains that preferentially bind to binding sites on molecules, such as epitopes, cell-surface receptors, etc., with a higher binding affinity than background interactions between molecules. In the context of the invention, "background interactions" are typically interactions with an affinity lower than a K_{D} of 10E-4 M. Preferably, specific binding domains bind with an affinity higher than a K_{D} of about 10E-5 M.

The term "binding" is defined as interactions between molecules that can be distinguished from background interactions.

Throughout the specification, the term "fragment" refers to an amino acid sequence which is part of a protein domain or which builds up an intact protein domain. Binding fragments according to the invention must have binding specificity for the respective target such as a cell-surface receptor, e.g. on the surface of a diseased cell such as a tumor cell.

The term "ADC" or "antibody-drug conjugate" has its regular scientific meaning known to the skilled person, and here refers to a class of biopharmaceutical drugs designed as a targeted therapy for treating e.g. cancer. Unlike chemotherapy, ADCs are intended to target and kill tumor cells while sparing healthy cells. ADCs are composed of an antibody linked to a biologically active cytotoxic (anticancer) payload or drug. ADCs combine the targeting capabilities of monoclonal antibodies with the cancer-killing ability of cytotoxic drugs. They are designed with the intention to discriminate between healthy cells and diseased tissue such as tumor cells in a tumor.

The term "Saponinum album" has its normal meaning and here refers to a mixture of saponins produced by Merck KGaA (Darmstadt, Germany) containing saponins from *Gypsophila paniculata* and *Gypsophila arostii*, containing SA1657 and mainly SA1641.

The term "Quillajasaponin" has its normal meaning and here refers to the saponin fraction of Quillaja saponaria and thus the source for all other QS saponins, mainly containing QS-18 and QS-21.

"QS-21" or "QS21" has its regular scientific meaning and here refers to a mixture of QS-21 A-apio (-63%), QS-21 A-xylo (-32%), QS-21 B-apio (-3.3%), and QS-21 B-xylo (-1.7%).

Similarly, "QS-21A" has its regular scientific meaning and here refers to a mixture of QS-21 A-apio (-65%) and QS-21 A-xylo (-35%).

Similarly, "QS-21B" has its regular scientific meaning and here refers to a mixture of QS-21 B-apio (-65%) and QS-21 B-xylo (-35%).

The term "Quil-A" refers to a commercially available semi-purified extract from Quillaja saponaria and contains variable quantities of more than 50 distinct saponins, many of which incorporate the triterpene-trisaccharide substructure Gal-(1→2)-[Xyl-(1→3)]-GlcA- at the C-3beta-OH group found in QS-7, QS-17, QS18, and QS-21. The saponins found in Quil-A are listed in van Setten (1995), Table *2 [*Dirk C. van Setten, Gerrit van de Werken, Gijsbert Zomer and Gideon F. A. Kersten, Glycosyl Compositions and Structural Characteristics of the Potential Immuno-adjuvant Active Saponins in the Quillaja saponaria Molina Extract Quil A, RAPID COMMUNICATIONS IN MASS SPECTROMETRY, VOL. 9,660-666 (1995*)].* Quil-A and also Quillajasaponin are fractions of saponins from Quillaja saponaria and both contain a large variety of different saponins with largely overlapping content. The two fractions differ in their specific composition as the two fractions are gained by different purification procedures.

The term "QS1861" and the term "QS1862" refer to QS-7 and QS-7 api. QS1861 has a molecular mass of 1861 Dalton, QS1862 has a molecular mass of 1862 Dalton. QS1862 is described in Fleck et al. (2019) in Table 1, row no. 28 [Juliane Deise Fleck, Andresa Heemann Betti, Francini Pereira da Silva, Eduardo Artur Troian, Cristina Olivaro, Fernando Ferreira and Simone Gasparin Verza, Saponins from Quillaja saponaria and Quillaja brasiliensis: Particular Chemical Characteristics and Biological Activities, Molecules 2019, 24, 171; doi:10.3390/molecules24010171]. The described structure is the api-variant QS1862 of QS-7. The molecular mass is 1862 Dalton as this mass is the formal mass including proton at the glucuronic acid. At neutral pH, the molecule is deprotonated. When measuring in mass spectrometry in negative ion mode, the measured mass is 1861 Dalton.

The terms first, second, third and the like in the description and in the claims, are used for distinguishing between similar elements and not necessarily for describing a sequential or chronological order. The terms are interchangeable under appropriate circumstances. The embodiments of the invention can operate in other sequences than described or illustrated herein.

Furthermore, the various embodiments, although referred to as "preferred" or "e.g." or "for example" or "in particular" are to be construed as exemplary manners in which the invention may be implemented rather than as limiting the scope of the invention.

The term "comprising", used in the claims, should not be interpreted as being restricted to the elements or steps listed thereafter; it does not exclude other elements or steps. It needs to be interpreted as specifying the presence of the stated features, integers, steps or components as referred to, but does not preclude the presence or addition of one or more other features, integers, steps or components, or groups thereof. Thus, the scope of the expression "a pharmaceutical composition comprising A and B" should not be limited to a pharmaceutical composition consisting only of components A and B, rather with respect to the present invention, the only enumerated components of the pharmaceutical composition are A and B, and further the claim should be interpreted as including equivalents of those components. Similarly, the scope of the expression "a method comprising step A and step B" should not be limited to a method consisting only of steps A and B, rather with respect to the present invention, the only enumerated steps of the method are A and B, and further the claim should be interpreted as including equivalents of those steps.

In addition, reference to a feature by the indefinite article "a" or "an" does not exclude the possibility that more than one of the features such as for example a component, excipient, saponin, etc. are present, unless the context clearly requires that there is one and only one of the features. The indefinite article "a" or "an" thus usually means "at least one".

### BRIEF DESCRIPTION OF DRAWINGS

Fig1: HSP27BNA gene silencing activity of HSP27BNA, HSP27BNA-SO1861 and HSP27BNA-dendron-(SO1861)⁴ in A431 cancer cell lines.
Fig 2: **A.** MALDI-TOF-MS spectrum of BSA-SO1861. **B.** MALDI-TOF-MS spectrum of BSA.
Fig 3: **A.** H-NMR spectrum of SO1861. **B.** H-NMR spectrum of SO1861-EMCH ((EMCH = N-ε-maleimidocaproic acid hydrazide) conjugate.
Fig 4: **A.** MALDI-TOF-MS spectrum of SO1861-EMCH conjugate. **B.** MALDI-TOF-MS spectrum of SO1861-EMCH-mercaptoethanol conjugate.
Fig 5: **A.** synthesis scheme of SO1861-EMCH. **B.** MALDI-TOF-MS spectrum of SO1861 in negative reflector mode. TFA: trifluoroacetic acid, r.t: room temperature, h: hours, and MW: molecular weight. **C.** MALDI-TOF-MS spectrum of SO1861-EMCH in negative reflector mode. TFA: trifluoroacetic acid, r.t: room temperature, h: hours, and MW: molecular weight.
Fig 6: Reaction scheme of of Dendron(-L-SO1861)⁴ synthesis.
Fig 7: Reaction scheme of Dendron(-L-SO1861)⁸ synthesis.
Fig 8: **A.** MALDI-TOF-MS spectrum of Cy3-PAMAM. **B.** MALDI-TOF-MS spectrum of Cy3-PAMAM-SO1861 with 5 SO1861 attached per PAMAM. **C.** MALDI-TOF-MS spectrum of Cy3-PAMAM-SO1861 with 13 SO1861 attached per PAMAM. **D.** MALDI-TOF-MS spectrum of Cy3-PAMAM-SO1861 51 SO1861 attached per PAMAM.
Fig 9: **A.** MALDI-TOF-MS spectrum of Cy3-PAMAM-SO1861 with 5 equivalents feed SO1861-EMCH. **B.** MALDI-TOF-MS spectrum of Cy3-PAMAM-SO1861 with 30 equivalents feed SO1861-EMCH.
Fig 10: MALDI-TOF-MS spectra of Cy3-PAMAM-NC-SO1861 (NC = stable bond ("non-cleavable").
Fig 11: **A.** Reaction scheme of the thiolation of PAMAM using the thiolation reagent 2-iminothiolane. **B.** MALDI-TOF-MS spectrum of native PAMAM. **C.** MALDI-TOF-MS spectrum of thiolated PAMAM-(SH)₁₆. **D.** MALDI-TOF-MS spectrum of thiolated PAMAM-(SH)₆₅. **E.** MALDI-TOF-MS spectrum of thiolated PAMAM-(SH)₁₀₈.
Fig 12: **A.** Reaction scheme of the PEGylation of PAMAM using the PEGylating reagent mPEG₂ₖ-NHS. **B.** MALDI-TOF-MS spectrum of native PAMAM.C. MALDI-TOF-MS spectrum of PEGylated PAMAM-(mPEG₂ₖ)₃. **D**.MALDI-TOF-MS spectrum of PEGylated PAMAM-(mPEG₂ₖ)₈. **E.** MALDI-TOF-MS spectrum of PEGylated PAMAM-(mPEG₂ₖ)₁₈.
Fig 13: **A.** Reaction scheme of Cy3-PAMAM-NC-SO1861- Dibenzocyclooctyne (DBCO). **B.** MALDI-TOF MS spectrum of Cy3-PAMAM-NC-SO1861- Dibenzocyclooctyne (DBCO). **C.** MALDI-TOF-MS spectrum of Cy3-PAMAM-(SO1861)₅-DBCO. **D.** MALDI-TOF-MS spectrum of Cy3-PAMAM-(SO1861)₂₇-DBCO.
Fig 14: **A.** Reaction scheme of dianthin-EGF-Alexa488. **B.** Reaction scheme of dianthin-EGF-Alexa488-SS-PEG-Ns. **C.** MALDI-TOF-MS spectrum of dianthin-EGF. **D.** MALDI-TOF-MS spectrum of dianthin-EGF-Alexa488. E. MALDI-TOF-MS spectrum of dianthin-EGF-Alexa488-SS-PEG-N₃.
Fig 15: **A.** Reaction scheme of dianthin-Alexa488. **B.** Reaction scheme of dianthin-Alexa488-SS-PEG-N₃. **C.** MALDI-TOF-MS spectrum of Dianthin. **D.** MALDI-TOF-MS spectrum of dianthin-Alexa488. **E.** MALDI-TOF-MS spectrum of dianthin-Alexa488-SS-PEG-N₃.
Fig 16: Fluorescence images of SDS-PAGE gel performed on a VersaDoc imaging system. M = marker, P = Cy3-PAMAM-(SO1861)27-DBCO, D = dianthin-EGF-Alexa488-SS-PEG-N3, C1 = Cy3-PAMAM-(SO1861)5-Dianthin-EGF-Alexa488, C2 = Cy3-PAMAM-NC-SO1861-Dianthin-EGF-Alexa488, and C3 = Cy3-PAMAM-(SO1861)27-Dianthin-EGF-Alexa488.
Fig 17: **A.** Synthesis scheme of Cy3-PAMAM-NC-SO1861 via reductive amination. **B.** MALDI-TOF-MS spectrum of Cy3-PAMAM-NC-SO1861 synthesized via reductive amination with 10 SO1861 per PAMAM. **C.** MALDI-TOF-MS spectrum of Cy3-PAMAM-NC-SO1861 synthesized via reductive amination with 30 SO1861 per PAMAM.
Fig 18: **A.** MALDI-TOF-MS spectrum of native peptide. **B.** MALDI-TOF-MS spectrum of peptide-SO1861 conjugate.
Fig 19: SO1861 structure with highlighted chemical groups for conjugation of endosomal escape enhancing saponins to a polymeric structure. Highlighted groups are aldehyde (black circle), carboxylic acid (dashed circle), alkene (dashed pentagon), and alcohol (dashed box).
Fig 20: **A.** Schematic representation of the production of stable 'ready-to conjugate' endosomal escape enhancer saponins. **B.** Schematic representation of the production of cleavable 'ready-to conjugate' endosomal escape enhancer saponins.
Fig 21: Fig 26: Hydrolysis of the hydrazone bond of SO1861-EMCH under acidic conditions.
Fig 22: **A.** Standard molecular structure of SO-1861-EMCH conjugate. Maleimide group is marked with a circle. **B.** 3D model of SO1861-EMCH conjugate. Maleimide group is marked with a circle.
Fig 23: **A.** MALDI-TOF-MS spectrum of SO1861-EMCH before hydrolysis in HCI solution at pH 3. **B.** MALDI-TOF-MS spectrum of SO1861-EMCH after hydrolysis in HCI solution at pH 3.
Fig 24: Reaction scheme of SO1861-EMCH conjugation to any amine-bearing polymeric structure.
Fig 25: **A.** Reaction scheme of SO1861-EMCH conjugation to a cyanine 3 dye labeled polyamidoamine (PAMAM) G5 dendrimer. **B.** Reaction scheme of SO1861-HATU (HATU = 1-[Bis(dimethylamino)methylene]-1H-1,2,3-triazolo[4,5-b]pyridinium 3-oxid hexafluorophosphate) conjugation to a cyanine 3 dye labeled polyamidoamine (PAMAM) G5 dendrimer.
Fig 26: Molecular structure of G4-dendron with protected amino groups.
Fig 27: Synthesis scheme for the generation of dendron based scaffolds.
Fig 28: **A.** Reaction scheme for partial dye labeling and deprotection of the G4-dendron. **B.** MALDI-TOF-MS spectrum of deprotected and partially dye labeled G4-dendron.
Fig 29: **A.** MALDI-TOF-MS spectrum of G4-dendron-SO1861 scaffold with 22 feed equivalents of SO1861-EMCH. **B.** MALDI-TOF-MS spectrum of G4-dendron-SO1861 scaffold with 10 feed equivalents of SO1861-EMCH. **C.** MALDI-TOF-MS spectrum of G4-dendron-SO1861 scaffold with 3 feed equivalents of SO1861-EMCH.

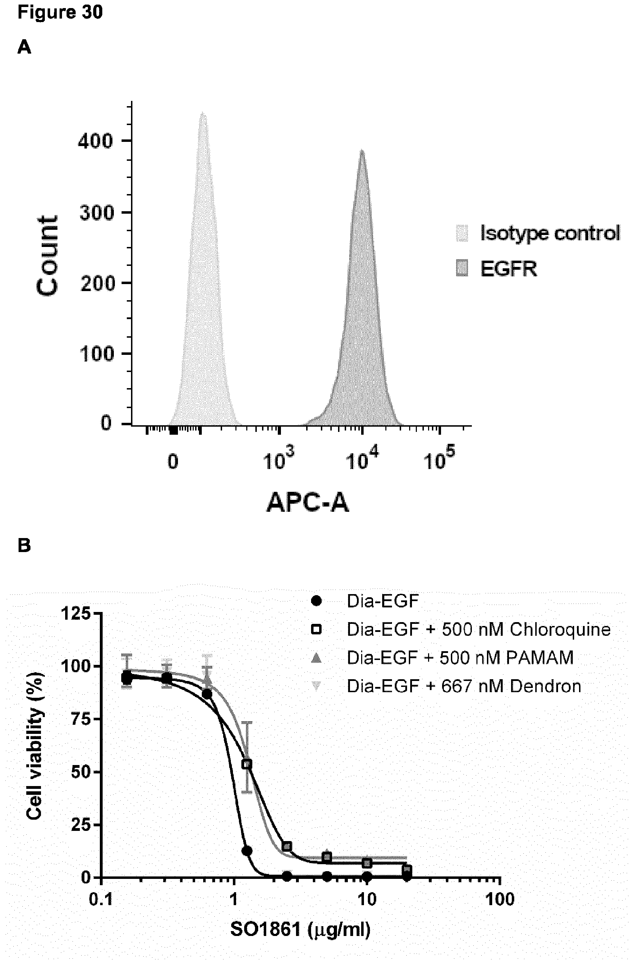

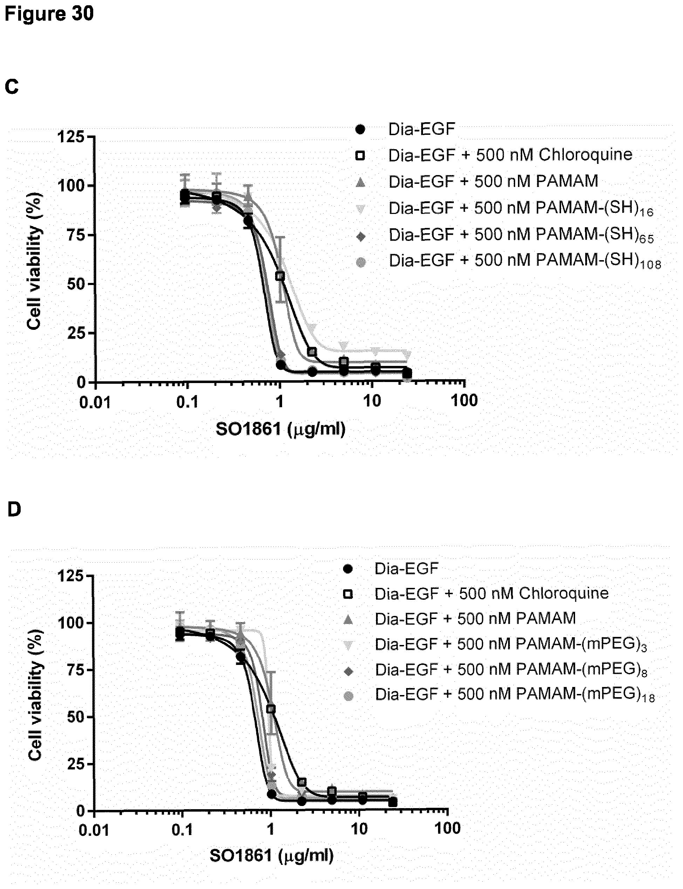

Fig 30: **A.** EGFR cell surface expression as determined by FACS analyses of HeLa cells. **B.** Cell viability of HeLa cells treated with SO1861 + dianthin-EGF (Dia-EGF), SO1861 + dianthin-EGF + 500 nM chloroquine, SO1861 + dianthin-EGF + 500 nM PAMAM, SO1861 + dianthin-EGF + 667 nM dendron. **C.** Cell viability of HeLa cells treated with SO1861 + dianthin-EGF, SO1861 + dianthin-EGF + 500 nM chloroquine, SO1861 + dianthin-EGF + 500 nM PAMAM, SO1861 + dianthin-EGF + 500 nM PAMAM-(SH)₁₆, SO1861 + dianthin-EGF + 500 nM PAMAM-(SH)ss, SO1861 + dianthin-EGF + 500 nM PAMAM-(SH)₁₀₈.**D**. Cell viability of HeLa cells treated with SO1861 + dianthin-EGF, SO1861 + dianthin-EGF + 500 nM chloroquine, SO1861 + dianthin-EGF + 500 nM PAMAM, SO1861 + dianthin-EGF + 500 nM PAMAM-(mPEG)₃, SO1861 + dianthin-EGF + 500 nM PAMAM-(mPEG)₈, SO1861 + dianthin-EGF + 500 nM PAMAM-(mPEG)₁₈.

### DETAILED DESCRIPTION

In order for a bioactive molecule to work, the molecule must be able to engage with its target, e.g. in the blood serum, on the outside of the cell surface or inside a cell or an organelle. The active moiety of almost all protein-based targeted toxins, e.g., must enter the cytosol of the target cell to mediate its target modulatory effect. In many constellations the toxin remains ineffective since (1) the targeting moiety is poorly internalized and remains bound to the outside of the cells, (2) is recycled back to the cell surface after internalization or (3) transported to the endolysosomes where it is degraded. Although these fundamental issues are known for decades and more than 500 targeted toxins have been investigated in the past decades, the problems have not been solved yet and only one antibody-targeted protein toxin, moxetumomab pasudotox-tdfk (LUMOXITI^{®}, AstraZeneca Pharmaceuticals LP), has been approved for relapsed or refractory hairy cell leukemia by the FDA to date. Further approved ADCs are Elzonris, Ontak.

To overcome these problems, many strategies have been described including approaches to redirect the toxins to endogenous cellular membrane transport complexes of the biosynthetic pathway in the endoplasmic reticulum and techniques to disrupt or weaken the membrane integrity of endosomes, i.e. the compartments of the endocytic pathway in a cell, and thus facilitating the endosomal escape. This comprises the use of lysosomotropic amines, carboxylic ionophores, calcium channel antagonists, various cell-penetrating peptides of viral, bacterial, plant, animal, human and synthetic origin, other organic molecules and light-induced techniques. Although the efficacy of the targeted toxins was typically augmented in cell culture hundred- or thousand-fold, in exceptional cases more than million-fold, the requirement to co-administer endosomal escape enhancers with other substances harbors new problems including additional side effects, loss of target specificity, difficulties to determine the therapeutic window and cell type-dependent variations.

All strategies, including physicochemical techniques, require enhancer molecules that interact more or less directly with membranes and comprise essentially small chemical molecules, secondary metabolites, peptides and proteins. A common feature of all these substances is that they are *per se* not target cell-specific and distribute with other kinetics than the targeted toxins. This is one major drawback of the current approaches.

The present invention will be described with respect to particular embodiments but the invention is not limited thereto but only by the claims. The embodiments of the invention described herein can operate in combination and cooperation, unless specified otherwise.

While the invention has been described in terms of several embodiments, it is contemplated that alternatives, modifications, permutations and equivalents thereof will become apparent to one having ordinary skill in the art upon reading the specification and upon study of the drawings and graphs. The invention is not limited in any way to the illustrated embodiments. Changes can be made without departing from the scope which is defined by the appended claims.

An aspect of the invention relates to an effector moiety capable of inducing an intracellular effect when present inside a mammalian cell, the effector moiety conjugated with at least one saponin, wherein the at least one saponin is covalently bound to the effector moiety via at least one linker, or is covalently bound directly to said effector moiety.

The inventors established that the therapeutic window of a conjugate of a saponin and an effector moiety, increases when administered to a tumor-bearing mammal (mouse) to whom the conjugate is administered, wherein said conjugate comprises at least one covalently bound saponin. The conjugate of the invention has at least one glycoside such as a saponin bound thereto, preferably covalently, more preferably via a cleavable linker. The saponin augments the therapeutic efficacy of the effector moiety bound to the saponin, likely by enhancing the endosomal escape of the effector moiety into the cytosol where the activity of the effector moiety is desired. This way, already at a lower dose than the conventional dose of the effector moiety, when part of a conjugate such as an ADC or an AOC, therapeutic effect is established under influence of the presence of the saponin(s) comprised by the conjugate of the invention thereby bringing the saponin(s) and effector moieties near, at and/or inside the targeted cell together. The target cell is for example a diseased cell such as a tumor cell or an auto-immune cell or a B-cell disease related B-cell, etc. The effector moiety is for example a toxin such as a toxin which is part of an ADC, or an oligonucleotide such as an antisense BNA which is part of an AOC, according to the invention.

An embodiment is the effector moiety of the invention, comprising or consisting of at least one oligonucleotide, a nucleic acid, a xeno nucleic acid, preferably selected from any one or more of a vector, a gene, a cell suicide inducing transgene, deoxyribonucleic acid (DNA), ribonucleic acid (RNA), anti-sense oligonucleotide (ASO, AON), short interfering RNA (siRNA), microRNA (miRNA), DNA aptamer, RNA aptamer, mRNA, mini-circle DNA, peptide nucleic acid (PNA), phosphoramidate morpholino oligomer (PMO), locked nucleic acid (LNA), bridged nucleic acid (BNA), 2'-deoxy-2'-fluoroarabino nucleic acid (FANA), 2'-O-methoxyethyl-RNA (MOE), 2'-O,4'-aminoethylene bridged nucleic acid, 3'-fluoro hexitol nucleic acid (FHNA), a plasmid, glycol nucleic acid (GNA) and threose nucleic acid (TNA), or a derivative thereof, more preferably a BNA, for example a BNA for silencing HSP27 protein expression.

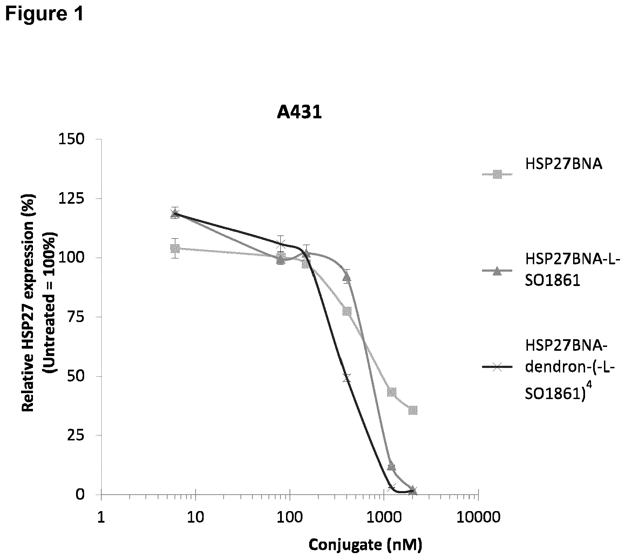

To the surprise of the inventors, a conjugate of antisense BNA such as for silencing HSP27 gene expression, and a saponin such as SO1861 , covalently linked together, effectively silence HSP27 expression in rapidly growing/dividing tumor cells, such as mouse A431 cancer cell line cells, compared to control. The BNA-saponin conjugate resulted in an about threefold lower HSP27 expression in cells, compared to HSP27 expression in cells exposed to the same concentration of the free unconjugated BNA in the absence of saponin. See also Figure 1 of the Examples. Thus, contacting tumor cells with an antisense oligonucleotide such as antisense BNA results in gene silencing which is improved or enhanced when saponin such as SO1861 is covalently linked to the oligonucleotide. Therewith, the therapeutic window with regard to the oligonucleotide is enlarged, since the same gene silencing effect in the tumor cells can now be obtained with a threefold lower dose of the antisense BNA when a conjugate of BNA and saponin according to the invention is applied.

An embodiment is the effector moiety of the invention, comprising at least one proteinaceous molecule, the proteinaceous molecule preferably selected from any one or more of a peptide, a protein, an enzyme such as urease and Cre-recombinase, a ribosome-inactivating protein, a proteinaceous toxin such as any one or more of a protein toxin selected from Table A5 and/or a bacterial toxin or plant toxin, more preferably selected from any one or more of a viral toxin such as apoptin; a bacterial toxin such as Shiga toxin, Shiga-like toxin, Pseudomonas aeruginosa exotoxin (PE) or exotoxin A of PE, full-length or truncated diphtheria toxin (DT), cholera toxin; a fungal toxin such as alpha-sarcin; a plant toxin including ribosome-inactivating proteins and the A chain of type 2 ribosome-inactivating proteins such as dianthin e.g. dianthin-30 or dianthin-32, saporin e.g. saporin-S3 or saporin-S6, bouganin or de-immunized derivative debouganin of bouganin, shiga-like toxin A, pokeweed antiviral protein, ricin, ricin A chain, modeccin, modeccin A chain, abrin, abrin A chain, volkensin, volkensin A chain, viscumin, viscumin A chain; or an animal or human toxin such as frog RNase, or granzyme B or angiogenin from humans, or any fragment or derivative thereof; preferably the protein toxin is dianthin and/or saporin.

An embodiment is the effector moiety of the invention, comprising at least one payload, the payload preferably selected from any one or more of a toxin targeting ribosomes, a toxin targeting elongation factors, a toxin targeting tubulin, a toxin targeting DNA and a toxin targeting RNA, more preferably any one or more of emtansine, pasudotox, maytansinoid derivative DM1, maytansinoid derivative DM4, monomethyl auristatin E (MMAE, vedotin), monomethyl auristatin F (MMAF, mafodotin), a Calicheamicin, N-Acetyl-γ-calicheamicin, a pyrrolobenzodiazepine (PBD) dimer, a benzodiazepine, a CC-1065 analogue, a duocarmycin, Doxorubicin, paclitaxel, docetaxel, cisplatin, cyclophosphamide, etoposide, docetaxel, 5-fluorouracyl (5-FU), mitoxantrone, a tubulysin, an indolinobenzodiazepine, AZ13599185, a cryptophycin, rhizoxin, methotrexate, an anthracycline, a camptothecin analogue, SN-38, DX-8951f, exatecan mesylate, truncated form of *Pseudomonas aeruginosa* exotoxin (PE38), a Duocarmycin derivative, an amanitin, α-amanitin, a spliceostatin, a thailanstatin, ozogamicin, tesirine, Amberstatin269 and soravtansine, or a derivative thereof.

An embodiment is the effector moiety of the invention, wherein the at least one saponin is a triterpenoid saponin and/or a bisdesmosidic triterpene saponin belonging to the type of a 12,13-dehydrooleanane with an aldehyde function in position C-23 and optionally comprising a glucuronic acid function in a carbohydrate substituent at the C-3beta-OH group of the saponin, and/or a saponin isolated from a *Gypsophila* species and/or a *Saponaria* species and/or an *Agrostemma* species and/or a *Quillaja* species such as *Quillaja saponaria.*

An embodiment is the effector moiety of the invention, wherein the at least one saponin is a single specific saponin or is a mixture of two or more different saponins.

An embodiment is the effector moiety of the invention, wherein the saponin is one or more of the saponins in Table A1 or Scheme I, SO1861, SA1657, GE1741, SA1641, QS-21, QS-21A, QS-21 A-api, QS-21 A-xyl, QS-21B, QS-21 B-api, QS-21 B-xyl, QS-7-xyl, QS-7-api, QS-17-api, QS-17-xyl, QS1861, QS1862, Quillajasaponin, Saponinum album, QS-18, Quil-A, Gyp1, gypsoside A, AG1, AG2, SO1542, SO1584, SO1658, SO1674, SO1832, or any of their stereomers and/or any combinations thereof, preferably the saponin is SO1861 and/or GE1741 and/or SA1641 and/or QS-21 and/or saponin with aquillaic acid aglycon core, a Gal-(1→2)-[Xyl-(1→3)]-GIcA carbohydrate substituent at the C-3beta-OH group and a Glc-(1→3)-Xyl-(1→4)-Rha-(1→2)-[Xyl-(1→3)-4-OAc-Qui-(1→4)]-Fuc carbohydrate substituent at the C-28-OH group, and/or is 3-O-beta-D-galactopyranosyl-(1→2)-[beta-D-xylopyranosyl-(1→3)]-beta-D-glucuronopyranosyl quillaic acid 28-O-beta-D-glucopyranosyl-(1→3)-beta-D-xytopyranosyt-(1→4)- alpha-L-rhamnopyranosyl-(1→2)-[beta-D-xylopyranosyl-(1→3)-4-OAc-beta-D-quinovopyranosyl-(1→4)]-beta-D-fucopyranoside, more preferably the saponin is SO1861 and/or QS-21.

Surprisingly, the inventors now demonstrate that a water-soluble saponin fraction from Quillaja saponaria, comprising QS-21 and its family members QS-21A, QS-21 A-api, QS-21 A-xyl, QS-21B, QS-21 B-api, QS-21 B-xyl, QS-7-xyl, QS-7-api, QS-17-api, QS-17-xyl, QS1861, QS1862, QS-18 and Quil-A, also exhibits the ability to potentiate a biological effect in vitro of e.g. a nucleic acid bound to a monoclonal antibody or a protein toxin bound to a monoclonal antibody, when administered to tumor cells of a mammalian species (human) in the form of a covalent conjugate comprising a monoclonal antibody and the at least one glycoside such as the QS-21 and its family member saponins encompassed by such QS-21 preparation (e.g. water soluble fraction of Quillaja saponaria), herein the effector molecule and the glycoside, e.g. saponin fraction of Quillaja saponaria, QS-21, SO1861, SA1641, GE1741, are covalently bound to for example a proteinaceous molecules directly or via a linker or via a polymeric or oligomeric scaffold, either directly or via at least one linker. Without wishing to be bound by any theory, the observed stimulation or potentiation of for example antisense BNA mediated reduction of tumor-cell HSP27 expression (HSP27 gene silencing) in the presence of saponins derived from Quillaja saponaria in vitro may (also) relate to activation of the inflammasome in the tumor cell by the saponins, for example resulting in tumor cell pyroptosis. The inventors established that an antibody conjugated to for example antisense BNA or dianthin or saporin, exerted any anti-tumor cell activity in vitro at all or improved anti-tumor cell activity when contacted with cells in bio-based cell assays, when in the presence of saponin coupled to a targeting antibody, and targeted to the same (tumor) cells, whereas in the absence of the saponin, no such activity towards the tumor cell was observed.

QS-21, and also the water-soluble saponins fraction comprising QS-21 from Quillaja saponaria is already for a long time known and previously intensively applied for its immune-potentiating abilities, e.g. as an adjuvant in e.g. sub-unit vaccines. For example, QS-21 is applied in two phase III clinical trials with human patients, who were vaccinated with a sub-unit vaccine mixed with an adjuvant comprising QS-21 (Glaxo-Smith-Kline, MAGRIT trial, DERMA study), wherein the sub-unit was MAGE-A3 protein, which is specifically expressed and presented by tumor cells. The anti-tumor vaccinations, potentiated with QS-21, aimed for extension of disease-free survival of the cancer patients (melanoma; non-small cell lung cancer). In addition, QS-21 has been tested as an adjuvant in clinical trials for developing anti-cancer vaccine treatment, for vaccines for HIV-1 infection, in development of a vaccine against hepatitis B, and for anti-malaria vaccine development using QS-21 comprising adjuvants AS01 and AS02 of Glaxo-Smith-Kline. Previous studies revealed an immune response elicited against MAGE-A3 peptides presented at the cancer cell surface, under influence of the QS-21 saponin comprising adjuvant (AS15; GSK). To the surprise of the inventors, the saponin fraction of Quillaja saponaria, and thus likely QS-21 (as part of the water soluble saponin fraction of Quillaja saponaria) potentiates the anti-tumor cell activity of e.g. a payload such as a protein toxin (dianthin), bound to the second proteinaceous molecule (e.g. the ligand EGF).

An embodiment is the effector moiety of the invention, wherein the saponin is a bisdesmosidic saponin having a molecular mass of at least 1.500 Dalton and comprising an oleanan-type triterpene containing an aldehyde group at the C-23 position and optionally a hydroxyl group at the C-16 position, with a first branched carbohydrate side chain at the C-3 position which first branched carbohydrate side chain optionally contains glucuronic acid, wherein the saponin contains an ester group with a second branched carbohydrate side chain at the C-28 position which second branched carbohydrate chain preferably comprises at least four carbohydrate units and optionally contains at least one acetyl residue such as two acetyl residues and/or optionally comprises one or more deoxy carbohydrates and/or quinovose and/or glucose and/or 4-methoxycinnamic acid and/or optionally comprising 5-O-[5-O-Ara/Api-3,5-dihydroxy-6-methyl-octanoyl]-3,5-dihydroxy-6-methyl-octanoic acid and/or optionally comprising 5-O-[5-O-Rha-(1→2)-Ara/Api-3,5-dihydroxy-6-methyl-octanoyl]-3,5-dihydroxy-6-methyl-octanoic acid bound to a carbohydrate via an ester bond, or wherein the at least one saponin is QS-21 or any one or more of QS-21A, QS-21 A-api, QS-21 A-xyl, QS-21B, QS-21 B-api, QS-21 B-xyl, QS-7-xyl, QS-7-api, QS-17-api, QS-17-xyl, QS-18, QS1861, protonated QS1861 (QS1862), Quil-A.

The inventors show that a tumor-cell targeting monoclonal antibody provided with covalently coupled antisense BNA such as BNA(HSP27), and contacted with the tumor cells together with an antibody with couopled saponin (e.g. SO1861, QS-21), both the BNA and the saponin coupled to the respective antibody (e.g. cetuximab) via a cleavable bond, is capable of silencing HSP27 in vivo in tumors, compared to control and compared to AOC only, without presence of an antibody with coupled saponin. Co-administering an ADC or an antibody-oligonucleotide conjugate (AOC), such as an antibody-BNA conjugate, with a an antibody with linked saponin thus endows the ADC or AOC with anti-tumor cell activity not seen with only the ADC or only the AOC at the same dose. Noteworthy, the AOC and the monoclonal antibody with covalently coupled saponin increase HSP27 expression in tumor cells, when administered to tumor-bearing mice separately in separate groups of mice, compared to a control group (vehicle administered, only). Only co-administration of the AOC comprising the effector moiety and an antibody with covalently coupled saponin, displays reduced HSP27 expression when compared to controls. The antisense BNA (HSP27) was BNA with oligo nucleic acid sequence 5'-GGCacagccagtgGCG-3' according to Zhang et al. (2011) [Y Zhang, Z Qu, S Kim, V Shi, B Liao1, P Kraft, R Bandaru, Y Wu, LM Greenberger and ID Horak, Down-modulation of cancer targets using locked nucleic acid (LNA)-based antisense oligonucleotides without transfection, Gene Therapy (2011) 18, 326-333]. Noteworthy, to the best of the knowledge of the inventors, BNA is designed for application as a free nucleic acid. The inventors are now the first to demonstrate that the antisense BNA can be covalently coupled through a (non-)cleavable linker with a ligand or an antibody, in a way that gene-silencing activity is retained in vitro and more importantly in vivo in the tumor cells of a tumor-bearing animal. This approach of providing BNA based AOCs opens new ways to administer targeted BNA to human (cancer) patients in need thereof.

The inventors disclose here that covalently coupling saponins such as saponins in the water-soluble fraction of Quillaja saponaria, QS-21 , SA1641, SO1861, Table A1, Scheme I, to for example an antisense oligonucleotide such as a BNA, or for example to a proteinaceous molecule, such as via a tri-functional linker, e.g. the tri-functional linker of Scheme II, or via an oligomeric or polymeric structure of a scaffold comprising covalently bound saponins, results in improved cell toxicity exerted by the effector moiety such as a toxin, comprised by the effector moiety of the invention, under influence of the covalently coupled saponin in the effector moiety of the invention.

According to the invention, typically the saponin is a saponin listed in Table A1, Scheme I. It has been proven beneficial for the activity of the saponin, e.g. the endosomal escape enhancing activity inside cells when the entry into the cell and the accumulation inside the cytosol of an effector moiety of the invention comprising coupled saponin, is considered, when the saponin is covalently coupled to the payload comprised by the effector moiety (e.g. a BNA) involving a hydrazone bond, and/or a hydrazide bond, and/or a disulphide bond. Such bond types readily cleave under the acidic conditions inside (late) endosomes and lysosomes of mammalian cells, e.g. human cells, and/or under the reductive conditions. Alternatively, the inventors also demonstrate that covalent coupling of saponin to the payload comprised by the effector moiety of the invention via a bond that is not readily cleavable under the physiological conditions inside cells, e.g. (late) endosomes, lysosomes, cytosol, is also beneficial to the potentiating activity of the saponin on the biological effect of e.g. an effector moiety such as a nucleic acid (e.g. BNA silencing HSP27) and a proteinaceous toxin such as saporin. Throughout the application, including the claims, the term `cleavable linker', `cleavable bond', etc., is also referred to as `labile linker' (`L') and `labile bond', for example in the context of cleavage of such a bond or linker in the (late) endosome and/or lysosome when a conjugate of the invention, e.g. an effector moiety such as BNA-SO1861, or antisense oligonucleotide-saponin conjugate (the saponin being any of the saponins in Table A1, Scheme I), optionally comprising a scaffold with saponins coupled to payload through a linker and/or via the scaffold via hydrazone bonds or disulphide bonds, is referred to. For example, the inventors showed the in vivo HSP27 gene silencing in human tumors in mice. The tumor-bearing mice were treated with an antibody consisting of monoclonal antibody with saponin bound thereto via a labile linker (hydrazone bond) according to the invention, whereas a different portion of the same antibody comprised bound antisense BNA for silencing the HSP27 gene in the tumor cells, covalently coupled to the monoclonal antibody (being same type of antibody as the first monoclonal antibody) via a a disulphide bond. That is to say, without wishing to be bound by any theory, the hydrazone bond and the disulphide bond are cleaved in the (late) endosomes and/or lysosomes of the targeted tumor cells that express the epitope on the targeted cell-surface molecule, here the EGFR, at the cell surface, once the therapeutic combination of the invention is internalized by e.g. endocytosis. Cleavage of the bonds likely contributes to the endosomal escape enhancing activity of the saponin when the entry of the BNA from the endosome and/or lysosome into the cytosol is considered, although such cleavage is not a necessity for observing the gene silencing effect of the combination of the cetuximab-SO1861 conjugate and the cetuximab-BNA conjugate of the invention.

The skilled person will appreciate that a tri-functional linker is a scaffold of the invention suitable for covalently coupling one, two or three saponin moieties. For the tri-functional linker covalent coupling of one or two saponin moieties is preferred. The second and/or third binding site is for example suitable for covalent coupling an payload, such that an effector moiety of the invention is provided. Moreover, the second or third binding site of the tri-functional linker is suitable for covalent coupling of an immunoglobulin such as a monoclonal antibody, preferably a tumor-cell specific molecule, more preferably a tumor cell receptor that is specifically (over-)expressed at the surface of the tumor cell. Similarly, the immunoglobulin, or any fragment(s) and/or domain(s) thereof which encompass the binding specificity of the immunoglobulin, is suitable for binding to a cell surface molecule such as a receptor, expressed at the surface of an autoimmune cell. The tri-functional linker then comprises a covalently coupled saponin and a covalently coupled payload, therewith providing the effector moiety of the invention, and in addition a covalently coupled e.g. antibody, for targeting the effector moiety to a selected cell such as a tumor cell expressing the tumor-cell specific binding site for the coupled antibody, e.g. a specific tumor-cell receptor. Thus, in an embodiment, the effector moiety of the invention comprises the tri-functional linker, said linker comprises or consists of a covalently bound saponin and effector moiety, e.g. QS-21, SO1861 and e.g. antisense BNA, protein toxin, and the covalently bound antibody for (specific) binding to a tumor cell, an auto-immune cell, a diseased cell, an aberrant cell, a non-healthy cell, a B-cell disease.

An embodiment is the effector moiety of the invention, wherein the at least one saponin is a bisdesmosidic triterpene saponin belonging to the type of a 12,13-dehydrooleanane with an aldehyde function in position C-23, wherein the at least one saponin is covalently coupled to an amino-acid residue, when present, of the effector moiety via an aldehyde function in the saponin, preferably said aldehyde function in position C-23, preferably via at least one linker, more preferably via at least one cleavable linker, wherein the amino-acid residue preferably is selected from cysteine and lysine.

An embodiment is the effector moiety of the invention, wherein the at least one saponin is a bisdesmosidic triterpene saponin belonging to the type of a 12,13-dehydrooleanane with an aldehyde function in position C-23 and comprising a glucuronic acid function in a carbohydrate substituent at the C-3beta-OH group of the saponin, wherein the at least one saponin is covalently coupled to an amino-acid residue, when present, of the effector moiety via the glucuronic acid function in the carbohydrate substituent at the C-3beta-OH group of the saponin, preferably via at least one linker, wherein the amino-acid residue preferably is selected from cysteine and lysine.

As an example, BNAoligo, antisense BNA oligo targeting the mRNA transcript of the cancer target (upregulated in cancer cells), heat shock protein 27 (HSP27BNA) was conjugated to SO1861-EMCH (HSP27BNA-L-SO1861) or dendron(-L-SO1861)⁴ (HSP27BNA-dendron(-L-SO1861)⁴) and co-administrated to an A431 cancer cell line, according to the invention. As said, to the surprise of the inventors, a conjugate of antisense BNA such as for silencing HSP27 gene expression, and a saponin such as SO1861, covalently linked together, effectively silence HSP27 expression in rapidly growing/dividing tumor cells, such as mouse A431 cancer cell line cells, compared to control. Covalently conjugating any of the saponins of the invention with an oligonucleotide, such as an antisense oligonucleotide, for example a BNA such as an antisense BNA, results in increased gene silencing efficacy at the same dose of BNA when compared to free BNA in the absence of conjugated saponin and in the absence of free saponin. The BNA-saponin conjugate resulted in an about threefold lower HSP27 expression in cells, compared to HSP27 expression in cells exposed to the same concentration of the free unconjugated BNA in the absence of saponin. See also Figure 1 of the Examples. Thus, contacting tumor cells with an antisense oligonucleotide such as antisense BNA results in gene silencing which is improved or enhanced when saponin such as SO1861 is covalently linked to the oligonucleotide. Therewith, the therapeutic window with regard to the oligonucleotide is enlarged, since the same gene silencing effect in the tumor cells can now be obtained with a threefold lower dose of the antisense BNA when a conjugate of BNA and saponin according to the invention is applied. The inventors established that the conjugate of BNA with one saponin is more effective when gene silencing is considered than the gene silencing activity obtainable with free BNA administered to the cells. Moreover, the inventors established that the conjugate of BNA with four saponins is increasingly effective when gene silencing is considered and when compared to the gene silencing activity obtainable with free BNA administered to the cells. That is to say, improved gene silencing with BNA(-saponin)4 is even higher than improved gene silencing obtainable with BNA-saponin, according to the invention. The linker(s) is/are cleavable linkers.

An embodiment is the effector moiety of the invention, wherein the at least one linker comprises at least one non-cleavable linker and/or at least one cleavable linker, wherein optionally said cleavable linker is subject to cleavage under acidic, reductive, enzymatic or light-induced conditions, and preferably the cleavable linker comprises a cleavable bond selected from a hydrazone bond and a hydrazide bond subject to cleavage under acidic conditions, and/or a bond susceptible to proteolysis, for example proteolysis by Cathepsin B, and/or a bond susceptible for cleavage under reductive conditions such as a disulphide bond.

An embodiment is the effector moiety of the invention, wherein the at least one linker comprises at least one cleavable linker which is subject to cleavage *in vivo* under acidic conditions as present in endosomes and/or in lysosomes of mammalian cells, preferably of human cells, preferably at pH 4.0 - 6.5, and more preferably at pH ≤ 5.5.

An embodiment is the effector moiety of the invention, wherein the at least one saponin is covalently bound to a lysine side chain, forming an amide bond, and/or to a cysteine side chain, forming a thio-ether linkage, or a disulphide bond, wherein the lysine and/or cysteine is/are comprised by the effector moiety, and wherein the saponin is bound either directly to the effector moiety, or bound via at least one linker.

An embodiment is the effector moiety of the invention, wherein the at least one saponin is covalently bound to the effector moiety via at least one linker, wherein the linker is or comprises a scaffold comprising a polymeric or oligomeric structure and a chemical group for covalently coupling of the scaffold to the effector moiety.

An embodiment is the effector moiety of the invention, wherein the at least one saponin is covalently bound to the polymeric or oligomeric structure of the scaffold via a cleavable bond.

An embodiment is the effector moiety of the invention, wherein the cleavable bond is subject to cleavage under any of acidic conditions, reductive conditions, enzymatic conditions and light-induced conditions, more preferably the cleavable bond is a hydrazone bond or a hydrazide bond subject to cleavage under acidic conditions, and/or is a bond susceptible to proteolysis, for example proteolysis by Cathepsin B, and/or is a bond susceptible for cleavage under reductive conditions such as a disulphide bond.

An embodiment is the effector moiety of the invention, wherein the cleavable bond is subject to cleavage *in vivo* under acidic conditions as present in endosomes and/or in lysosomes of mammalian cells, preferably of human cells, preferably at pH 4.0 - 6.5, and more preferably at pH ≤ 5.5.

An embodiment is the effector moiety of the invention, wherein the at least one saponin is covalently bound to the polymeric or oligomeric structure of the scaffold via an imine bond, a hydrazone bond, a hydrazide bond, an oxime bond, a 1,3-dioxolane bond, a disulphide bond, a thio-ether bond, an amide bond, a peptide bond and/or an ester bond, preferably via at least one linker, preferably an amide bond, a hydrazide bond, a thio-ether bond and/or a hydrazone bond.

An embodiment is the effector moiety of the invention, wherein the at least one saponin is covalently bound to the polymeric or oligomeric structure of the scaffold, involving in the covalent bond the aldehyde function in position C-23 of the at least one saponin, when present, the covalent bond being preferably an imine bond or a hydrazone bond or an amide bond or a thio-ether bond or a disulphide bond, and/or involving in the covalent bond the glucuronic acid function in the carbohydrate substituent at the C-3beta-OH group of the at least one saponin, when present, wherein preferably the covalent bond is an amide bond or a disulphide bond or a thio-ether bond.

An embodiment is the effector moiety of the invention, wherein the aldehyde function in position C-23 of the at least one saponin is covalently coupled to linker N-ε-maleimidocaproic acid hydrazide, which linker is covalently coupled via a thio-ether bond to a sulfhydryl group in the polymeric or oligomeric structure of the scaffold, such as a sulfhydryl group of a cysteine.

An embodiment is the effector moiety of the invention, wherein the glucuronic acid function in the carbohydrate substituent at the C-3beta-OH group of the at least one saponin is covalently coupled to linker 1-[Bis(dimethylamino)methylene]-1H-1,2,3-triazolo[4,5-b]pyridinium 3-oxid hexafluorophosphate, which linker is covalently coupled via an amide bond to an amine group in the polymeric or oligomeric structure of the scaffold, such as an amine group of a lysine or an N-terminus of the polymeric or oligomeric structure of the scaffold.

An embodiment is the effector moiety of the invention, wherein the chemical group of the scaffold, for covalently coupling of the scaffold to the effector moiety, is a click chemistry group, preferably selected from a tetrazine, an azide, an alkene or an alkyne, or a cyclic derivative of these groups, more preferably the click chemistry group is an azide.

An embodiment is the effector moiety of the invention, wherein the polymeric or oligomeric structure of the scaffold comprises a linear, branched and/or cyclic polymer, oligomer, dendrimer, dendron, dendronized polymer, dendronized oligomer, a DNA, a polypeptide, poly-lysine, a polyethylene glycol, or an assembly of these polymeric or oligomeric structures which assembly is preferably built up by covalent cross-linking.

An embodiment is the effector moiety of the invention, wherein the at least one saponin is a defined number of saponins or a defined range of saponins, preferably 1-128 saponins or at least 2, 3, 4, 5, 6, 8, 10, 16, 32, 64 or 128 saponins, or any number of saponins therein between, such as 7, 9, 12 saponins.

An embodiment is the effector moiety of the invention, wherein the effector moiety comprises more than one saponin, preferably 2, 3, 4, 5, 6, 8, 10, 16, 32, 64 or 1-100 saponins, or any number of saponins therein between, such as 7, 9, 12 saponins, covalently bound directly to an amino-acid residue of the effector moiety, preferably to a cysteine and/or to a lysine, and/or covalently bound via at least one linker and/or via at least one cleavable linker and/or via at least one polymeric or oligomeric scaffold of any one of the claims 14-23, preferably 1-8 of such scaffolds or 2-4 of such scaffolds, wherein 1-32 saponins, preferably 2, 3, 4, 5, 6, 8, 10, 16 or 32 saponins, are covalently bound to the at least one scaffold.

The inventors established that administering free oligonucleotide such as antisense BNA to the mice was not very effective when gene silencing in (rapidly growing) tumor cells is considered. Surprisingly, the inventors now established that beneficial anti-tumor activity in various in vitro mammalian cell-based bioassays can be achieved by treating the animals with conjugates according to the invention, optionally comprising a scaffold according to the invention such as a G4-dendron. The scaffold for example being a tri-functional linker with at least one covalently bound saponin (e.g. SO1861, QS-21) via a cleavable or non-cleavable linkage, and/or with a covalently bound effector moiety (e.g. an antisense oligonucleotide, silencing BNA (HSP27) via a non-cleavable bond or a cleavable bond, or the scaffold being a dendron, such as a dendron to which for example four moieties can bind such as four saponin molecules, or a dendron for binding for example two saponins and two effector molecules. Reference is made to the Examples section, exemplifying linkers and scaffolds according to the invention, showing in vitro anti-tumor cell activity when gene silencing exerted by e.g. antisense BNA(HSP27) is considered thus whengene silencing in the tumor cell is considered.

With the term "improving or enhancing an effect of an effector moiety" is meant that the glycoside molecule, preferably a saponin of the invention, increases the functional efficacy of that effector moiety (e.g. the therapeutic index of a toxin or a drug or an oligonucleotide such as a BNA; the metabolic efficacy of a modifier in biotechnological processes; the transfection efficacy of genes in cell culture research experiments), preferably by enabling or improving its target engagement. Acceleration, prolongation, or enhancement of antigen-specific immune responses are preferably not included. Therapeutic efficacy includes but is not limited to a stronger therapeutic effect, preferably with lower dosing and/or with less side effects. "Improving an effect of an effector moiety" can also mean that an effector moiety, which could not be used because of lack of effect (and was e.g. not known as being an effector moiety), becomes effective when used in combination with the present invention. Any other effect, which is beneficial or desired and can be attributed to the combination of effector moiety and the second or third proteinaceous molecule, as provided by the invention is considered to be "an improved effect". In an embodiment, the scaffold comprising bound saponin(s) and comprised by the effector moiety enhances an effect of the payload / effector moiety which effect is intended and/or desired.

A number of preferred features can be formulated for endosomal escape enhancers comprised by effecor moiety, i.e. a glycoside or saponin, preferably a saponin according to the invention: (1) they are preferably not toxic and do not invoke an immune response, (2) they preferably do not mediate the cytosolic uptake of the effector moiety into off-target cells, (3) their presence at the site of action is preferably synchronized with the presence of the effector moiety, (4) they are preferably biodegradable or excretable, and (5) they preferably do not substantially interfere with biological processes of the organism unrelated to the biological activity of the effector molecule with which the endosomal escape enhancer is combined with, e.g. interact with hormones. Examples of glycoside molecules such as saponins of the invention that fulfill the before mentioned criteria, at least to some extent, are bisdesmosidictriterpenes, preferably bisdesmosidictriterpene saponins, such as SO1861, SA1641, QS-21, GE1741, and the saponins in Table A1, Scheme I.

To explain the invention in more detail, the process of cellular uptake of substances (although the inventors do not wish to be bound by any theory) and the used terminology in this invention is described. The uptake of extracellular substances such as the effector moiety of the invention, into a cell by vesicle budding is called endocytosis. Said vesicle budding can be characterized by (1) receptor-dependent ligand uptake mediated by the cytosolic protein clathrin, (2) lipid-raft uptake mediated by the cholesterol-binding protein caveolin, (3) unspecific fluid uptake (pinocytosis), or (4) unspecific particle uptake (phagocytosis). All types of endocytosis run into the following cellular processes of vesicle transport and substance sorting called the endocytic pathways. The endocytic pathways are complex and not fully understood. Without wishing to be bound by any theory, organelles may be formed *de novo* and mature into the next organelle along the endocytic pathway. It is however, now hypothesized that the endocytic pathways involve stable compartments that are connected by vesicular traffic. A compartment is a complex, multifunctional membrane organelle that is specialized for a particular set of essential functions for the cell. Vesicles are considered to be transient organelles, simpler in composition, and are defined as membrane-enclosed containers that form *de novo* by budding from a preexisting compartment. In contrast to compartments, vesicles can undergo maturation, which is a physiologically irreversible series of biochemical changes. Early endosomes and late endosomes represent stable compartments in the endocytic pathway while primary endocytic vesicles, phagosomes, multivesicular bodies (also called endosome carrier vesicles), secretory granules, and even lysosomes represent vesicles. The endocytic vesicle, which arises at the plasma membrane, most prominently from clathrin-coated pits, first fuses with the early endosome, which is a major sorting compartment of approximately pH 6.5. A large part of the cargo and membranes internalized are recycled back to the plasma membrane through recycling vesicles (recycling pathway). Components that should be degraded are transported to the acidic late endosome (pH lower than 6) via multivesicular bodies. Lysosomes are vesicles that can store mature lysosomal enzymes and deliver them to a late endosomal compartment when needed. The resulting organelle is called the hybrid organelle or endolysosome. Lysosomes bud off the hybrid organelle in a process referred to as lysosome reformation. Late endosomes, lysosomes, and hybrid organelles are extremely dynamic organelles, and distinction between them is often difficult. Degradation of an endocytosed molecule occurs inside an endolysosome or lysosome. Endosomal escape is the active or passive release of a substance from the inner lumen of any kind of compartment or vesicle from the endocytic pathway, preferably from clathrin-mediated endocytosis, or recycling pathway into the cytosol. Endosomal escape thus includes but is not limited to release from endosomes, endolysosomes or lysosomes, including their intermediate and hybrid organelles.

Unless specifically indicated otherwise and in particular when relating to the endosomal escape mechanism of the glycoside molecule such as the saponin of the invention, whenever the word "endosome" or "endosomal escape" is used herein, it also includes the endolysosome and lysosome, and escape from the endolysosome and lysosome, respectively. After entering the cytosol, said substance might move to other cell units such as the nucleus.

In formal terms, a glycoside is any molecule in which a sugar group is bound through its anomeric carbon to another group via a glycosidic bond. Glycoside molecules, such as saponins, in the context of the invention are such molecules that are further able to enhance the effect of an effector moiety, without wishing to be bound by any theory, in particular by facilitating the endosomal escape of the effector moiety. Without wishing to be bound by any theory, the glycoside molecules (saponins, such as those listed in Table A1 and in Scheme I and those exemplified in the various embodiments) interact with the membranes of compartments and vesicles of the endocytic and recycling pathway and make them leaky for said effector moieties resulting in augmented endosomal escape. With the term "the scaffold is able to augment endosomal escape of the effector moiety" is meant that the at least one saponin (glycoside molecule), which is coupled to the polymeric or oligomeric structure of the scaffold, is able to enhance endosomal escape of a payload comprised by an effector moiety of the invention when both molecules are within an endosome, e.g. a late endosome, optionally and preferably after the at least one glycoside such as a saponin is released from the effector moiety such as from a linker or polymeric or oligomeric structure comprised by said effector moiety, e.g., by cleavage of a cleavable bond between the at least one glycoside (saponin) and the payload (for example via a polymeric or oligomeric structure of a scaffold and/or via a linker). Even though a bond between the at least one glycoside such as a saponin according to the invention and the payload comprised by the effector moiety of the invention, optionally via a linker or a scaffold, may be a "stable bond", that does not mean that such bond cannot be cleaved in the endosomes by, e.g., enzymes. For instance, the glycoside or saponin, optionally together with a linker or a part of the oligomeric or polymeric structure of a scaffold, may be cleaved off from the remaining linker fragment or oligomeric or polymeric structure. It could, for instance be that a protease cuts a (proteinaceous) linker or proteinaceous polymeric structure, e.g., albumin, thereby releasing the at least one glycoside, saponin. It is, however, preferred that the glycoside molecule (preferably saponin) is released in an active form, preferably in the original form that it had before it was (prepared to be) coupled to the payload comprised by the effector moiety of the invention, optionally via a linker and/or an oligomeric or polymeric scaffold; thus the glycoside (saponin) has its natural structure after such cleavage or the glycoside (saponin) has (part of) a chemical group or linker bound thereto, after such cleavage, while glycoside biological activity (saponin biological activity), e.g. endosomal/lysosomal escape enhancing activity towards a payload present in the same endosome or lysosome, is maintained or restored upon said cleavage of the bond between the glycoside (saponin) and e.g. the payload or a carrier molecule comprised by the effector moiety of the invention, optionally comprising a linker and/or a scaffold of the invention. With regard to the present invention the term "stable" with respect to bonds between e.g. saponins and amino-acid residues of the payload, a linker, a polymeric or oligomeric structures (of the scaffold), ligands, (monoclonal) immunoglobulins or binding domains or -fragments thereof, and/or furher effectors (effector moieties, effector molecules), is meant that the bond is not readily broken or at least not designed to be readily broken by, e.g., pH differences, salt concentrations, or UV-light, reductive conditions. With regard to the present invention the term "cleavable" with respect to bonds between e.g. saponins and the (proteinaceous) payload comprised by the effector moiety of the invention, linkers, amino-acid residues, polymeric or oligomeric structures of the scaffold, ligands, antibodies and/or further effectors, is meant that the bond is designed to be readily broken by, e.g., pH differences, salt concentrations, under reductive conditions, and the like. The skilled person is well aware of such cleavable bonds and how to prepare them.

Before the present invention one of the major hurdles of introducing ADCs and AOCs on the market was the small therapeutic window: a therapeutically effective dose of an ADC or an AOC is accompanied with (unacceptable) side effects, hampering development and implication in treatment of patients with the ADCs. By the application of the effector moiety of the invention as a semi-finished product for the manufacture of an ADC-saponin conjugate or an AOC-saponin conjugate, it has now become possible to guide one or multiple glycoside molecules (saponin) to a (target) cell, together with the ADC carrying a payload or together with a (monoclonal) antibody conjugated with an oligonucleotide such as a BNA according to the invention, thus the antibody being provided with covalently bound effector moiety of the invention which comprises covalently coupled saponin of the invention. In particular, it was previously not possible to specifically guide an effector moiety of a payload comprised by the effector moiety of the invention and a (predefined, controllable) particular number or range of glycoside molecules (saponins) per payload at the same time to the cytosol of cells, such as via the endocytic pathway of a cell. Now, the effector moiety of the invention can be covalently coupled to a monoclonal antibody for binding to an aberrant cell specific epitope such as a tumor-cell receptor that is specifically exposed on the surface of said tumor cell, such that the effector moiety of the invention is applied as a semi-finished product for providing an ADC-saponin conjugate or an AOC-saponin conjugate, according to the invention.

A solution provided for by the invention comprises the covalent binding of at least one saponin to a payload, therewith providing an effector moiety of the invention. A further solution provided for by the invention comprises (first) polymerizing the glycoside molecules (saponins) using an oligomeric or polymeric scaffold, and providing the payload comprised by the effector moiety of the invention with a cluster of covalently bound saponins, enabling re-monomerization of the one or more saponins at the intracellular site where the mode of action of the saponin is desired, e.g. after endocytosis. "Polymerizes" in this context means the reversible and/or irreversible multiple conjugation of saponin molecules to the payload molecule, either via linker, or directly or via a polymeric or oligomeric structure to form a scaffold or the reversible and/or irreversible multiple conjugation of (modified) saponins thereby forming a polymeric or oligomeric structure to form a scaffold. "Re-monomerization" in this context means the cleavage of the saponins from the payload molecule (e.g. a protein toxin, an antsense BNA), from the linker linking the saponin(s) to the payload molecule in the effector moiety of the invention or from the scaffold, for example after endocytosis, and regaining the (native) chemical state of the unbound saponins, which unbound saponins may or may not comprise additional chemical groups such as a chemical group for linking the saponin to a linker, an amino-acid residue of the payload or to the scaffold, and/or a (chemical) linker bound to a chemical group of the saponin such as an aldehyde group or carboxylic acid group. Due to the complex chemistry of the saponins for example the 'polymerization' of saponins at a scaffold or other linking linker and their 're-monomerization' at a desired location such as intracellularly e.g. after endocytosis, was a challenging task. In particular, the chemical reactions used for providing the linkers and the scaffold comprising covalently linked glycosides for covalent binding to the payload, e.g. triterpenoid saponins (polymerization of the glycosides), normally occur in water-free organic solvents, but saponins and for example biocompatible polymers applied as a scaffold for bearing bound saponins, are water-soluble molecules.

Without wishing to be bound by any theory, it is preferred to synchronize the presence of both, the at least one saponin, and the effector moiety, preferably a toxin or an oligonucleotide, in compartments or vesicles of the endocytic pathway of the target cell, e.g. a tumor cell or an auto-immune cell. With e.g. BNA and free saponin, synchronizing the presence of the molecules in the late endosomes, in order to obtain the synergistic effects *in vivo* cannot be beneficially obtained. In one aspect, the invention preferably solves at least the following problem with respect to combining the effector moiety comprised by the conjugate of the invention and the saponins comprised by the conjugate of the invention: without wishing to be bound by any theory the only reasonable chemical group within, e.g., the saponins that can be used for (covalent), in particular single and cleavable, retainable coupling is required for the endosomal escape activity. Known restrictions are most likely the reason why saponins have not been used in combination with pharmaceutically active substances in clinical investigations other than the application of saponins in vaccination regimes wherein the use of an immune-potentiating adjuvant substance was implied, although the striking endosomal escape enhancer effect of, e.g., saponins listed in Table A1 and Scheme I is known for more than 10 years. For example providing a conjugate of the invention with a covalently conjugated scaffold solves these difficulties, at least in part. Surprisingly, the saponins previously applied for their immune-potentiating activity in the vaccination context involving saponins as adjuvant component, are now also suitably for (covalent) coupling to ADCs or AOCs of the invention, for anti-tumor activity in vitro and in vivo.

An aspect of the invention relates to an antibody-drug conjugate comprising the effector moiety according to the invention, or a ligand-drug conjugate comprising the effector moiety of the invention, the effector moiety comprising covalently coupled saponin. The effector moiety of the invention is a conjugate comprising at least one saponin and at least one effector molecule, covalently coupled to each other, either directly, or via at least one linker, optionally comprising a cleavable linker, and optionally via an oligomeric or polymeric scaffold to which the at least one saponin and/or the at least one effector moiety are covalently bound.

An embodiment is the antibody-drug conjugate or ligand-drug conjugate of the invention, wherein the antibody can bind to any one of CD71, CA125, EpCAM(17-1A), CD52, CEA, CD44v6, FAP, EGF-IR, integrin, syndecan-1, vascular integrin alpha-V beta-3, HER2, EGFR, CD20, CD22, Folate receptor 1, CD146, CD56, CD19, CD138, CD27L receptor, PSMA, CanAg, integrin-alphaV, CA6, CD33, mesothelin, Cripto, CD3, CD30, CD239, CD70, CD123, CD352, DLL3, CD25, ephrinA4, MUC1, Trop2, CEACAM5, CEACAM6, HER3, CD74, PTK7, Notch3, FGF2, C4.4A, FLT3, CD38, FGFR3, CD7, PD-L1, CTLA4, CD52, PDGFRA, VEGFR1, VEGFR2, preferably CD71, HER2, EGFR, and/or wherein the antibody of the antibody-drug conjugate is or comprises any one of cetuximab, daratumumab, gemtuzumab, trastuzumab, panitumumab, brentuximab, inotuzumab, moxetumomab, polatuzumab, obinutuzumab, OKT-9 anti-CD71 monoclonal antibody of the IgG type, pertuzumab, rituximab, ofatumumab, Herceptin, alemtuzumab, pinatuzumab, OKT-10 anti-CD38 monoclonal antibody, an antibody of Table A2 or Table A3 or Table A4, preferably cetuximab or trastuzumab or OKT-9, or at least one tumor-cell specific receptor binding-fragment thereof and/or at least one tumor-cell specific receptor binding-domain thereof, and/or wherein the antibody-drug conjugate comprises any one of Gemtuzumab ozogamicin, Brentuximab vedotin, Trastuzumab emtansine, Inotuzumab ozogamicin, Moxetumomab pasudotox and Polatuzumab vedotin and an antibody-drug conjugate of Table A2 and Table A3, and/or wherein the ligand-drug conjugate comprises or consists of at least one non-proteinaceous ligand and/or at least one proteinaceous ligand for binding to a cell-surface molecule such as EGF or a cytokine.

An aspect of the invention relates to a therapeutic combination comprising: (a) the effector moiety of the invention, comprising at least one saponin, and optionally a pharmaceutically acceptable excipient; and (b) an antibody-drug conjugate or a ligand-drug conjugate, and optionally a pharmaceutically acceptable excipient.

An embodiment is the therapeutic combination of the invention, wherein the antibody-drug conjugate can bind to any one of tumor-cell receptors CD71, CA125, EpCAM(17-1A), CD52, CEA, CD44v6, FAP, EGF-IR, integrin, syndecan-1, vascular integrin alpha-V beta-3, HER2, EGFR, CD20, CD22, Folate receptor 1, CD146, CD56, CD19, CD138, CD27L receptor, PSMA, CanAg, integrin-alphaV, CA6, CD33, mesothelin, Cripto, CD3, CD30, CD239, CD70, CD123, CD352, DLL3, CD25, ephrinA4, MUC1, Trop2, CEACAM5, CEACAM6, HER3, CD74, PTK7, Notch3, FGF2, C4.4A, FLT3, CD38, FGFR3, CD7, PD-L1, CTLA4, CD52, PDGFRA, VEGFR1, VEGFR2, preferably CD71, HER2, EGFR, and/or wherein the antibody of the antibody-drug conjugate is or comprises any one of cetuximab, daratumumab, gemtuzumab, trastuzumab, panitumumab, brentuximab, inotuzumab, moxetumomab, polatuzumab, obinutuzumab, OKT-9 anti-CD71 monoclonal antibody of the IgG type, pertuzumab, rituximab, ofatumumab, Herceptin, alemtuzumab, pinatuzumab, OKT-10 anti-CD38 monoclonal antibody, an antibody of Table A2 or Table A3 or Table A4, preferably cetuximab or trastuzumab or OKT-9, or at least one tumor-cell specific receptor binding-fragment thereof and/or at least one tumor-cell specific receptor binding-domain thereof, and/or wherein the antibody-drug conjugate comprises any one of Gemtuzumab ozogamicin, Brentuximab vedotin, Trastuzumab emtansine, Inotuzumab ozogamicin, Moxetumomab pasudotox and Polatuzumab vedotin and an antibody-drug conjugate of Table A2 and Table A3, and/or wherein the ligand-drug conjugate comprises or consists of at least one non-proteinaceous ligand and/or at least one proteinaceous ligand for binding to a cell-surface molecule such as EGF or a cytokine.

An effector moiety useful in the present invention preferably relies on late endosomal escape for exerting its effect. Some effectors, such as, e.g., a pseudomonas exotoxin, are rerouted to other organelles prior to the "late endosomal stage" and, thus, would normally not benefit from coupling to the second proteinaceous molecule according to the present invention. However, such toxin may be adapted for use with the present invention, e.g., by deleting the signal peptide responsible rerouting. In particular toxins that are highly toxic and would require only one molecule to escape the endosomes to kill a cell maybe modified to be less potent. It is preferred to use a toxin that kills a cell if at least 2, more preferably at least 5, more preferably at least 10, more preferably at least 20, more preferably at least 50, most preferably at least 100 toxin molecules escape the endosome. It is further preferred that a conjugate of the invention comprising linked effector moiety - saponin, comprises a covalently conjugated functionalized scaffold, i.e. a scaffold comprising covalently bound effector moietie(s) for delivering the scaffold comprising the bound effector moietie(s) at a target cell such as a tumor cell or an auto-immune cell, together with the covalently bound saponin. Further, in order to reduce off-target toxicity, cell membrane non-permeable small molecule toxins are preferred effector molecules over cell membrane permeable toxins.

The term "ligand" as used in this invention has its ordinary meaning and preferably means a molecule or structure that is able to bind another molecule or structure on the cell surface of a target cell, wherein said molecule or structure on the cell surface can be endocytosed and is preferably absent or less prominent on off-target cells. Preferably, said molecule or structure on the cell surface is constitutively endocytosed. More preferably a ligand in this invention induces endocytosis of said molecule or structure on the cell surface of target cells after binding to said molecule or structure. This is for instance the case for Epidermal Growth Factor Receptor (EGFR), present on the surface of a variety of cancer cells. Examples of molecules or structures on the cell surface of target cells that are constitutively endocytosed, are for instance Claudin-1 or major histocompatibility complex class glycoproteins. A ligand can, e.g., be an antibody, a growth factor or a cytokine. Combining in e.g. a carrier molecule an effector moiety of the invention, e.g. a toxin coupled to a saponin, with a ligand is one possibility to create a targeted toxin. A toxin that is only toxic in a target cell because it interferes with processes that occur in target cells only can also be seen as a targeted toxin (as in off-target cells it cannot exert its toxic action, e.g. apoptin). Preferably, a targeted toxin is a toxin that is combined with a ligand or e.g. a monoclonal antibody in order to be active in target cells and not in off-target cells (as it is only bound to and endocytosed by target cells). In for example a functionalized scaffold comprising a carrier molecule comprising a ligand and an effector moiety of the invention, the ligand or the monoclonal antibody guides the effector moiety with the saponin bound thereto, and scaffold to the target cells. After internalization, the at least one glycoside, preferably a saponin comprised by the effector moiety of the invention, mediates the endosomal escape of the effector moiety. The saponin is typically a saponin listed in Table A1 and Scheme I, and preferably the saponin is SO1861 and/or QS-21, and/or SA1641 and/or GE1741.

A payload, and an effector molecule, or effector moiety of the invention, in the context of this invention is any substance that affects the metabolism of a cell by interaction with an intracellular effector molecule target, wherein this effector molecule target is any molecule or structure inside cells excluding the lumen of compartments and vesicles of the endocytic and recycling pathway but including the membranes of these compartments and vesicles. Said structures inside cells thus include the nucleus, mitochondria, chloroplasts, endoplasmic reticulum, Golgi apparatus, other transport vesicles, the inner part of the plasma membrane and the cytosol. Cytosolic delivery of a payload or an effector moiety in the context of the invention preferably means that the payload or the effector moiety is able to escape the endosome (and/or lysosome), which, as defined previously, also includes escaping the endolysosome and the lysosome, and is preferably able to reach the payload orthe effector moiety target as described herein. The invention also encompasses a new type of molecule, referred to as scaffold that serves to bring both a payload and at least one glycoside molecule such as a saponin, or an effector moiety of the invention in an endosome at the same time in a pre-defined ratio, when the effector moiety comprises the payload and the saponin. Within the context of the present invention, the polymeric or oligomeric structure of the scaffold is a structurally ordered formation such as a polymer, oligomer, dendrimer, dendronized polymer, or dendronized oligomer or it is an assembled polymeric structure such as a hydrogel, microgel, nanogel, stabilized polymeric micelle or liposome, but excludes structures that are composed of non-covalent assemblies of monomers such as cholesterol/phospholipid mixtures. The terms "polymer, oligomer, dendrimer, dendronized polymer, or dendronized oligomer" have their ordinary meaning. In particular a polymer is a substance which has a molecular structure built up chiefly or completely from a large number of equal or similar units bonded together and an oligomer is a polymer whose molecules consist of relatively few repeating units. There is no consensus about one specific cutoff for "many" and "a few" as used in the above definition of polymer and oligomer, respectively. However, as the scaffold may comprise a polymeric or an oligomeric structure, or both, the full range of numbers of similar units bonded together applies to such structure, i.e. from 2 monomeric units to 100 monomeric units, 1000 monomeric units, and more. A structure comprising 5 or less, for instance maybe called an oligomeric structure, whereas a structure comprising 50 monomeric units maybe called a polymeric structure. A structure of 10 monomeric units maybe called either oligomeric or polymeric. A scaffold as defined herein, further comprises at least one glycoside molecule such as a saponin of the invention. A scaffold preferably includes a polymeric or oligomeric structure such as poly- or oligo(amines), e.g., polyethylenimine and poly(amidoamine), and biocompatible structures such as polyethylene glycol, poly- or oligo(esters), such as poly(lactids), poly(lactams), polylactide-co-glycolide copolymers, and poly(dextrin), poly- or oligosaccharides, such as cyclodextrin or polydextrose, and poly- or oligoamino acids, such as poly-lysine or a peptide or a protein, or DNA oligo- or polymers. An assembled polymeric structure as defined herein comprises at least one scaffold and, optionally, other individual polymeric or oligomeric structures. Other individual polymeric or oligomeric structures of said assembly may be (a) scaffolds (thus comprising at least one glycoside molecule such as a saponin of the invention), (b) functionalized scaffolds (thus comprising at least one glycoside molecule such as a saponin, and a ligand, antibody, etc., (c) polymeric or oligomeric structures without a glycoside molecule such as a saponin of the invention (See Table A1 for example), without a ligand, antibody, etc.. A functionalized assembled polymeric structure is an assembled polymeric structure that contains (a) at least one functionalized scaffold or (b) at least one scaffold and at least one polymeric structure comprising at least one ligand, antibody, etc.. Polymeric or oligomeric structures within an assembled polymeric structure that do not comprise any of the above mentioned molecules (i.e. no glycosides such as saponins, no first proteinaceous molecule such as ligands, antibodies) are in particular added as structural components of the assembled structures, which help to build up or to stabilize the assembled structure ("glue-like").Without wishing to be bound by any theory, the acidic environment seems to be a prerequisite for the synergistic action between glycoside (saponin) and effector moiety.

A pharmaceutically active substance in this invention is an effector moiety that is used to achieve a beneficial outcome in an organism, preferably a vertebrate, more preferably a human being such as a cancer patient or an auto-immune patient. Benefit includes diagnosis, prognosis, treatment, cure and/or prevention of diseases and/or symptoms. The pharmaceutically active substance may also lead to undesired harmful side effects. In this case, pros and cons must be weighed to decide whether the pharmaceutically active substance is suitable in the particular case. If the effect of the pharmaceutically active substance inside a cell is predominantly beneficial for the whole organism, the cell is called a target cell. If the effect inside a cell is predominantly harmful for the whole organism, the cell is called an off-target cell. In artificial systems such as cell cultures and bioreactors, target cells and off-target cells depend on the purpose and are defined by the user.

An effector moiety that is a polypeptide may be, e.g., a polypeptide that recover a lost function, such as for instance enzyme replacement, gene regulating functions, or a toxin.

Preferably, the effector moiety, which effect is enhanced by the saponins bound thereto, detaches from the saponin and/or e.g. an antibody, when endocytosed. This can be achieved by a cleavable bond that breaks, e.g. under acidic, reductive, enzymatic or light-induced conditions.

An aspect of the invention relates to a pharmaceutical composition comprising the effector moiety of the invention or the antibody-drug conjugate of the invention or the ligand-drug conjugate of the invention, comprising at least one saponin covalently linked to the effector molecule, and optionally a pharmaceutically acceptable excipient.

An aspect of the invention relates to the effector moiety of the invention or the antibody-drug conjugate of the invention or the therapeutic combination of the invention or the ligand-drug conjugate of the invention or the pharmaceutical composition of the invention, for use as a medicament.

An aspect of the invention relates to the effector moiety of the invention or the antibody-drug conjugate of the invention or the therapeutic combination of the invention or the ligand-drug conjugate of the invention orthe pharmaceutical composition of the invention, for use in the treatment or prevention of a cancer or an autoimmune disease.

The inventors disclose here that covalently coupling saponins such as saponins in the water-soluble fraction of Quillaja saponaria, QS-21, SA1641, SO1861, Table A1, Scheme I, to an effector moiety such as an oligonucleotide, e.g. an antisense BNA, such as via a tri-functional linker, e.g. the tri-functional linker of Scheme II, or via an oligomeric or polymeric structure of a scaffold, such as a dendron, e.g. a G4-dendron, comprising covalently bound saponins, results in improved cell toxicity exerted by the effector moiety such as a toxin and preferably an antisense oligonucleotide such as an antisense BNA, comprised by the conjugate, under influence of the covalently coupled saponin in the conjugate, i.e. the effector moiety of the invention. In particular, the effector moiety of the invention is an antisense oligonucleotide covalently conjugated with a dendron, which has multiple saponins covalently bound to it, such as 2, 4, 8, 16 saponins, preferably 4 saponins.

An embodiment is the conjugate of the invention comprising the effector moiety and a saponin comprising one or several or all of the indicated structural features of the saponin of Structure A in Scheme I, the saponin of structure A referred to as a saponin with an `ideal' structure when endosomal escape enhancing activity towards an effector moiety present in the endosome of a cell contacted with conjugate of the invention, and/or a saponin selected from any one or more of the further saponins in Scheme I:

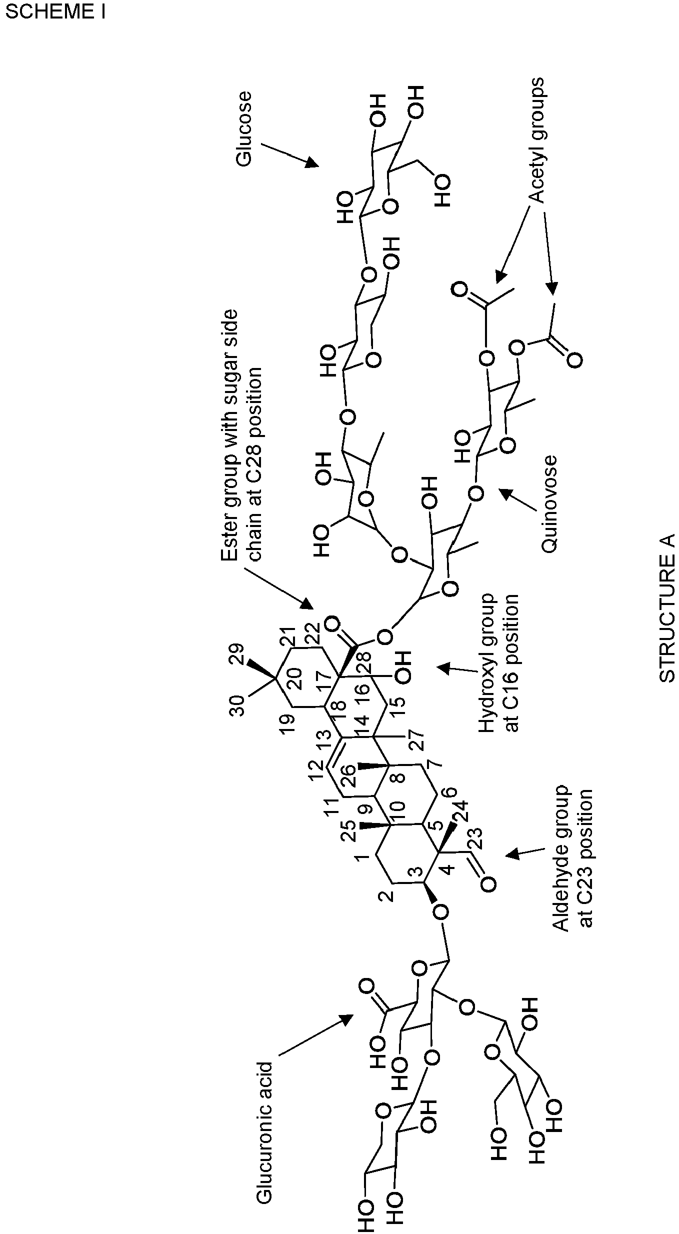

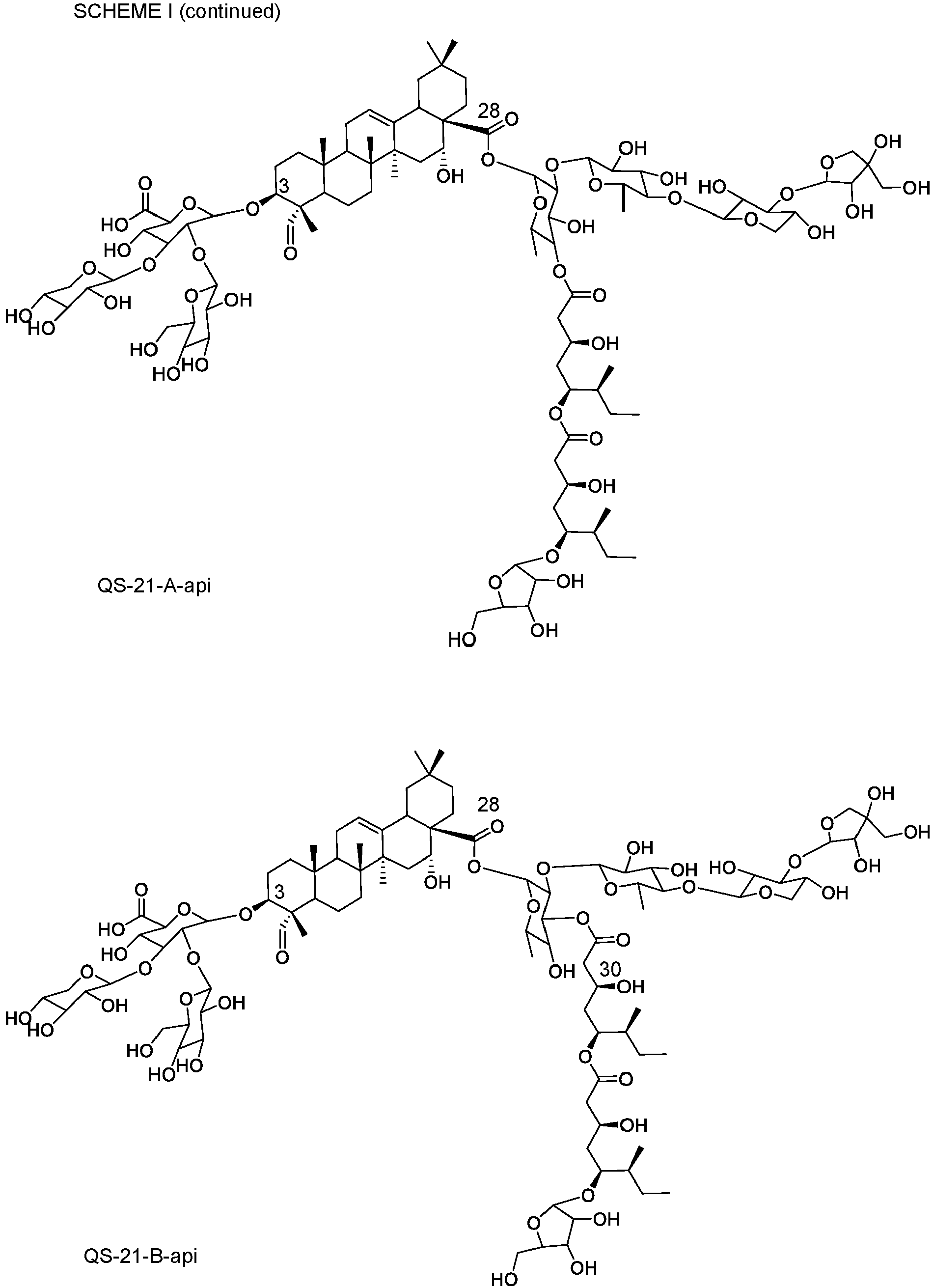

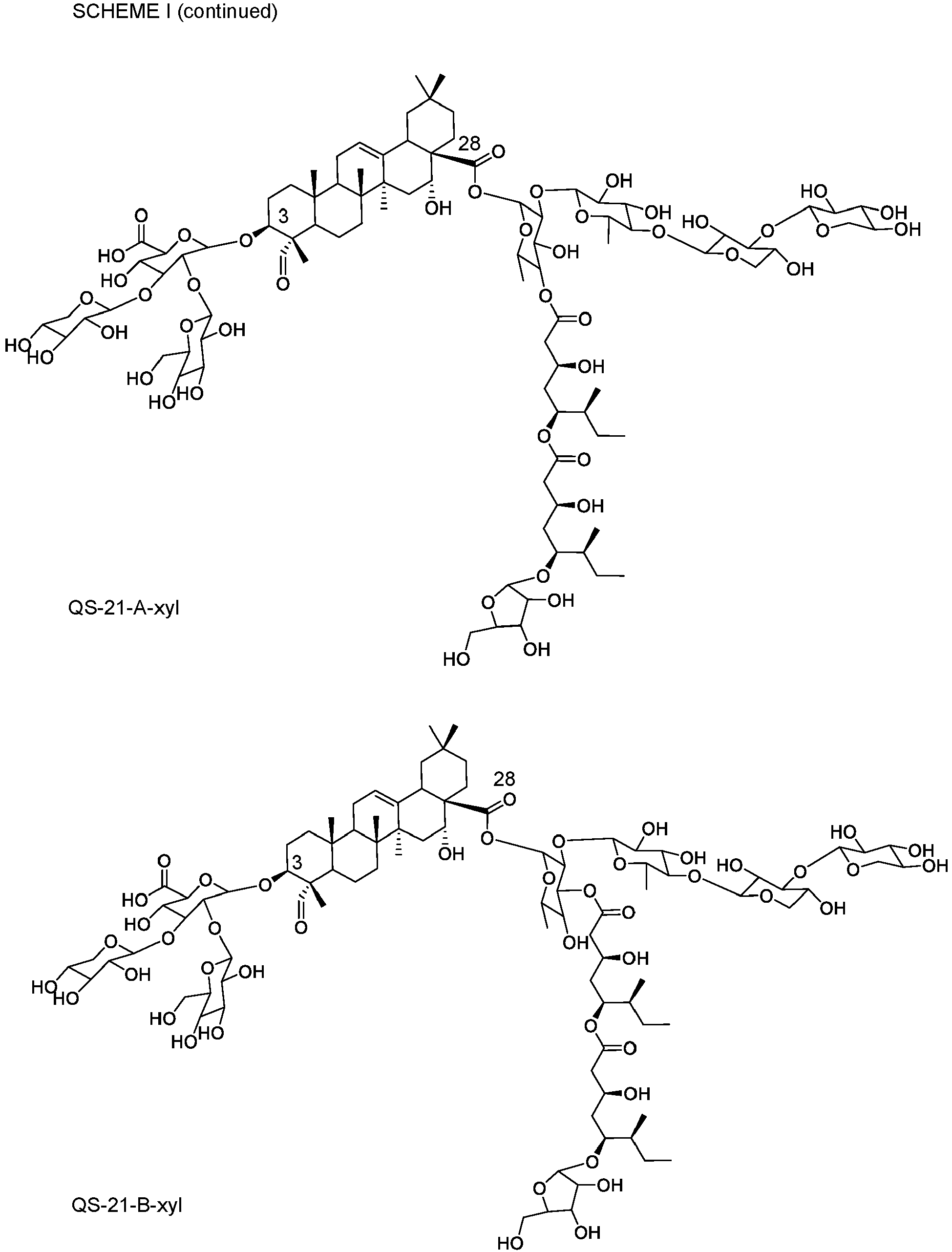

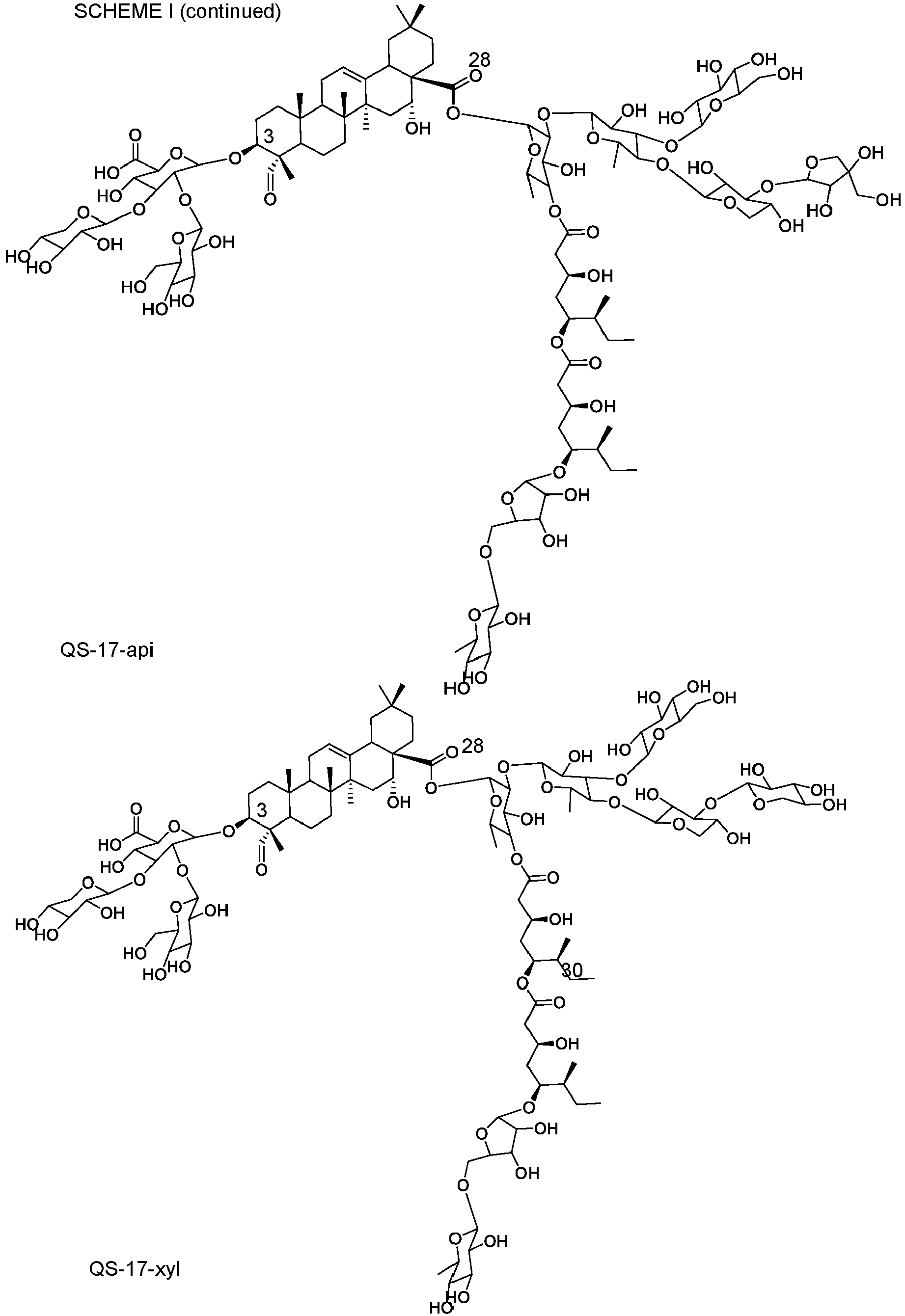

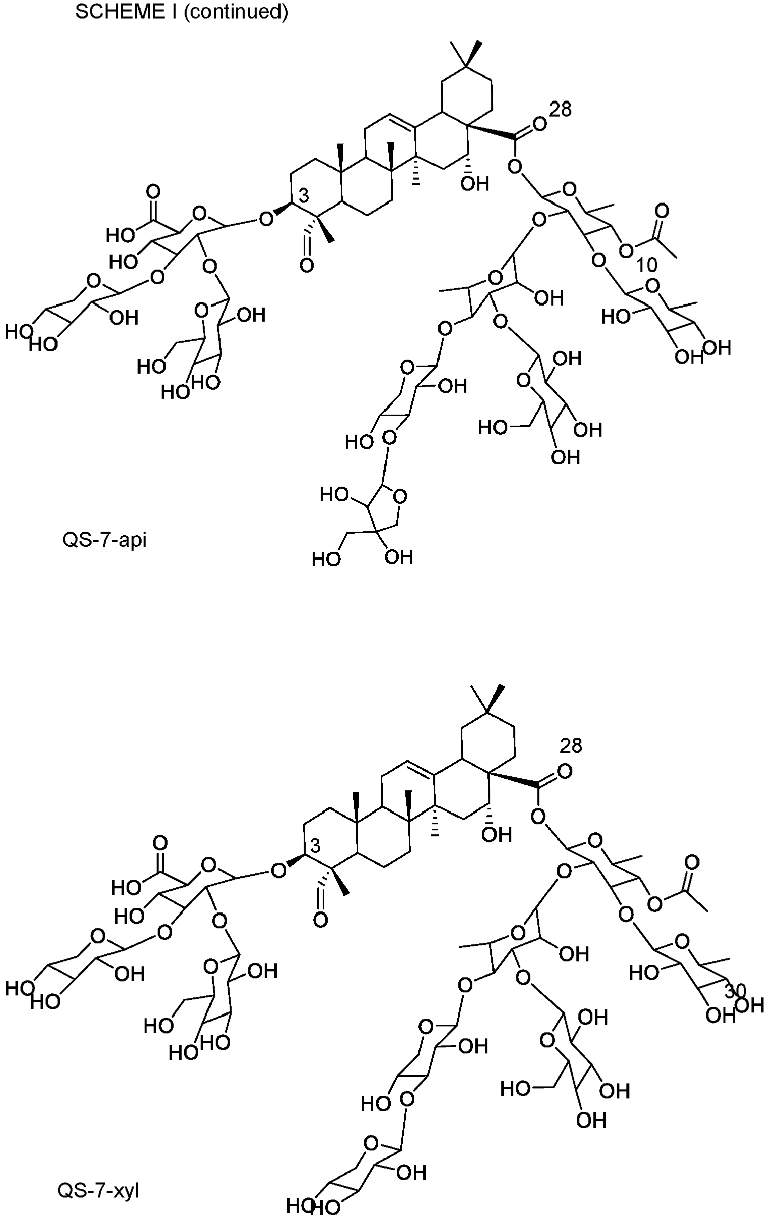

According to the invention, a glycoside, such as a saponin according to the invention, bound to the effector moiety comprised by the conjugate of the invention, which has the 'ideal' structure for the purpose of enhancing endosomal escape of an effector molecule comprised by the conjugate of the invention is a bisdesmosidic saponin according to Structure A of Scheme I, having a molecular mass of at least 1.500 Dalton and comprising an oleanan-type triterpene containing an aldehyde group at the C-23 position and optionally a hydroxyl group at the C-16 position, with a first branched carbohydrate side chain at the C-3 position which first branched carbohydrate side chain optionally contains glucuronic acid, wherein the saponin contains an ester group with a second branched carbohydrate side chain at the C-28 position which second branched carbohydrate chain preferably comprises at least four carbohydrate units, optionally containing at least one acetyl residue such as two acetyl residues and/or optionally comprising deoxy carbohydrates and/or optionally comprising quinovose and/or optionally comprising glucose and/or optionally comprising 4-methoxycinnamic acid and/or optionally comprising 5-O-[5-O-Ara/Api-3,5-dihydroxy-6-methyl-octanoyl]-3,5-dihydroxy-6-methyl-octanoic acid and/or optionally comprising 5-O-[5-O-Rha-(1→2)-Ara/Api-3,5-dihydroxy-6-methyl-octanoyl]-3,5-dihydroxy-6-methyl-octanoic acid bound to a carbohydrate via an ester bond.

SO1861 is different from the "*ideal structure*" displayed in Scheme I, Structure A, only in having only one acetyl residue at the quinovose and having an additional xylose. The "ideal structure" of a saponin for enhancing endosomal escape of an effector molecule or effector moiety, such as an antisense oligonucleotide such as a BNA, is a saponin which preferably has the Structure A of Scheme I, and saponins which display the endosomal escape enhancing activity have one or more of the structural features displayed in Structure A of Scheme I. Without wishing to be bound by any theory, the inventors belief that the Structure A of Scheme I represents an "ideal saponin" (and not a minimum requirement saponin) for endosomal escape enhancing activity, which means that not all of the structures (chemical groups) can or must be present in each saponin with at least sufficient endosomal escape enhancing activity to promote accumulation of the effector moiety in the cytosol, and which means that some saponins might have other structure elements such as acyl chains, and/or for yet other saponins that display endosomal escape enhancing activity, the sugars can be different than the sugars displayed in Scheme I. For example, the QS-21 saponin and some of the saponins in the water soluble fraction of Quillaja saponaria (Quillaja saponins; Quil-A) differ in the carbohydrate modification at C-28 when the ideal structure of Structure A in Scheme I is considered: presence of an acyl chain in QS-21 for example. In the water soluble fraction of Quillaja saponaria, saponins such as QS-7, QS1862, are similar to the ideal Structure A, and are similar to SO1861.

An embodiment is effector moiety of the invention, comprising the oligomeric tri-functional linker as the scaffold core structure, according to Scheme II: 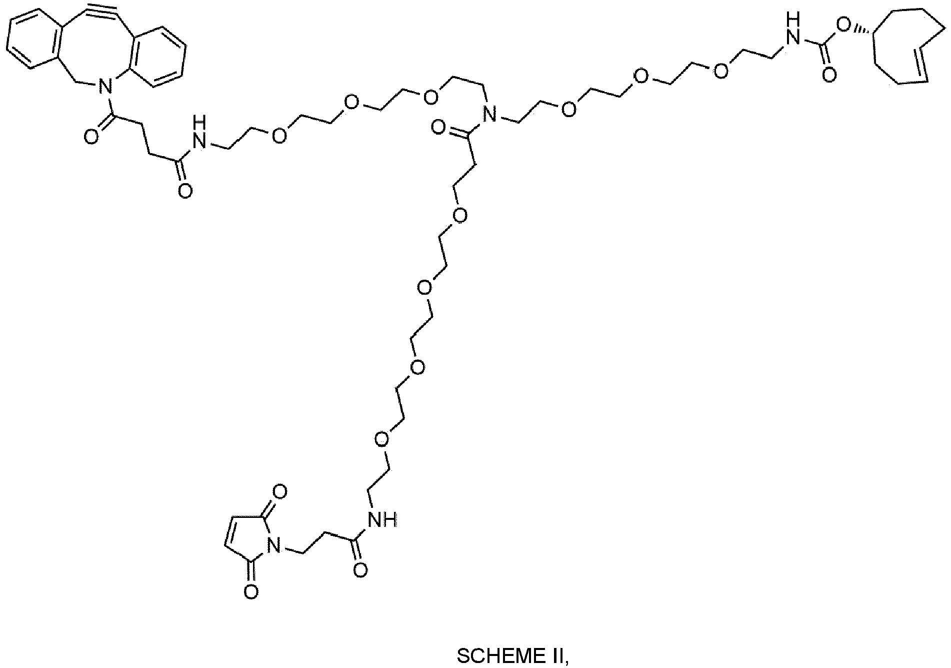 wherein the at least one saponin is covalently bound to the tri-functional linker scaffold via a labile, cleavable hydrazone linker (acid sensitive) and/or via a maleimide comprising bond, whereas the binding of the scaffold to the effector moiety such as an oligonucleotide is established via a labile, cleavable hydrazone linker (acid sensitive) and/or via a maleimide comprising bond with cysteines in the binding site, therewith forming Structure B: 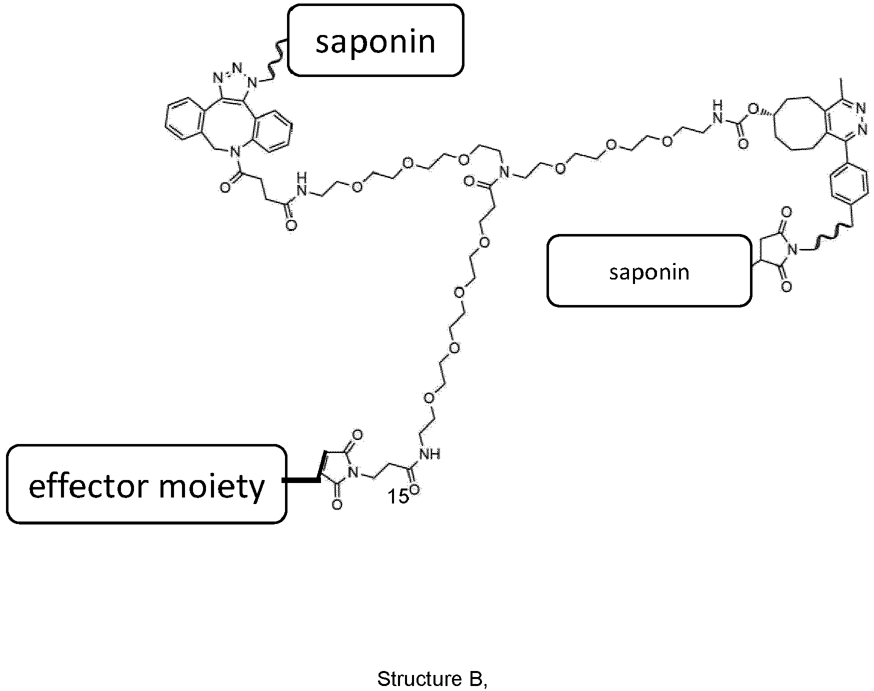 such as 1, 2, 3 or 4 cysteines such that 1-4 scaffolds are covalently bound to a single effector moiety.

| TABLE A1. Saponins displaying (late) endosomal/lysosomal escape enhancing activity, and saponins comprising a structure reminiscent to such saponins displaying (late) endosomal/lysosomal escape enhancing activity | | | |
|---|---|---|---|
| **Saponin Name** | **Aglycon core** | **Carbohydrate substituent at the C-3beta-OH group** | **Carbohydrate substituent at the C-28-OH group** |
| NP-005236 | 2alpha-Hydroxyoleanolic acid | GlcA- | Glc/Gal- |
| AMA-1 | 16alpha-Hydroxyoleanolic acid | Glc- | Rha-(1→2)-[Xyl-(1→4)]-Rha- |
| AMR | 16alpha-Hydroxyoleanolic acid | Glc- | Rha-(1→2)-[Ara-(1→3)-Xyl-(1→4)]-Rha- |
| alpha-Hederin | Hederagenin (23-Hydroxyoleanolic acid) | Rha-(1→2)-Ara- | - |
| NP-012672 | 16alpha,23- Dihydroxyoleanolic acid | Ara/Xyl-(1→4)-Rha/Fuc- (1→2)-Glc/Gal-(1→2)- Rha/Fuc-(1→2)-GlcA- | Ara/Xyl- |
| NP-017777 | Gypsogenin | Gal-(1→2)-[Xyl-(1→3)]-GlcA- | Xyl-(1→4)-Rha-(1→2)-[R-1→4)]-Fuc- (R = 4E- Methoxycinnamic acid) |
| NP-017778 | Gypsogenin | Gal-(1→2)-[Xyl-(1→3)]-GlcA- | Xyl-(1→4)-Rha-(1→2)-[R-1→4)]-Fuc- (R = 4Z- Methoxycinnamic acid) |
| NP-017774 | Gypsogenin | Gal-(1→2)-[Xyl-(1→3)]-GlcA- | Xyl-(1→4)-[Gal-(1→3)]-Rha-(1→2)-4-OAc- Fuc- |
| NP-018110^{c}, NP-017772^{d} | Gypsogenin | Gal-(1→2)-[Xyl-(1→3)]-GlcA- | Xyl-(1→4)-[Glc-(1→3)]-Rha-(1→2)-3,4-di- OAc-Fuc- |
| NP-018109 | Gypsogenin | Gal-(1→2)-[Xyl-(13)]-GlcA- | Xyl-(1→4)-[Glc-(1→3)]-Rha-(1→2)-[R-(→4)]- 3-OAc-Fuc- (R = 4E-Methoxycinnamic acid) |
| NP-017888 | Gypsogenin | Gal-(1→2)-[Xyl-(1→3)]-GlcA- | Glc-(1→3)-Xyl-(1→4)-[Glc-(1→3)]-Rha- (1->2)-4-OAc-Fuc- |
| NP-017889 | Gypsogenin | Gal-(1→2)-[Xyl-(1→3)]-GlcA- | Glc-(1→3)-Xyl-(1→4)-Rha-(1→2)-4-OAc-Fuc- |
| NP-018108 | Gypsogenin | Gal-(1→2)-[Xyl-(1→3)]-GlcA- | Ara/Xyl-(1→3)-Ara/Xyl-(1→4)-Rha/Fuc- (1→2)-[4-OAc-Rha/Fuc-(1→4)]-Rha/Fuc- |
| SA1641^{a}, AE X55^{b} | Gypsogenin | Gal-(1→2)-[Xyl-(1→3)]-GlcA- | Xyl-(1→3)-Xyl-(1→4)-Rha-(1→2)-[Qui- (1→4)]-Fuc- |
| NP-017674 | Quillaic acid | Gal-(1→2)-[Xyl-(1→3)]-GlcA- | Api-(1→3)-Xyl-(1→4)-[Glc-(1→3)]-Rha- (1→2)-Fuc- |
| NP-017810 | Quillaic acid | Gal-(1→2)-[Xyl-(1→3)]-GlcA- | Xyl-(1→4)-[Gal-(1→3)]-Rha-(1→2)-Fuc- |
| AG1 | Quillaic acid | Gal-(1→2)-[Xyl-(1→3)]-GlcA- | Xyl-(1→4)-[Glc-(1→3)]-Rha-(1→2)-Fuc- |
| NP-003881 | Quillaic acid | Gal-(1→2)-[Xyl-(1→3)]-GlcA- | Ara/Xyl-(1→4)-Rha/Fuc-(1→4)-[Glc/Gal-(1→2)]-Fuc- |
| NP-017676 | Quillaic acid | Gal-(1→2)-[Xyl-(1→3)]-GlcA- | Api-(1→3)-Xyl-(1→4)-[Glc-(1→3)]-Rha-(1→2)-[R-(→4)]-Fuc- (R = 5-O-[5-O-Ara/Api-3,5-dihydroxy-6-methyl-octanoyl]-3,5-dihydroxy-6-methyl-octanoic acid) |
| NP-017677 | Quillaic acid | Gal-(1→2)-[Xyl-(1→3)]-GlcA- | Api-(1→3)-Xyl-(1→4)-Rha-(1→2)-[R-(→4)]- Fuc- (R = 5-O-[5-O-Ara/Api-3,5-dihydroxy-6- methyl-octanoyl]-3,5-dihydroxy-6-methyl- octanoic acid) |
| NP-017706 | Quillaic acid | Gal-(1→2)-[Xyl-(1→3)]-GlcA- | Api-(1→3)-Xyl-(1→4)-Rha-(1→2)-[Rha- (1->3)]-4-OAc-Fuc- |
| NP-017705 | Quillaic acid | Gal-(12)-[Xyl-(1→3)]-GlcA- | Api-(1→3)-Xyl-(1→4)-[Glc-(1→3)]-Rha- (1→2)-[Rha-(1→3)]-4-OAc-Fuc- |
| NP-017773 | Quillaic acid | Gal-(1→2)-[Xyl-(1→3)]-GlcA- | 6-OAc-Glc-(1→3)-Xyl-(1→4)-Rha-(1→2)-[3- OAc-Rha-(1→3)]-Fuc- |
| NP-017775 | Quillaic acid | Gal-(1→2)-[Xyl-(1→3)]-GlcA- | Glc-(1→3)-Xyl-(1→4)-Rha-(1→2)-[3-OAc-- Rha-(1→3)]-Fuc- |
| SA1657 | Quillaic acid | Gal-(1→2)-[Xyl-(1→3)]-GlcA- | Xyl-(1→3)-Xyl-(1→4)-Rha-(1→2)-[Qui- (1→4)]-Fuc- |
| AG2 | Quillaic acid | Gal-(1→2)-[Xyl-(1→3)]-GlcA- | Glc-(1→3)-[Xyl-(1→4)]-Rha-(1→2)-[Qui-(1→4)]-Fuc- |
| SO1861 | Quillaic acid | Gal-(1→2)-[Xyl-(1→3)]-GlcA- | Glc-(1→3)-Xyl-(1→4)-Rha-(1→2)-[Xyl-(1→3)-4-OAc-Qui-(1→4)]-Fuc- |
| GE1741 | Quillaic acid | Gal-(1→2)-[Xyl-(1→3)]-GlcA- | Xyl-(1→3)-Xyl-(1→4)-Rha-(1→2)-[3,4-di-OAc-Qui-(1→4)]-Fuc- |
| SO1542 | Quillaic acid | Gal-(1→2)-[Xyl-(1→3)]-GlcA- | Glc-(1→3)-[Xyl-(1→4)]-Rha-(1→2)-Fuc- |
| SO1584 | Quillaic acid | Gal-(1→2)-[Xyl-(1→3)]-GlcA- | 6-OAc-Glc-(1→3)-[Xyl-(1→4)]-Rha-(1→2)-Fuc- |
| SO1658 | Gypsogenin | Gal-(1→2)-[Xyl-(1→3)]-GlcA- | Glc-(1→3)-[Xyl-(1→3)-Xyl-(1→4)]-Rha-(1→2)-Fuc- |
| SO1674 | Quillaic acid | Gal-(1→2)-[Xyl-(1→3)]-GlcA- | Glc-(1→3)-[Xyl-(1→3)-Xyl-(1→4)]-Rha-(1→2)-Fuc- |
| SO1832 | Quillaic acid | Gal-(1→2)-[Xyl-(1→3)]-GlcA- | Xyl-(1→3)-Xyl-(1→4)-Rha-(1→2)-[Xyl-(1→3)-4-OAc-Qui-(1→4)]-Fuc- |
| QS-7 (also referred to as QS1861) | Quillaic acid | Gal-(1→2)-[Xyl-(1→3)]-GlcA- | Api/Xyl-(1→3)-Xyl-(1→4)-[Glc-(1→3)]-Rha-(1→2)-[Rha-(1→3)]-4OAc-Fuc- |
| QS-7 api (also referred to as QS1862) | Quillaic acid | Gal-(1→2)-[Xyl-(1→3)]-GlcA- | Api-(1→3)-Xyl-(1→4)-[Glc-(1→3)]-Rha-(1→2)-[Rha-(1→3)]-4OAc-Fuc- |
| QS-17 | Quillaic acid | Gal-(1→2)-[Xyl-(1→3)]-GlcA- | Api/Xyl-(1→3)-Xyl-(1→4)-[Glc-(1→3)]-Rha-(1→2)-[R-(→)]-Fuc-(R = 5-O-[5-O-Rha-(1→2)-Ara/Api-3,5-dihydroxy-6-methyl-octanoyl]-3,5-dihydroxy-6-methyl-octanoic acid) |
| QS-18 | Quillaic acid | Gal-(1→2)-[Xyl-(1→3)]-GlcA- | Api/Xyl-(1→3)-Xyl-(1→4)-[Glc-(1→3)]-Rha-(1→2)-[R-(→4)]-Fuc-(R = 5-0-[5-0-Ara/Api-3,5-dihydroxy-6-methyl-octanoyl]-3,5-dihydroxy-6-methyl-octanoic acid) |
| QS-21 A-apio | Quillaic acid | Gal-(1→2)-[Xyl-(1→3)]-GlcA- | Api-(1→3)-Xyl-(1→4)-Rha-(1→2)-[R-(→4)]-Fuc-(R = 5-0-[5-0-Ara/Api-3,5-dihydroxy-6-methyl-octanoyl]-3,5-dihydroxy-6-methyl-octanoic acid) |
| QS-21 A-xylo | Quillaic acid | Gal-(1→2)-[Xyl-(1→3)]-GlcA- | Xyl-(1→3)-Xyl-(1→4)-Rha-(1→2)-[R-(→4)]-Fuc-(R = 5-0-[5-0-Ara/Api-3,5-dihydroxy-6-methyl-octanoyl]-3,5-dihydroxy-6-methyl-octanoic acid) |
| QS-21 B-apio | Quillaic acid | Gal-(1→2)-[Xyl-(1→3)]-GlcA- | Api-(1→3)-Xyl-(1→4)-Rha-(1→2)-[R-(→3)]-Fuc-(R = 5-0-[5-0-Ara/Api-3,5-dihydroxy-6-methyl-octanoyl]-3,5-dihydroxy-6-methyl-octanoic acid) |
| QS-21 B-xylo | Quillaic acid | Gal-(1→2)-[Xyl-(1→3)]-GlcA- | Xyl-(1→3)-Xyl-(1→4)-Rha-(1→2)-[R-(→3)]-Fuc-(R = 5-0-[5-0-Ara/Api-3,5-dihydroxy-6-methyl-octanoyl]-3,5-dihydroxy-6-methyl-octanoic acid) |
| beta-Aescin (described: Aescin la) | Protoaescigenin-21 (2-methylbut-2-enoate)-22-acetat | Glc-(1→2)-[Glc-(1→4)]-GlcA- | - |
| Teaseed saponin I | 23-Oxo-barringtogenol C - 21,22-bis(2-methylbut-2-enoate) | Glc-(1→2)-Ara-(1→3)-[Gal-(1→2)]-GlcA- | - |
| Teaseedsaponin J | 23-Oxo-barringtogenol C - 21,22-bis(2-methylbut-2-enoate) | Xyl-(1→2)-Ara-(1→3)-[Gal-(1→2)]-GlcA- | - |
| Assamsaponin F | 23-Oxo-barringtogenol C - 21 (2-methylbut-2-enoate)-16,22-diacetat | Glc-(1→2)-Ara-(1→3)-[Gal-(1→2)]-GlcA- | - |
| Digitonin | Digitogenin | Glc-(1→3)-Gal-(1→2)-[Xyl-(1→3)]-Glc-(1→4)-Gal- | - |
| Primula acid 1 | 3,16,28-Trihydroxyoleanan-12-en | Rha-(1→2)-Gal-(1→3)-[Glc-(1→2)]-GlcA- | - |
| AS64R | Gypsogenic acid | - | Glc-(1→3)-[Glc-(1→6)]-Gal- |

| | | **Carbohydrate substituent at the C-23-OH group** | |
|---|---|---|---|
| AS6.2 | Gypsogenic acid | Gal- | Glc-(1→3)-[Glc-(1→6)]-Gal- |
| b: Different names refer to different isolates of the same structure | | | |
| c, d: Different names refer to different isolates of the same structure | | | |

**TABLE A2 - ADCs which were previously investigated in the human clinical setting, and subsequently retracted from further clinical investigation**

| **Drug Name** | **Indication** | **Target** | **Last Development Stage** | |
|---|---|---|---|---|
| Monoclonal Antibody Conjugate to Target EGFR for Oncology | Oncology | Cells Expressing Epidermal Growth Factor Receptor (Proto Oncogene c ErbB 1 or Receptor Tyrosine Protein Kinase erbB 1 or HER1 or ERBB1 or EGFR or EC 2.7.10.1) | Discovery | |
| Affilutin | Multiple Myeloma (Kahler Disease) | | Discovery | |
| IMGN-779 | Myelodys-plastic Syndrome | Cells Expressing Myeloid Cell Surface Antigen CD33 (Sialic Acid Binding Ig Like Lectin 3 or gp67 or CD33) | IND/CTA Filed | |
| Neuradiab | Non-Hodgkin Lymphoma | Cells Expressing Tenascin (Cytotactin or GMEM or GP 150-225 or Glioma Associated Extracellular Matrix Antigen or Hexabrachion or JI or Myotendinous Antigen or Neuronectin or Tenascin C or TNC) | Phase I | |
| IMGN-779 | Refractory Acute Myeloid Leukemia; Relapsed Acute Myeloid Leukemia | Cells Expressing Myeloid Cell Surface Antigen CD33 (Sialic Acid Binding Ig Like Lectin 3 or gp67 or CD33) | Phase I | |
| AGS-67E | Acute Myelocytic Leukemia (AML, Acute Myeloblas-tic Leukemia) | Cells Expressing Leukocyte Antigen CD37 (Tetraspanin 26 or CD37) | Phase I | |
| AGS-67E | Hairy Cell Leukemia; Non-Hodgkin Lymphoma; Refractory Chronic Lymphocy-tic Leukemia (CLL); Relapsed Chronic Lymphocy-tic Leukemia (CLL); T-Cell Leukemia | Cells Expressing Leukocyte Antigen CD37 (Tetraspanin 26 or CD37) | Phase I | |
| ASG-15ME | Metastatic Transitional (Urothelial) Tract Cancer | Cells Expressing SLIT And NTRK Like Protein 6 (SLITRK6) | Phase I | |
| vandortuzumab vedotin | Metastatic Hormone Refractory (Castration Resistant, Androgen-Indepen-dent) Prostate Cancer | Cells Expressing Metalloreductase STEAP1 (Six Transmembrane Epithelial Antigen Of The Prostate 1 or STEAP1 or EC 1.16.1.) | Phase I | |
| CDX-014 | Ovarian Cancer | Cells Expressing Hepatitis A Virus Cellular Receptor 1 (Kidney Injury Molecule 1 or T Cell Immunoglobulin And Mucin Domain Containing Protein 1 or T-Cell Immunoglobulin Mucin Receptor 1 or T Cell Membrane Protein 1 or CD365 or HAVCR1) | Phase I | |
| AGS-16M18 | Liver Cancer; Renal Cell Carcinoma | | Phase I | |
| vorsetuzumab mafodotin | Non-Hodgkin Lymphoma; Renal Cell Carcinoma | Cells Expressing CD70 Antigen (CD27 Ligand or Tumor Necrosis Factor Ligand Superfamily Member 7 or CD70) | Phase I | |
| denintuzumab mafodotin | Acute Lymphocy-tic Leukemia (ALL, Acute Lympho-blastic Leukemia); B-Cell Non-Hodgkin Lymphoma; Burkitt Lymphoma; Lympho-blastic Lymphoma; Mantle Cell Lymphoma | Cells Expressing B Lymphocyte Antigen CD19 (B Lymphocyte Surface Antigen B4 or Differentiation Antigen CD19 or T Cell Surface Antigen Leu 12 or CD19) | Phase I | |
| SGN-CD70A | Diffuse Large B-Cell Lymphoma; Follicular Lymphoma; Mantle Cell Lymphoma; Metastatic Renal Cell Carcinoma; Non-Hodgkin Lymphoma | Cells Expressing CD70 Antigen (CD27 Ligand or Tumor Necrosis Factor Ligand Superfamily Member 7 or CD70) | Phase I | |
| RG-7636 | Metastatic Melanoma | Endothelin B Receptor (Endothelin Receptor Non Selective Type or EDNRB) | Phase I | |
| SC-006 | Metastatic Colorectal Cancer | | Phase I | |
| MM-310 | Breast Cancer; Endome-trial Cancer; Esophageal Cancer; Gastric Cancer; Gastroeso-phageal (GE) Junction Carcino-mas; Head And Neck Cancer Squamous Cell Carcinoma; Non-Small Cell Lung Cancer; Ovarian Cancer; Pancreatic Ductal Adenocar-cinoma; Prostate Cancer; Small-Cell Lung Cancer; Soft Tissue Sarcoma; Solid Tumor; Transitional Cell Carcinoma (Urothelial Cell Carcinoma) | Ephrin Type A Receptor 2 (Epithelial Cell Kinase or Tyrosine Protein Kinase Receptor ECK or EPHA2 or EC 2.7.10.1) | Phase I | |
| PF-06647263 | Metastatic Breast Cancer; Ovarian Cancer | Cells Expressing Ephrin A4 (EPH Related Receptor Tyrosine Kinase Ligand 4 or EFNA4) | Phase I | |
| PF-06263507 | Solid Tumor | Cells Expressing Trophoblast Glycoprotein (M6P1 or 5T4 Oncofetal Antigen or 5T4 Oncofetal Trophoblast Glycoprotein or Wnt Activated Inhibitory Factor 1 or TPBG) | Phase I | |
| PF-06650808 | Metastatic Breast Cancer; Non-Small Cell Lung Cancer; Ovarian Cancer | Cells Expressing Neurogenic Locus Notch Homolog Protein 3 (NOTCH3) | Phase I | |
| XMT-1522 | Breast Cancer; Gastric Cancer; Non-Small Cell Lung Cancer | Receptor Tyrosine Protein Kinase ERBB 2 (Metastatic Lymph Node Gene 19 Protein or Proto Oncogene Neu or Proto Oncogene C ErbB 2 or Tyrosine Kinase Type Cell Surface Receptor HER2 or p185erbB2 or HER2 or CD340 or ERBB2 or EC 2.7.10.1); Tubulin | Phase I | |
| AMG-595 | Anaplastic Astrocyto-ma; Recurrent Glioblasto-ma Multiforme (GBM) | Cells Expressing Epidermal Growth Factor Receptor (Proto Oncogene c ErbB 1 or Receptor Tyrosine Protein Kinase erbB 1 or HER1 or ERBB1 or EGFR or EC 2.7.10.1) | Phase I | |
| pinatuzumab vedotin | Chronic Lymphocytic Leukemia (CLL) | Cells Expressing B Cell Receptor CD22 (B Lymphocyte Cell Adhesion Molecule or Sialic Acid Binding Ig Like Lectin 2 or T Cell Surface Antigen Leu 14 or CD22) | Phase I | |
| cantuzumab ravtansine | Colorectal Cancer; Non-Small Cell Lung Cancer; Pancreatic Cancer; Solid Tumor | | Phase I | |
| AVE-9633 | Acute Myelocytic Leukemia (AML, Acute Myeloblas-tic Leukemia) | Cells Expressing Myeloid Cell Surface Antigen CD33 (Sialic Acid Binding Ig Like Lectin 3 or gp67 or CD33) | Phase I | |
| **BIWI-1⁽¹⁾** | Breast Cancer; Carcino-mas; Esophageal Cancer; Head And Neck Cancer Squamous Cell Carcinoma | Cells Expressing CD44 Antigen (CDw44 or Epican or Extracellular Matrix Receptor III or GP90 Lymphocyte Homing/Adhesion Receptor or HUTCH I or Heparan Sulfate Proteoglycan or Hermes Antigen or Hyaluronate Receptor or Phagocytic Glycoprotein 1 or CD44) | Phase I | |
| RG-7882 | Epithelial Ovarian Cancer; Fallopian Tube Cancer; Pancreatic Cancer; Peritoneal Cancer | Cells Expressing Mucin 16 (Ovarian Cancer Related Tumor Marker CA125 or Ovarian Carcinoma Antigen CA125 or MUC16) | Phase I | |
| ASG-5ME | Adenocar-cinoma; Hormone Refractory (Castration Resistant, Androgen-Indepen-dent) Prostate Cancer; Metastatic Adenocar-cinoma of The Pancreas | Cells Expressing Choline Transporter Like Protein 4 (Solute Carrier Family 44 Member 4 or SLC44A4) | Phase I | |
| DCDS-0780A | B-Cell Non-Hodgkin Lymphoma | | Phase I | |
| SC-004 | Endome-trial Cancer; Epithelial Ovarian Cancer; Fallopian Tube Cancer; Peritoneal Cancer | | Phase I | |
| RG-7600 | Ovarian Cancer; Pancreatic Ductal Adenocar-cinoma | | Phase I | |
| sofituzumab vedotin | Epithelial Ovarian Cancer; Fallopian Tube Cancer; Ovarian Cancer; Pancreatic Cancer; Peritoneal Cancer | Cells Expressing Mucin 16 (Ovarian Cancer Related Tumor Marker CA125 or Ovarian Carcinoma Antigen CA125 or MUC16) | Phase I | |
| IMGN-289 | Breast Cancer; Esophageal Cancer; Gastric Cancer; Head And Neck Cancer Squamous Cell Carcinoma; Non-Small Cell Lung Cancer; Solid Tumor | Cells Expressing Epidermal Growth Factor Receptor (Proto Oncogene c ErbB 1 or Receptor Tyrosine Protein Kinase erbB 1 or HER1 or ERBB1 or EGFR or EC 2.7.10.1) | Phase I | |
| SAR-428926 | Breast Cancer; Colorectal Cancer; Gastric Cancer; Non-Small Cell Lung Cancer; Ovarian Cancer; Prostate Cancer; Solid Tumor | Cells Expressing Lysosome Associated Membrane Glycoprotein 1 (CD107 Antigen Like Family Member A or CD107a or LAMP1) | Phase I | |
| SGNCD-19B | B-Cell Non-Hodgkin Lymphoma; Diffuse Large B-Cell Lymphoma; Follicular Lymphoma | Cells Expressing B Lymphocyte Antigen CD19 (B Lymphocyte Surface Antigen B4 or Differentiation Antigen CD19 or T Cell Surface Antigen Leu 12 or CD19) | Phase I | |
| SGNCD-123A | Refractory Acute Myeloid Leukemia; Relapsed Acute Myeloid Leukemia | Cells Expressing Interleukin 3 Receptor Subunit Alpha (CD123 or IL3RA) | Phase I | |
| SGNCD-352A | Refractory Multiple Myeloma; Relapsed Multiple Myeloma | Cells Expressing SLAM Family Member 6 (Activating NK Receptor or NK T B Antigen or CD352 or SLAMF6) | Phase I | |
| RG-7841 | Breast Cancer; Non-Small Cell Lung Cancer; Solid Tumor | Cells Expressing Lymphocyte Antigen 6E (Retinoic Acid Induced Gene E Protein or Stem Cell Antigen 2 or Thymic Shared Antigen 1 or LY6E) | Phase I | |
| IMGN-388 | Solid Tumor | Cells Expressing Integrin Alpha V (Vitronectin Receptor Subunit Alpha or CD51 or ITGAV) | Phase I | |
| lorvotuzumab mertansine | Refractory Multiple Myeloma; Relapsed Multiple Myeloma | Cells Expressing Neural Cell Adhesion Molecule 1 (Antigen Recognized By Monoclonal Antibody 5.1H11 or CD56 or NCAM1) | Phase I | |
| lorvotuzumab mertansine | Neuroendo-crine Carcinoma; Neuroendo-crine Tumors; Non-Small Cell Lung Cancer; Ovarian Cancer; Skin Cancer | Cells Expressing Neural Cell Adhesion Molecule 1 (Antigen Recognized By Monoclonal Antibody 5.1H11 or CD56 or NCAM1) | Phase I | |
| BAY-794620 | Lung Cancer; Solid Tumor | Cells Expressing Carbonic Anhydrase 9 (Carbonate Dehydratase IX or pMW1 or Membrane Antigen MN or P54/58N or Renal Cell Carcinoma Associated Antigen G250 or CA9 or EC 4.2.1.1) | Phase I | |
| RG-7598 | Refractory Multiple Myeloma; Relapsed Multiple Myeloma | | Phase I | |
| Oncolysin B | B-Cell Leukemia; Lymphoma | Cells Expressing B Lymphocyte Antigen CD19 (B Lymphocyte Surface Antigen B4 or Differentiation Antigen CD19 or T Cell Surface Antigen Leu 12 or CD19) | Phase I | |
| **ADCT-502⁽¹⁾** | Bladder Cancer; Breast Cancer; Esophageal Cancer; Gastric Cancer; Non-Small Cell Lung Cancer | Cells Expressing Receptor Tyrosine Protein Kinase ERBB 2 (Metastatic Lymph Node Gene 19 Protein or Proto Oncogene Neu or Proto Oncogene C ErbB 2 or Tyrosine Kinase Type Cell Surface Receptor HER2 or p185erbB2 or HER2 or CD340 or ERBB2 or EC 2.7.10.1) | Phase I | |
| AMG-172 | Renal Cell Carcinoma | Cells Expressing CD70 Antigen (CD27 Ligand or Tumor Necrosis Factor Ligand Superfamily Member 7 or CD70) | Phase I | |
| ImmuRAIT-LL2 | B-Cell Non-Hodgkin Lymphoma | Cells Expressing B Cell Receptor CD22 (B Lymphocyte Cell Adhesion Molecule or Sialic Acid Binding Ig Like Lectin 2 or T Cell Surface Antigen Leu 14 or CD22) | Phase I/II | |
| indusatumab vedotin | Adenocar-cinoma Of The Gastroe-sophageal Junction; Gastric Cancer | Cells Expressing Heat Stable Enterotoxin Receptor (Guanylyl Cyclase C or or Intestinal Guanylate Cyclase or GUCY2C or EC 4.6.1.2) | Phase I/II | |
| clivatuzumab tetraxetan | Pancreatic Cancer | Cells Expressing Mucin 1 (Breast Carcinoma Associated Antigen DF3 or Episialin or H23AG or Krebs Von Den Lungen 6 or PEMT or Peanut Reactive Urinary Mucin or Polymorphic Epithelial Mucin or Tumor Associated Epithelial Membrane Antigen or Tumor Associated Mucin or CD227 or MUC1) | Phase I/II | |
| **depatuxizumab mafodotin⁽²⁾** | Recurrent Malignant Glioma | Epidermal Growth Factor Receptor (Proto Oncogene c ErbB 1 or Receptor Tyrosine Protein Kinase erbB 1 or HER1 or ERBB1 or EGFR or EC 2.7.10.1) | Phase I/II | |
| CDX-014 | Metastatic Renal Cell Carcinoma; Papillary Renal Cell Carcinoma | Cells Expressing Hepatitis A Virus Cellular Receptor 1 (Kidney Injury Molecule 1 or T Cell Immunoglobulin And Mucin Domain Containing Protein 1 or T-Cell Immunoglobulin Mucin Receptor 1 orT Cell Membrane Protein 1 or CD365 or HAVCR1) | Phase I/II | |
| **vadastuximab talirine⁽¹⁾** | Refractory Acute Myeloid Leukemia; Relapsed Acute Myeloid Leukemia | Cells Expressing Myeloid Cell Surface Antigen CD33 (Sialic Acid Binding Ig Like Lectin 3 or gp67 or CD33) | Phase I/II | |
| vadastuximab talirine | Myelodys-plastic Syndrome | Cells Expressing Myeloid Cell Surface Antigen CD33 (Sialic Acid Binding Ig Like Lectin 3 or gp67 or CD33) | Phase I/II | |
| MLN-2704 | Metastatic Hormone Refractory (Castration Resistant, Androgen-Indepen-dent) Prostate Cancer | Cells Expressing Glutamate Carboxypeptidase 2 (Folate Hydrolase 1 or Prostate Specific Membrane Antigen or PSMA or Pteroylpoly Gamma Glutamate Carboxypeptidase or Cell Growth Inhibiting Gene 27 Protein or FOLH1 or EC 3.4.17.21) | Phase I/II | |
| Oncolysin B | AIDS - Related Lymphoma | Cells Expressing B Lymphocyte Antigen CD19 (B Lymphocyte Surface Antigen B4 or Differentiation Antigen CD19 or T Cell Surface Antigen Leu 12 or CD19) | Phase I/II | |
| coltuximab ravtansine | Diffuse Large B-Cell Lymphoma | Cells Expressing B Lymphocyte Antigen CD19 (B Lymphocyte Surface Antigen B4 or Differentiation Antigen CD19 or T Cell Surface Antigen Leu 12 or CD19) | Phase II | |
| coltuximab ravtansine | Acute Lymphocy-tic Leukemia (ALL, Acute Lympho-blastic Leukemia) | Cells Expressing B Lymphocyte Antigen CD19 (B Lymphocyte Surface Antigen B4 or Differentiation Antigen CD19 or T Cell Surface Antigen Leu 12 or CD19) | Phase II | |
| coltuximab ravtansine | Diffuse Large B-Cell Lymphoma | Cells Expressing B Lymphocyte Antigen CD19 (B Lymphocyte Surface Antigen B4 or Differentiation Antigen CD19 or T Cell Surface Antigen Leu 12 or CD19) | Phase II | |
| **indusatumab vedotin⁽²⁾** | Adenocar-cinoma Of The Gastroe-sophageal Junction; Gastric Cancer; Metastatic Adenocar-cinoma of The Pancreas | Cells Expressing Heat Stable Enterotoxin Receptor (Guanylyl Cyclase C or or Intestinal Guanylate Cyclase or GUCY2C or EC 4.6.1.2) | Phase II | |
| depatuxizumab mafodotin | Squamous Non-Small Cell Lung Cancer | Epidermal Growth Factor Receptor (Proto Oncogene c ErbB 1 or Receptor Tyrosine Protein Kinase erbB 1 or HER1 or ERBB1 or EGFR or EC 2.7.10.1) | Phase II | |
| **depatuxizumab mafodotin⁽²⁾** | Anaplastic Astrocyto-ma; Anaplastic Oligoastro-cytoma; Gliosar-coma; High-Grade Glioma; Oligoden-droglioma; Pediatric Diffuse Intrinsic Pontine Glioma; Recurrent Glioblasto-ma Multiforme (GBM) | Epidermal Growth Factor Receptor (Proto Oncogene c ErbB 1 or Receptor Tyrosine Protein Kinase erbB 1 or HER1 or ERBB1 or EGFR or EC 2.7.10.1) | Phase II | |
| lifastuzumab vedotin | Non-Small Cell Lung Cancer | Sodium Dependent Phosphate Transport Protein 2B (Sodium Phosphate Transport Protein 2B or NaPi3b or Sodium/Phosphate Cotransporter 2B or NaPi 2b or Solute Carrier Family 34 Member 2 or SLC34A2) | Phase II | |
| lifastuzumab vedotin | Ovarian Cancer | Sodium Dependent Phosphate Transport Protein 2B (Sodium Phosphate Transport Protein 2B or NaPi3b or Sodium/Phosphate Cotransporter 2B or NaPi 2b or Solute Carrier Family 34 Member 2 or SLC34A2) | Phase II | |
| Bismab-A | Acute Myelocytic Leukemia (AML, Acute Myeloblas-tic Leukemia) | Cells Expressing Myeloid Cell Surface Antigen CD33 (Sialic Acid Binding Ig Like Lectin 3 or gp67 or CD33) | Phase II | |
| denintuzumab mafodotin | Diffuse Large B-Cell Lymphoma; Follicular Lymphoma | Cells Expressing B Lymphocyte Antigen CD19 (B Lymphocyte Surface Antigen B4 or Differentiation Antigen CD19 or T Cell Surface Antigen Leu 12 or CD19) | Phase II | |
| **Avicidin⁽¹⁾** | Colorectal Cancer; Prostate Cancer | Cells Expressing Epithelial Cell Adhesion Molecule (Adenocarcinoma Associated Antigen or Cell Surface Glycoprotein Trop 1 or Epithelial Cell Surface Antigen or Epithelial Glycoprotein 314 or KS 1/4 Antigen or KSA or Tumor Associated Calcium Signal Transducer 1 or CD326 or EPCAM) | Phase II | |
| pinatuzumab vedotin | Diffuse Large B-Cell Lymphoma; Follicular Lymphoma | Cells Expressing B Cell Receptor CD22 (B Lymphocyte Cell Adhesion Molecule or Sialic Acid Binding Ig Like Lectin 2 or T Cell Surface Antigen Leu 14 or CD22) | Phase II | |
| SGN-15 | Metastatic Breast Cancer; Non-Small Cell Lung Cancer; Ovarian Cancer; Prostate Cancer | Cells Expressing Lewis Y Antigen (CD174) | Phase II | |
| cantuzumab ravtansine | Gastric Cancer; Gastroe-sophageal (GE) Junction Carcino-mas | | Phase II | |
| ASP-6183 | Ovarian Cancer | | Phase II | |
| SAR-566658 | Metastatic Breast Cancer | Cells Expressing Sialoglycotope CA6 Antigen | Phase II | |
| Oncolysin S | Small-Cell Lung Cancer | Cells Expressing Neural Cell Adhesion Molecule 1 (Antigen Recognized By Monoclonal Antibody 5.1H11 or CD56 or NCAM1) | Phase II | |
| lorvotuzumab mertansine | Small-Cell Lung Cancer | Cells Expressing Neural Cell Adhesion Molecule 1 (Antigen Recognized By Monoclonal Antibody 5.1H11 or CD56 or NCAM1) | Phase II | |
| glembatumumab vedotin | Metastatic Melanoma; Metastatic Uveal Melanoma; Osteosar-coma; Squamous Non-Small Cell Lung Cancer | Cells Expressing Transmembrane Glycoprotein NMB (Transmembrane Glycoprotein HGFIN or GPNMB) | Phase II | |
| MM-302 | Metastatic Breast Cancer | Cells Expressing Receptor Tyrosine Protein Kinase ERBB 2 (Metastatic Lymph Node Gene 19 Protein or Proto Oncogene Neu or Proto Oncogene C ErbB 2 or Tyrosine Kinase Type Cell Surface Receptor HER2 or p185erbB2 or HER2 or CD340 or ERBB2 or EC 2.7.10.1) | Phase II/III | |
| Neuradiab | Brain Cancer; Glioblasto-ma Multiforme (GBM) | Cells Expressing Tenascin (Cytotactin or GMEM or GP 150-225 or Glioma Associated Extracellular Matrix Antigen or Hexabrachion or JI or Myotendinous Antigen or Neuronectin or Tenascin C or TNC) | Phase III | |
| clivatuzumab tetraxetan | Metastatic Adenocar-cinoma of The Pancreas | Cells Expressing Mucin 1 (Breast Carcinoma Associated Antigen DF3 or Episialin or H23AG or Krebs Von Den Lungen 6 or PEMT or Peanut Reactive Urinary Mucin or Polymorphic Epithelial Mucin or Tumor Associated Epithelial Membrane Antigen or Tumor Associated Mucin or CD227 or MUC1) | Phase III | |
| **depatuxizumab mafodotin⁽²⁾** | Glioblasto-ma Multiforme (GBM) | Epidermal Growth Factor Receptor (Proto Oncogene c ErbB 1 or Receptor Tyrosine Protein Kinase erbB 1 or HER1 or ERBB1 or EGFR or EC 2.7.10.1) | Phase III | |
| **vadastuximab talirine⁽¹⁾** | Acute Myelocytic Leukemia (AML, Acute Myeloblas-tic Leukemia) | Cells Expressing Myeloid Cell Surface Antigen CD33 (Sialic Acid Binding Ig Like Lectin 3 or gp67 or CD33) | Phase III | |
| **glembatumumab vedotin⁽²⁾** | Metastatic Breast Cancer | Cells Expressing Transmembrane Glycoprotein NMB (Transmembrane Glycoprotein HGFIN or GPNMB) | Phase III | |
| Oncolysin B | B-Cell Leukemia; Lymphoma | Cells Expressing B Lymphocyte Antigen CD19 (B Lymphocyte Surface Antigen B4 or Differentiation Antigen CD19 or T Cell Surface Antigen Leu 12 or CD19) | Phase III | |
| ImmuRAIT-LL2 | B-Cell Leukemia | Cells Expressing B Cell Receptor CD22 (B Lymphocyte Cell Adhesion Molecule or Sialic Acid Binding Ig Like Lectin 2 or T Cell Surface Antigen Leu 14 or CD22) | Preclinical | |
| indusatumab vedotin | Metastatic Colorectal Cancer | Cells Expressing Heat Stable Enterotoxin Receptor (Guanylyl Cyclase C or or Intestinal Guanylate Cyclase or GUCY2C or EC 4.6.1.2) | Preclinical | |
| ASG-15ME | Lung Cancer | Cells Expressing SLIT And NTRK Like Protein 6 (SLITRK6) | Preclinical | |
| HTI-1511 | Bile Duct Cancer (Cholangiocarcinoma) ; Breast Cancer; Colorectal Cancer; Non-Small Cell Lung Cancer | Cells Expressing Epidermal Growth Factor Receptor (Proto Oncogene c ErbB 1 or Receptor Tyrosine Protein Kinase erbB 1 or HER1 or ERBB1 or EGFR or EC 2.7.10.1) | Preclinical | |
| ZW-33 | Gastric Cancer; Metastatic Breast Cancer | Cells Expressing Receptor Tyrosine Protein Kinase ERBB 2 (Metastatic Lymph Node Gene 19 Protein or Proto Oncogene Neu or Proto Oncogene C ErbB 2 or Tyrosine Kinase Type Cell Surface Receptor HER2 or p185erbB2 or HER2 or CD340 or ERBB2 or EC 2.7.10.1) | Preclinical | |
| ZW-33 | Ovarian Cancer | Cells Expressing Receptor Tyrosine Protein Kinase ERBB 2 (Metastatic Lymph Node Gene 19 Protein or Proto Oncogene Neu or Proto Oncogene C ErbB 2 or Tyrosine Kinase Type Cell Surface Receptor HER2 or p185erbB2 or HER2 or CD340 or ERBB2 or EC 2.7.10.1) | Preclinical | |
| SGNCD-352A | Non-Hodgkin Lymphoma | Cells Expressing SLAM Family Member 6 (Activating NK Receptor or NK T B Antigen or CD352 or SLAMF6) | Preclinical | |
| HuMax-CD74-ADC | Oncology | Cells Expressing HLA Class II Histocompatibility Antigen Gamma Chain (HLA DR Antigens Associated Invariant Chain or la Antigen Associated Invariant Chain or p33 or CD74) | Preclinical | |
| sacituzumab govitecan | Pancreatic Ductal Adenocar-cinoma | Cells Expressing Tumor Associated Calcium Signal Transducer 2 (Cell Surface Glycoprotein Trop 2 or Membrane Component Chromosome 1 Surface Marker 1 or Pancreatic Carcinoma Marker Protein GA733-1 or TACSTD2) | | |
| sacituzumab govitecan | Adenocar-cinoma; Cervical Cancer; Colorectal Cancer; Endome-trial Cancer; Epithelial Ovarian Cancer; Esophageal Cancer; Follicular Thyroid Cancer; Gastric Cancer; Glioblasto-ma Multiforme (GBM); Head And Neck Cancer Squamous Cell Carcinoma; Hepato-cellular Carcinoma; Kidney Cancer (Renal Cell Cancer); Metastatic Hormone Refractory (Castration Resistant, Androgen-Indepen-dent) Prostate Cancer; Metastatic Transitional (Urothelial) Tract Cancer; Transitional Cell Cancer (Urothelial Cell Cancer) | Cells Expressing Tumor Associated Calcium Signal Transducer 2 (Cell Surface Glycoprotein Trop 2 or Membrane Component Chromosome 1 Surface Marker 1 or Pancreatic Carcinoma Marker Protein GA733-1 or TACSTD2) | | |
| sacituzumab govitecan | Hepato-cellular Carcinoma | Cells Expressing Tumor Associated Calcium Signal Transducer 2 (Cell Surface Glycoprotein Trop 2 or Membrane Component Chromosome 1 Surface Marker 1 or Pancreatic Carcinoma Marker Protein GA733-1 or TACSTD2) | | |
| sacituzumab govitecan | Metastatic Breast Cancer; Transitional Cell Cancer (Urothelial Cell Cancer) | Cells Expressing Tumor Associated Calcium Signal Transducer 2 (Cell Surface Glycoprotein Trop 2 or Membrane Component Chromosome 1 Surface Marker 1 or Pancreatic Carcinoma Marker Protein GA733-1 or TACSTD2) | | |
| sacituzumab govitecan | Non-Small Cell Lung Cancer; Small-Cell Lung Cancer | Cells Expressing Tumor Associated Calcium Signal Transducer 2 (Cell Surface Glycoprotein Trop 2 or Membrane Component Chromosome 1 Surface Marker 1 or Pancreatic Carcinoma Marker Protein GA733-1 or TACSTD2) | | |

| **Drug Name** | | **Indication** | **Target** | **Last Development Stage** |
|---|---|---|---|---|
| sacituzumab govitecan | | Metastatic Breast Cancer | Cells Expressing Tumor Associated Calcium Signal Transducer 2 (Cell Surface Glycoprotein Trop 2 or Membrane Component Chromosome 1 Surface Marker 1 or Pancreatic Carcinoma Marker Protein GA733-1 or TACSTD2) | |
| | (1) Discontinued due to adverse events | | | |
| | (2) Discontinued due to lack of efficacy | | | |

**TABLE A3 - ADCs that reached phase III clinical development**

| **Drug Name** | **Indication** | **Development Stage** | **Last Development Stage** | **Reason for Discontinuation** |
|---|---|---|---|---|
| trastuzumab emtansine | Gastric Cancer | Marketed | Phase II/III | Unspecified |
| MM-302 | Metastatic Breast Cancer | Discontinued | Phase II/III | Business/Strategic Decision |
| trastuzumab emtansine | Metastatic Breast Cancer | Marketed | Phase III | Unspecified |
| trastuzumab emtansine | Gastric Cancer | Marketed | Phase III | Unspecified |
| ibritumomab tiuxetan | Diffuse Large B-Cell Lymphoma | Marketed | Phase III | |
| inotuzumab ozogamicin | Follicular Lymphoma | Marketed | Phase III | |
| inotuzumab ozogamicin | Diffuse Large B-Cell Lymphoma; Non-Hodgkin Lymphoma | Marketed | Phase III | Lack of Efficacy |
| rovalpituzumab tesirine | Small-Cell Lung Cancer | Phase III | Phase III | |
| rovalpituzumab tesirine | Small-Cell Lung Cancer | Phase III | Phase III | |
| Neuradiab | Brain Cancer; Glioblastoma Multiforme (GBM) | Inactive | Phase III | Unspecified |
| clivatuzumab tetraxetan | Metastatic Adenocarcinoma of The Pancreas | Inactive | Phase III | Unspecified |
| depatuxizumab mafodotin | Glioblastoma Multiforme (GBM) | Inactive | Phase III | Lack of Efficacy |
| vadastuximab talirine | Acute Myelocytic Leukemia (AML, Acute Myeloblastic Leukemia) | Discontinued | Phase III | Adverse Events |
| glembatumumab vedotin | Metastatic Breast Cancer | Discontinued | Phase III | Lack of Efficacy |
| Oncolysin B | B-Cell Leukemia; Lymphoma | Discontinued | Phase III | Business/Strategic Decision |

| **TABLE A4.** Tumor-specific cell-surface receptor targets which can be targeted by antibodies that can be used for the AOC-saponin conjugates comprising the conjugate of a payload and a saponin, i.e. the effecor moiety of the invention, ADC-saponin conjugates comprising the effector moiety of the invention comprising the conjugate of a payload and a saponin of the invention (not presented as a limitation; further immunoglobulins are equally suitable for the invention) | |
|---|---|
| **Target cell-surface receptor** | **Example monoclonal antibodies** |
| HER2 | anti-HER2 monoclonal antibody such as trastuzumab and pertuzumab |
| CD20 | anti-CD20 monoclonal antibody such as rituximab, ofatumumab, tositumomab and ibritumomab |
| CA125 | anti-CA125 monoclonal antibody such as oregovomab |
| EpCAM (17-1A) | anti-EpCAM (17-1A) monoclonal antibody such as edrecolomab |
| EGFR | anti-EGFR monoclonal antibody such as cetuximab, panitumumab and nimotuzumab |
| CD30 | anti-CD30 monoclonal antibody such brentuximab |
| CD33 | anti-CD33 monoclonal antibody such as gemtuzumab and huMy9-6 |
| vascular integrin alpha-v beta-3 | anti-vascular integrin alpha-v beta-3 monoclonal antibody such as etaracizumab |
| CD52 | anti-CD52 monoclonal antibody such as alemtuzumab |
| CD22 | anti-CD22 monoclonal antibody such as epratuzumab |
| CEA | anti-CEA monoclonal antibody such as labetuzumab |
| CD44v6 | anti-CD44v6 monoclonal antibody such as bivatuzumab |
| FAP | anti- FAP monoclonal antibody such as sibrotuzumab |
| CD19 | anti-CD19 monoclonal antibody such as huB4 |
| CanAg | anti-CanAg monoclonal antibody such as huC242 |
| CD56 | anti-CD56 monoclonal antibody such huN901 |
| CD38 | anti-CD38 monoclonal antibody such as daratumumab |
| CA6 | anti-CA6 monoclonal antibody such as DS6 |
| IGF-IR | anti-IGF-IR monoclonal antibody such as cixutumumab and 3B7 |
| integrin | anti-integrin monoclonal antibody such as CNTO 95 |
| syndecan-1 | anti-syndecan-1 monoclonal antibody such as B-B4 |

**Table A5: RIPs from plants"**

| **Plant Family** | **Plant Species** | **Proteins** | **Classification** |
|---|---|---|---|
| Adoxaceae | Sambucus ebulus L. | Ebulitin α, Ebulitin β, Ebulitin y | RIP 1 |
| | | Ebulin f, Ebulin I, Ebulin r1, Ebulin r2, SEA | RIP 2 |
| | | SEAII, SELfd, SELId, SELIm | lectin |
| | Sambucus nigra L. | α-Nigritin, β-Nigritin, γ-Nigritin, Nigritin f1, Nigritin f2 | RIP 1 |
| | | basic Nigrin b, Nigrin b = SNA-V, Nigrin f = SNA-Vf, Nigrin I1, Nigrin I2, Nigrin s, SNA-I, SNA-I', SNA-If, SNAflu-I, SNLRP1, SNLRP2 | RIP 2 |
| | | SNA-Id, SNA-Im, SNA-II, SNA-III, SNA-IV = SNA-IVf, SNA-IVI, SNApoI-I, SNApoI-II, TrSNA-I, TrSNA-If | lectin |
| | Sambucus racemosa L. | basic racemosin b, SRA | RIP 2 |
| | | SRLbm = SRAbm | lectin |
| | Sambucus sieboldiana (Miq.) Blume ex Graebn. | SSA = SSA-b-1, Sieboldin-b = SSA-b-2 | RIP 2 |
| | | SSA-b-3, SSA-b-4 | lectin |
| Aizoaceae | Mesembryanthe-mum crystallinum L. | RIP1 | RIP 1 |
| Amaranthaceae | Amaranthus caudatus L. | Amaranthin = ACA | lectin |
| | Amaranthus cruentus L. | ACL | lectin |
| | Amaranthus hypochondriacus L. [Syn.: Amaranthus leucocarpus S. Watson] | A. leucocarpus lectin | lectin |
| | Amaranthus mangostanus L. | Amaramangin | RIP 1 |
| | Amaranthus tricolor L. | AAP-27 | RIP 1 |
| | Amaranthus viridis L. | Amaranthin | RIP 1 |
| | Beta vulgaris L. | Beetin-27 = BE27, Beetin-29 = BE29, Betavulgin | RIP 1 |
| | Celosia argentea L. [Syn.: Celosia cristata L.] | CCP-25, CCP-27 | RIP 1 |
| | Chenopodium album L. | CAP30 | RIP 1 |
| | Spinacia oleracea L. | SoRIP1 = BP31 | RIP 1 |
| | | SoRIP2 | RIP 1 candidate |
| Araliaceae | Aralia elata (Miq.) Seem. | Aralin | RIP 2 |
| | Panax ginseng C.A.Mey | Panaxagin | peculiar RIP 1 candidate/RNase |
| | Panax quinquefolius L. | Quinqueginsin | peculiar RIP 1 candidate/RNase |
| Asparagaceae | Asparagus officinalis L. | Asparin 1, Asparin 2 | RIP 1 |
| | Drimia maritima (L.) Steam [Syn.: Charybdis maritima (L.) Speta] | Charybdin | RIP 1 |
| | Muscari armeniacum Leichtlin ex Baker | Musarmin 1, Musarmin 2, Musarmin 3, Musarmin 4 | RIP 1 |
| | Polygonatum multiflorum (L.) All. | PMRIPm, PMRIPt | RIP 2 |
| | Yucca gloriosa var. tristis Carrière [Syn.: Yucca recurvifolia Salisb.] | Yucca leaf protein = YLP | RIP 1 |
| Basellaceae | Basella rubra L. | Basella RIP 2a, Basella RIP 2b, Basella RIP 3 | RIP 1 |
| Caryophyllaceae | Agrostemma githago L. | Agrostin 2, Agrostin 5, Agrostin 6, Agrostin | RIP 1 |
| | Dianthus barbatus L. | Dianthin 29 | RIP 1 |
| | Dianthus caryophyllus L. | Dianthin 30, Dianthin 32 | RIP 1 |
| | Dianthus chinensis L. [Syn.: Dianthus sinensis Link] | D. sinensis RIP | RIP 1 |
| | Gypsophila elegans M.Bieb. | Gypsophilin | RIP 1 |
| | Silene chalcedonica (L.) E.H.L.Krause [Syn.: Lychnis chalcedonica L.] | Lychnin | RIP 1 |
| | Silene glaucifolia Lag. [Syn.: Petrocoptis glaucifolia (Lag.) Boiss.] | Petroglaucin 1, Petroglaucin 2 | RIP 1 |
| | Silene laxipruinosa Mayol & Rosselló [Syn.: Petrocoptis grandiflora Rothm.] | Petrograndin | RIP 1 |
| | Saponaria ocymoides L. | Ocymoidin | RIP 1 |
| | Saponaria officinalis L. | Saporin-L1 = SO-L1, Saporin-L2 = SO-L2, Saporin-L3 = SO-L3, Saporin-I = SO-I = SO-4, Saporin-R1 = SO-R1, Saporin-R2 = SO-R2, Saporin-R3 = SO-R3, SO3a, SO3b, Saporin-S5 = Saporin 5 = SO-S5, Saporin-S6 = Saporin 6 = SO-6 = SO-S6, Saporin-S8 = SO-S8, Saporin-S9 = Saporin 9 = SO-S9, SAP-C, SAP-S | RIP 1 |
| | Myosoton aquaticum (L.) Moench [Syn.: Stellaria aquatica (L.) Scop.] | Stellarin | RIP 1 |
| | Stellaria media (L.) Vill. | RIP Q3 | RIP 1 |
| | Vaccaria hispanica (Mill.) Rauschert [Syn.: Vaccaria pyramidata Medik.] | Pyramidatin | RIP 1 |
| Cucurbitaceae | Benincasa hispida (Thunb.) Cogn. | Hispin | RIP 1 |
| | | α-benincasin, β-benincasin | sRIP 1 |
| | Bryonia cretica subsp. dioica (Jacq.) Tutin. [Syn.: Bryonia dioica L.] | Bryodin 1 = BD1, Bryodin 2, Bryodin-L, Bryodin-R | RIP 1 |
| | | BDA | lectin/ RIP 2 like |
| | Citrullus colocynthis (L.) Schrad . | Colocin 1, Colocin 2 | RIP 1 |
| | Cucurbita foetidissima Kunth | Foetidissimin | peculiar RIP 2 |
| | | Foetidissimin II | RIP 2 |
| | Cucumis ficifolius A.Rich. [Syn.: Cucumis figarei Delile ex Naudin] | Cucumis figarei RIP = CF-RIP | RIP 1 candidate |
| | Cucurbita maxima Duchesne | Cucurmoschin | sRIP 1 candidate |
| | Cucurbita moschata Duchesne [Syn.: Cucurbita moschata (Duchesne ex Lam.) Duchesne ex Poir.] | Cucurmosin, Cucurmosin 2, C. moschata RIP, Moschatin, PRIP 1, PRIP 2 | RIP 1 |
| | | α-moschin, β-moschin | sRIP 1 candidate |
| | Cucurbita pepo L. | Pepocin | RIP 1 |
| | Cucurbita pepo var. texana (Scheele) D.S.Decker [Syn.: Cucurbita texana (Scheele) A. Gray] | Texanin | RIP 1 |
| | Gynostemma pentaphyllum (Thunb.) Makino | Gynostemmin | RIP 1 |
| | Lagenaria siceraria (Molina) Standi . | Lagenin | RIP 1 candidate |
| | Luffa acutangula (L.) Roxb. | Luffaculin-1, Luffaculin-2 | RIP 1 |
| | | Luffangulin | sRIP 1 |
| | | Luffa acutangula fruit lectin | lectin |
| | Luffa cylindrica (L.) M.Roem [Syn.: Luffa aegyptiaca Mill.] | Luffin, Luffin-a, Luffin-b, α-luffin, β-luffin, LRIP | RIP 1 |
| | | Luffacylin, Luffin P1 | sRIP 1 |
| | | Luffin-S, LuffinS(1), LuffinS(2) = luffin S2, LuffinS(3) | sRIP 1 candidate |
| | Marah oreganus (Torr. & A. Gray) Howell | MOR-I, MOR-II | RIP 1 |
| | Momordica balsamina L. | Balsamin, MbRIP-1, Momordin II | RIP 1 |
| | Momordica charantia L. | MAP 30, α-momorcharin = α-MC = α-MMC, β-momorcharin = β-MC = β-MMC, δ-momorcharin = δ-MMC, Momordin, Momordin = Momordica charantia inhibitor, Momordin II, Momordin-a, Momordin-b | RIP 1 |
| | | γ-momorcharin = γ-MMC, Charantin | sRIP 1 |
| | | RIP 1 candidate | RIP 1 candidate |
| | | MCL = M. charantia lectin, anti-H Lectin, Momordica agglutinin, Momordin, protein fraction 1, protein fraction 2 | lectin |
| | | MCL = Momordica charantia seed lectin = Momordica charantia lectin, MCL1 | RIP 2 |
| | Momordica cochinchinensis Spreng. | Cochinin B, Momorcochin, Momorcochin-S | RIP 1 |
| | Siraitia grosvenorii (Swingle) C.Jeffrey ex A.M.Lu & Zhi Y.Zhang [Syn.: Momordica grosvenorii Swingle] | Momorgrosvin | RIP 1 |
| | Sechium edule (Jacq.) Sw. | Sechiumin | RIP 1 |
| | | Sechium edule fruit lectin | lectin |
| | Trichosanthes anguina L. | Trichoanguin | RIP 1 |
| | | SGSL | lectin/ RIP 2 like |
| | Trichosanthes cordata Roxb. | TCA-I, TCA-II | lectin |
| | Trichosanthes cucumerina L. | TCSL | lectin/ RIP 2 candidate |
| | Trichosanthes cucumeroides (Ser.) Maxim. | β-trichosanthin = β-TCS | RIP 1 |
| | Trichosanthes kirilowii Maxim. | α-kirilowin, β-kirilowin, TAP 29, TK-35, Trichobitacin, Trichokirin, Trichomislin = TCM, Trichosanthin = Trichosanthes antiviral protein = TAP = TCS = α-trichosanthin = α-TCS = GLQ223, Trichosanthin, β-trichosanthin = β-TCS, γ-trichosanthin = γ-TCS | RIP 1 |
| | | Trichokirin S1, S-Trichokirin, Trichosanthrip | sRIP 1 |
| | | TKL-1 = Trichosanthes kirilowii lectin-1 | lectin/ RIP 2 candidate |
| | | TK-I, TK-II, TK-III, Trichosanthes kirilowii lectin | lectin |
| | Trichosanthes kirilowii Maximovicz var. japonica (Miquel) Kitamura | Karasurin-A, Karasurin-B, Karasurin-C | RIP 1 |
| | Trichosanthes lepiniate | Trichomaglin | RIP 1 |
| | Trichosanthes dioica Roxb. | TDSL | lectin/ RIP 2 candidate |
| | Trichosanthes sp. Bac Kan 8-98 | Trichobakin | RIP 1 |
| Cupressaceae | Thuja occidentalis L. | Arborvitae RIP | RIP candidate |
| Euphorbiaceae | Croton tiglium L. | Crotin I | RIP 1 candidate |
| | | Crotin 2 | RIP 1 |
| | Euphorbia characias L. | E. characias lectin | lectin |
| | Suregada multiflora (A.Juss.) Baill. [Syn.: Gelonium multiflorum A.Juss.] | Gelonin = GAP 31 | RIP 1 |
| | Hura Crepitans L. | Hura crepitans RIP, Hura crepitans RIP-5 | RIP 1 |
| | | Hura crepitans latex lectin | RIP 2 |
| | | Crepitin, Hurin, Hura crepitans seed lectin | lectin |
| | Jatropha curcas L. | Curcin, Curcin 2, Curcin-L, Jc-SCRIP | RIP 1 |
| | Manihot palmata Müll. Arg. | Mapalmin | RIP 1 |
| | Manihot esculenta Crantz. [Syn.: Manihot utilissima Pohl] | Manutin 1, Manutin 2 | RIP 1 |
| | Ricinus communis L. | Ricin = crystalline Ricin = Ricin D, Ricin E, RCA = Ricinus communis agglutinin = RCAI = RCA120 = R. communis hemagglutinin = RCB-PHA I, RCAII = RCA60 = RCB-PHA II | RIP 2 |
| | Ricinus communis, USA | Ricin 1, Ricin 2, Ricin 3 | RIP 2 |
| | Ricinus communis, India | Ricin I, Ricin II, Ricin III | RIP 2 |
| | Ricinus sanguienus, France | Ricin₁₁, Ricin₁₂, Ricin₂ | RIP 2 |
| Fabaceae | Abrus precatorius L. | Abrin, Abrin-a = Abrin C = Abrin-III, Abrin-b, Abrin-c = Abrin A = Abrin-I, Abrin-d, Abrin-II, APA = Abrus precatorius agglutinin = Abrus lectin = AAG, APA-I, APA-II | RIP 2 |
| | Abrus pulchellus Thwaites | Pulchellin, Pulchellin PI, Pulchellin PII, Pulchellin PIII | RIP 2 |
| | Pisum sativum subsp. sativum L. [Syn.: Pisum sativum var. arvense (L.) Poir.] | α-pisavin, β-pisavin | RIP 1 |
| | Pisum sativum var. macrocarpon | Sativin | RIP 1 candidate |
| Iridaceae | Iris hollandica var. Professor Blaauw | IrisRIP = IRIP, IrisRIP.A1, IrisRIP.A2, IrisRIP.A3 | RIP 1 |
| | | IRA, IRAb, IRAr | RIP 2 |
| Lamiaceae | Clerodendrum aculeatum (L.) Schltdl. | CA-SRI | RIP 1 candidate |
| | Clerodendrum inerme (L.) Gaertn . | CIP-29 | RIP 1 |
| | | CIP-34 | RIP 1 candidate |
| | Leonurus japonicus Houtt. | Leonurin | RIP candidate |
| Lauraceae | Cinnamomum bodinieri H. Lév. | Bodinierin | RIP 2 |
| | Cinnamomum camphora (L.) J.Presl | Camphorin | RIP 1 |
| | | Cinnamomin, Cinnamomin 1, Cinnamomin 2, Cinnamomin 3 | RIP 2 |
| | | Cinphorin | sRIP 2 |
| | Cinnamomum parthenoxylon (Jack) Meisn. [Syn.: Cinnamomum porrectum (Roxb.) Kosterm.] | Porrectin | RIP 2 |
| Malvaceae | Abelmoschus esculentus (L.) Moench | Abelesculin | RIP 1 |
| Nyctaginaceae | Boerhaavia diffusa L. | Boerhaavia inhibitor | RIP 1 candidate |
| | Bougainvillea spectabilis Willd. | BAP I, Bouganin = Bougainvillea RIP I | RIP 1 |
| | Bougainvillea × buttiana cv. Enid Lancester | BBP-24, BBP-28 | RIP 1 |
| | Bougainvillea × buttiana cv. Mahara | BBAP1 | RIP 1 |
| | Mirabilis expansa (Ruiz & Pav.) Standi. | ME1, ME2 | RIP 1 |
| | Mirabilis jalapa L. | MAP, MAP-2, MAP-3, MAP-4, MAP-S | RIP 1 |
| Olacaceae | Malania oleifera Chun & S. K. Lee | Malanin | lectin/ RIP 2 candidate |
| | Ximenia americana L. | Riproximin = Rpx, Rpx-I, Rpx-II | RIP 2 |
| Passifloraceae | Adenia digitata (Harv.) Engl. | Modeccin = Modeccin 4B, Modeccin 6B | RIP 2 |
| | Adenia ellenbeckii Harms | A. ellenbeckii lectin | RIP 2 candidate |
| | Adenia fruticosa Burtt Davy | A. fruticosa lectin | lectin |
| | Adenia glauca Schinz | A. glauca lectin | RIP 2 candidate |
| | Adenia goetzei Harms (unresolved name) | A. goetzei lectin | RIP 2 |
| | Adenia keramanthus Harms | A. keramanthus lectin | RIP 2 candidate |
| | Adenia lanceolata Engl. | Lanceolin | RIP 2 |
| | Adenia racemosa W. J. de Wilde | A. racemosa lectin | lectin |
| | Adenia spinosa Burtt Davy | A. spinosa lectin | RIP 2 candidate |
| | Adenia stenodactyla Harms | Stenodactylin | RIP 2 |
| | Adenia venenata Forssk. | A. venenata lectin | RIP 2 candidate |
| | Adenia volkensii Harms | Volkensin | RIP 2 |
| Phytolaccaceae | Phytolacca americana L. | α-PAP, PAP = Phytolacca americana protein = pokeweed antiviral protein, PAP-I, PAP-II, PAP-III, PAP-C, PAP-H, PAP-R, PAP-S, PAP-S1, PAP-S2 | RIP 1 |
| | Phytolacca dioica L. | Diocin 1, Diocin 2, PD-L1, PD-L2, PD-L3, PD-L4, PD-S1, PD-S2, PD-S3 | RIP 1 |
| | Phytolacca dodecandra L'Hér. | Dodecandrin, Dodecandrin C | RIP 1 |
| | Phytolacca heterotepala H. Walter | Heterotepalin 4, Heterotepalin 5b | RIP 1 |
| | Phytolacca insularis Nakai | Insularin = PIP = Phytolacca insularis antiviral protein, PIP2 = P. insularis antiviral protein 2 | RIP 1 |
| Poaceae | Hordeum vulgare L. | Barley toxin = Barley translation inhibitor = Barley Protein Synthesis Inhibitor = BPSI = RIP 30, Barley toxin I = Barley translation inhibitor I, Barley toxin II = Barley translation inhibitor II = Barley Protein Synthesis Inhibitor II = BPSI II, Barley toxin III = Barley translation inhibitor III, JIP60 | RIP 1 |
| | Oryza sativa L. | Oryza sativa RIP | RIP 1 |
| | Secale cereale L. | RPSI | RIP 1 |
| | Triticum aestivum L. | Tritin, Tritin 1, Tritin 2, Tritin 3, Tritin-S, Tritin-L | RIP 1 |
| | Zea mays L. | b-32 = maize RIP = maize proRIP1, Maize proRIP2 | RIP 3/ peculiar RIP 1 |
| Ranunculaceae | Eranthis hyemalis (L.) Salisb . | EHL | RIP 2 |
| Santalaceae | Phoradendron californicum Nutt. | PCL | RIP 2 |
| | Viscum album L. (Himalayan mistletoe) | HmRip, HmRip 1, HmRip 2, HmRip 3, HmRip 4 | RIP 2 |
| | Viscum album L. (European mistletoe) | ML-I = Mistletoe lectin I = Viscumin = Eu-ML = EML-1 = VAA-I, ML-II = Mistletoe lectin II = VAA-II, ML-III = Mistletoe lectin III = VAA-III | RIP 2 |
| | Viscum articulatum Burm. f. | Articulatin-D | RIP 2 |
| | Viscum coloratum (Kom.) Nakai [Syn.: Viscum album subsp. coloratum Kom.] | KML, KML-C, KML-IIL, KML-IIU, VCA | RIP 2 |
| Solanaceae | Nicotiana tabacum L. | CIP31 | RIP-like protein |
| | | TRIP | RIP 1 candidate |
| Thymelaeaceae | Phaleria macrocarpa (Scheff.) Boerl. | P. macrocarpa RIP | RIP candidate |
| * Schrot J, Weng A, Melzig MF, et al. Ribosome-inactivating and related proteins. Toxins (Basel). 2015 May 8;7(5):1556-615. | | | |

As said before, the at least one saponin that is comprised by the effector moiety according to the invention increases the efficacy of at least current and new effector moieties as defined in this invention. Potential side-effects will be decreased due to lowering of dosing of the effector moiety comprised by the conjugate formed with the saponin, without lowering the efficacy. Therefore, the invention provides an effector moiety according to the invention for use in medicine or for use as a medicament. Thus, an aspect of the invention relates to an effector moiety of the invention, for use as a medicament. Also provided is the use of an effector moiety according to the invention for manufacturing a medicament. Especially cancer medicines, and in particular the classical chemotherapy medicaments, are notorious for their side effects. Because of synchronization in time and place of both the pharmaceutically active substance comprised by the effector moiety and the saponin comprised by the effector moiety, since the payload and the saponin are covalently linked, a therapeutic composition according to the invention is especially valuable for use as a medicament, in particular for use in a method of treating cancer. The invention thus provides a therapeutic composition comprising an effector moiety according to the invention for use in a method of treating cancer. The invention also provides a therapeutic composition comprising an effector moiety according to the invention for use in a method of treating acquired or hereditary disorders, in particular monogenic deficiency disorders. The therapeutic composition thus comprises the effector moiety, e.g. a payload such as an antisense oligonucleotide. Thus, an aspect of the invention relates to a therapeutic composition comprising an effector moiety according to the invention, wherein payload comprises a covalently bound saponin, for use in a method for the treatment of a cancer or an auto-immune disease.

The invention is further illustrated by the following examples, which should not be interpreted as limiting the present invention in any way.

### EXAMPLES

### Example 1

### Materials and methods

The following chemicals were used as purchased: methanol (MeOH, LiChrosolv, Merck), N-ε-maleimidocaproic acid hydrazide (EMCH, 95%, TCI Chemicals), trifluoroacetic acid (TFA, 99.8%, Carl Roth), 2-mercaptoethanol (98%, Sigma-Aldrich), poly(amidoamine) (PAMAM dendrimer, ethylenediamine core, generation 5.0 solution, Sigma-Aldrich), cyanine 3 carboxylic acid (Cy3-COOH, 95%, Lumiprobe), 1-[Bis(dimethylamino)methylene]-1H-1,2,3-triazolo[4,5-b]pyridinium 3-oxid hexafluorophosphate, N-[(Dimethylamino)-1H-1,2,3-triazolo-[4,5-b]pyridin-1-ylmethylene]-N-methylmethanaminium hexafluorophosphate N-oxide (HATU, 97%, Sigma-Aldrich), bovine serum albumin fraction V (BSA, Carl Roth), dimethylsulfoxide (DMSO, 99%, Carl Roth), 2-lminothiolane hydrochloride (98%, Sigma-Aldrich), rhodamine b (RhodB, 95%, Merck), Dulbecco's phosphate buffered saline (PBS, Gibco), hydrochloric acid (HCl, 37%, Merck), NHS-PEG₁₃-DBCO (Click Chemistry Tools), Alexa Fluor^{™} 488 5-TFP (Thermo-Fischer), azido-PEG3-SS-NHS (Conju-Probe), sodium cyanoborohydride (NaCNBH₃, 95 %, Sigma-Aldrich), ammonium persulfate (APS, 98%, Sigma-Aldrich), N,N,N',N'-tetramethylethylenediamine (TMEDA, 99 %, Sigma-Aldrich), customized peptide SESDDAMFCDAMDESDSK (95%, PeptideSynthetics), azido-dPEG₁₂-NHS (95%, Quanta Biodesign), PFd-G4-Azide-NH-BOC Dendron (G4-dendron, 95%, Polymer Factory), Cyanin5-DBCO (Cy5-DBCO, 95%, Lumiprobe), Chloroform (CHCl₃, 99.5 %, Sigma), Amicon Ultra 0.5 mL centrifugal filters (3 kDa MWCO, Sigma), mPEG-SCM (mPEG₂ₖ-NHS, 95.6%, Creative PEG Works), Amicon Ultra 15 mL centrifugal filters (10 kDa MWCO, Sigma).

### MALDI-TOF-MS

MALDI-TOF spectra were recorded on a MALDI-Mass Spectrometer (Bruker Ultrafex III). Typically, the sample dissolved in MilliQ water in nanomolar to micromolar range was spotted on the target (MTP 384 target plate polished steel T F, Bruker Daltons) using either super-DHB (99%, Fluka) or sinapinic acid (SA, 99%, Sigma-Aldrich) as the matrix dissolved in acetonitrile (MADLI-TOF-MS tested, Sigma) / 0.1% TFA (7:3 v/v) via the dried-droplet-method. PepMix (Peptide Calibration Standard, Bruker Daltons) or ProteMass (Protein Calibration Standard, Sigma-Aldrich) served as calibration standards. RP mode refers to reflector positive mode. RN mode refers to reflector negative mode. LP mode refers to linear positive mode.

### H-NMR

¹H NMR analysis was performed using a Bruker 400 MHz NMR spectrometer. The sample preparation, in which 2 mg of sample had been dissolved in 0.8 mL of methanol-*D₄* (99%, Deutero), was performed 24 h prior to the measurement.

### Size Exclusion Chromatography

Size exclusion chromatography (SEC) was performed with Sephadex G 25 Superfine from GE Healthcare and on prepacked PD10 columns (GE Healthcare, Sephadex G 25 M). The material was activated by swelling in the respective eluent prior to performing chromatography.

### Dialysis

Regenerated cellulose membranes: MWCO = 1 and 2 kDa (Spectra/Por), and MWCO = 12-14 kDa (Carl Roth) were used to perform dialysis. Typically, dialysis was carried out for 24 h with 1 L of solvent that was exchanged after first 6 h of the process.

### SO1861-EMCH synthesis

SO1861 from Saponaria officinalis L (59 mg, 31.7 µmol) and EMCH (301 mg, 888 µmol) were placed in a round flask with stirrer and dissolved in 13 mL methanol. TFA (400 µL, cat.) was added to the solution and the reaction mixture was stirred for 3 h at 800 rpm and room temperature on a RCT B magnetic stirrer (IKA Labortechnik). After stirring for 3 h, the mix was diluted either with MilliQ water or PBS and dialyzed extensively for 24 h against either with MilliQ water or PBS using regenerated cellulose membrane tubes (Spectra/Por 7) with a MWCO of 1 kDa. After dialysis, the solution was lyophilized to obtain a white powder. Yield 62.4 mg (95 %). Dried aliquots were further used for characterization via ¹H NMR and MALDI-TOF-MS.

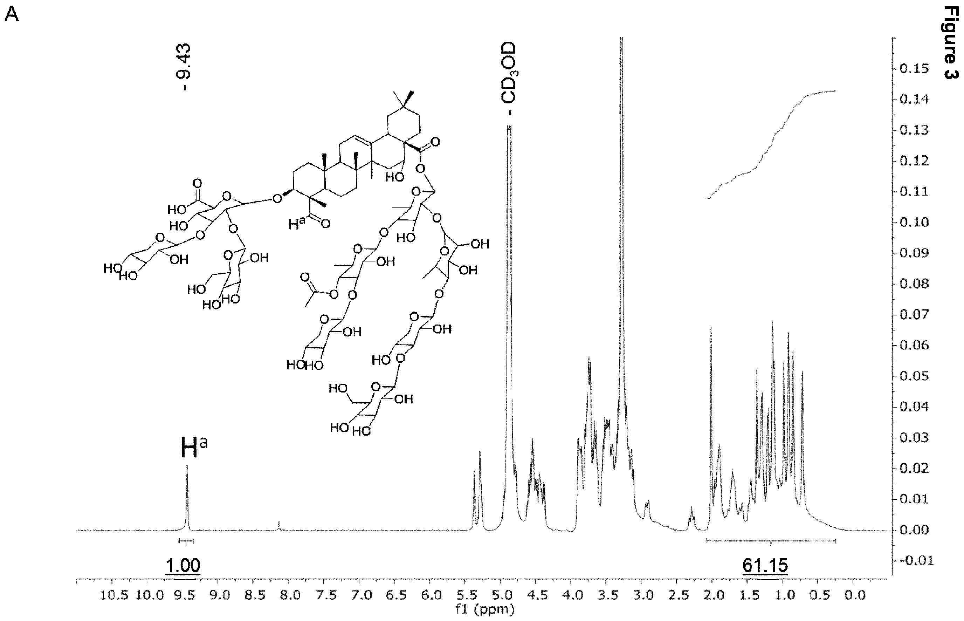

¹H NMR (400 MHz, methanol-*D*₄) (Figure 3 A, SO1861): *δ* = 0.50-5.50 (m, saponin triterpenoid and sugar backbone protons), 9.43 (1H, s, aldehyde proton of saponin, H^{a}).

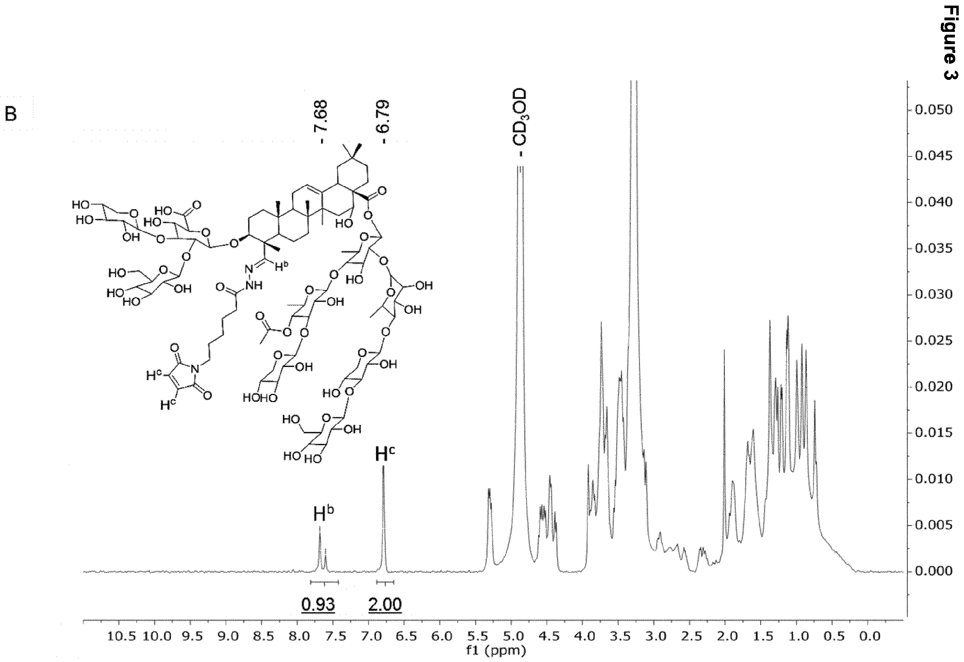

¹H NMR (400 MHz, methanol-*D*₄) (Figure 3 B. SO1861-EMCH, PBS workup): *δ* = 0.50-5.50 (m, saponin triterpenoid and sugar backbone protons), 6.79 (2 H, s, maleimide protons, H^{c}), 7.62-7.68 (1 H, m, hydrazone proton, H^{b}).

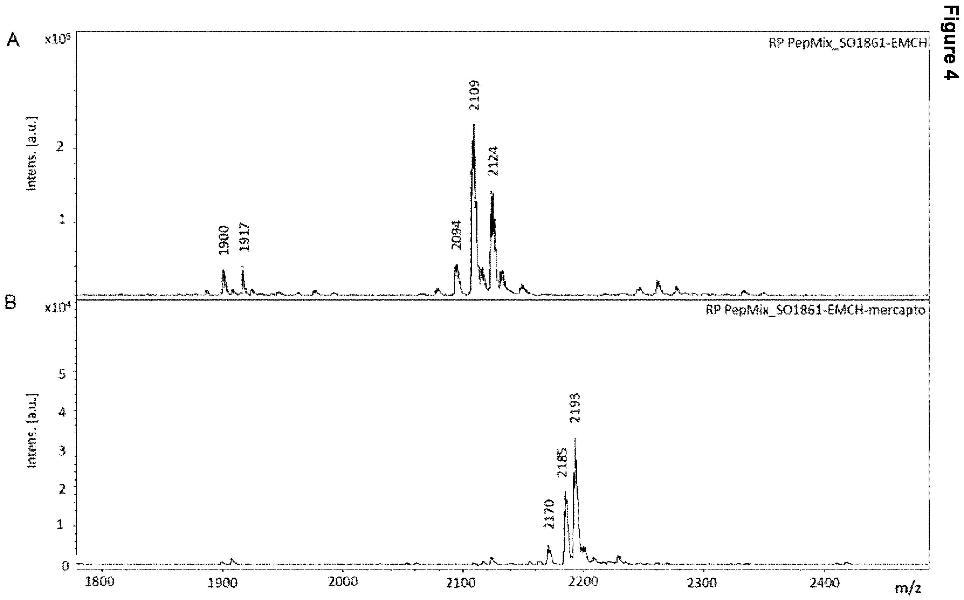

MALDI-TOF-MS (RP mode) (Figure 4 A): *m*/*z* 2124 Da ([M+K]⁺, saponin-EMCH), m/z 2109 Da ([M+K]⁺, SO1861-EMCH), *m*/*z* 2094 Da ([M+Na]⁺, SO1861-EMCH)

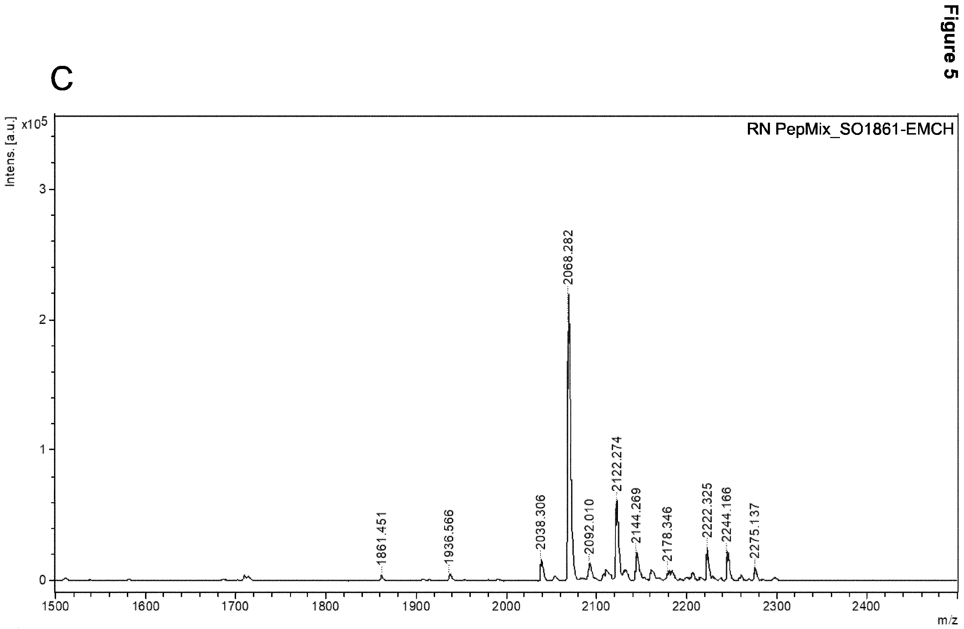

MALDI-TOF-MS (RN mode) (Figure 5 C): *m*/*z* 2275 Da ([M-H]⁻, saponin-EMCH conjugate), 2244 Da ([M-H]⁻, saponin-EMCH conjugate), 2222 Da ([M-H]⁻, saponin-EMCH conjugate), 2178 Da ([M-H]⁻, saponin-EMCH conjugate), 2144 Da ([M-H]⁻, saponin-EMCH conjugate), 2122 Da ([M-H]⁻, saponin-EMCH conjugate), 2092 Da ([M-H]⁻, saponin-EMCH conjugate), 2070 Da ([M-H]⁻, SO1861-EMCH), 2038 Da ([M-H]⁻, SO1832-EMCH), 1936 Da ([M-H]⁻, SO1730-EMCH), 1861 Da ([M-H]⁻, SO1861).

### BSA-SO1861 synthesis

2-iminothiolane (231 µg, 1.1 µmol) dissolved in 47 µL PBS was added to a BSA-RhodB solution (10 mg, 0.15 µmol) in 200 µL PBS and the mix was shaken for 40 min at 800 rpm and room temperature on a ThermoMixer C (Eppendorf). After shaking for 40 min, the reaction mix was immediately run through a Sephadex G25 superfine size exclusion column (16 mL column volume) and SO1861-EMCH (1 mg, 0.5 µmol) dissolved in 100 µL PBS was added to the collected BSA-SH fraction. The reaction mixture was shaken for 12 h at 800 rpm and room temperature on a ThermoMixer C (Eppendorf). After shaking for 12 h the BSA-SO1861 concentrated using centrifugal filtration at 4,000 rpm (15 °C) via Amicon Ultra 15 filters with a MWCO of 3 kDa. The conjugate was stored as solution in the fridge and aliquots were taken for analysis. Yield: not determined.

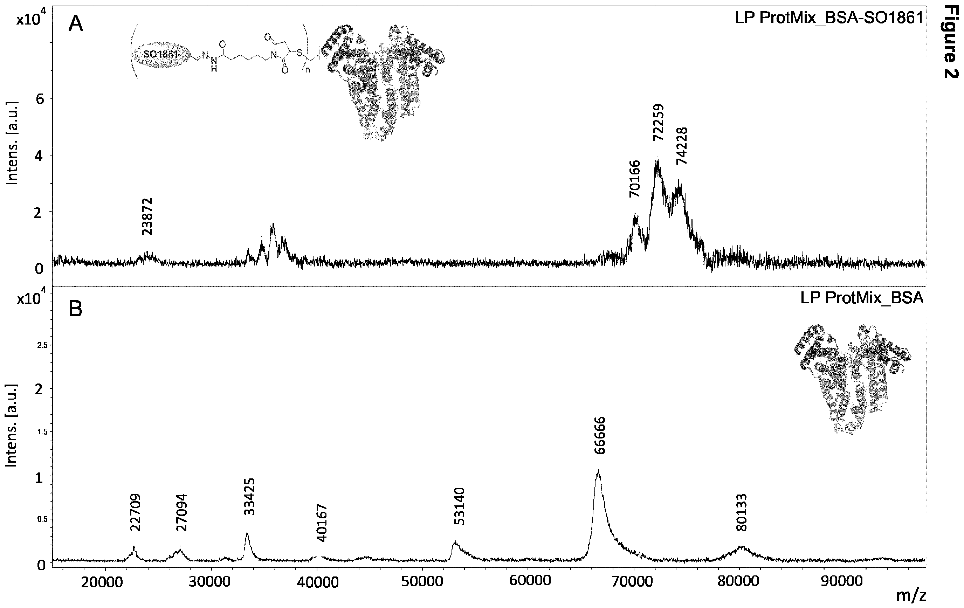

MALDI-TOF-MS (Figure 2 A) (LP mode): *m*/*z* 74.2 kDa ([M+H]⁺, BSA-SO1861 with 4 SO1861 attached), 72.2 kDa ([M+H]⁺, BSA-SO1861 with 3 SO1861 attached), 70.2 kDa ([M+H]⁺, BSA-SO1861 with 2 SO1861 attached), 37.0 kDa ([M+H]²⁺, BSA-SO1861 with 4 SO1861 attached), 35.9 kDa ([M+H]²⁺, BSA-SO1861 with 3 SO1861 attached), 34.7 kDa ([M+H]²⁺, BSA-SO1861 with 2 SO1861 attached).

### Results

First proof of concept for conjugation of SO1861-EMCH to a protein (example of a potential protein effector) was obtained by use of the amine of bovine serum albumin (BSA). After conjugation, mass spectrometry obtained the corresponding peaks of BSA-SO1861 at *m*/*z* ~ 70, ~72, and ~ 74 kDa (Figure 2A). In comparison with the detected mass of BSA with *m*/*z* 66 kDa (Figure 2B), the obtained masses of BSA-SO1861 correspond to a mixture of BSA-SO1861 conjugates consisting of 2, 3, and 4 SO1861 molecules per BSA. This shows that SO1861 molecules can be efficiently conjugated to potential protein effectors.

### Example 2

### Materials and methods

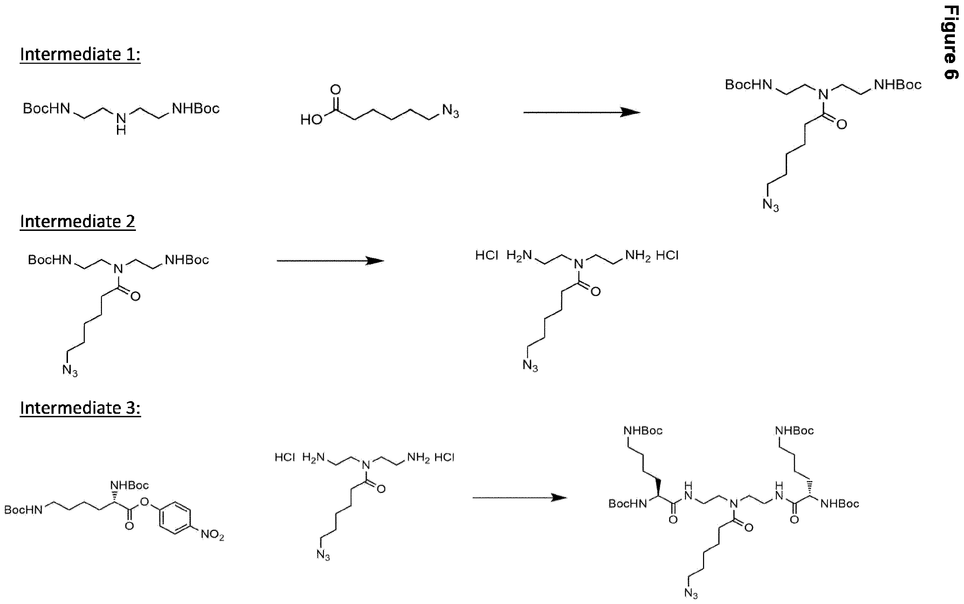

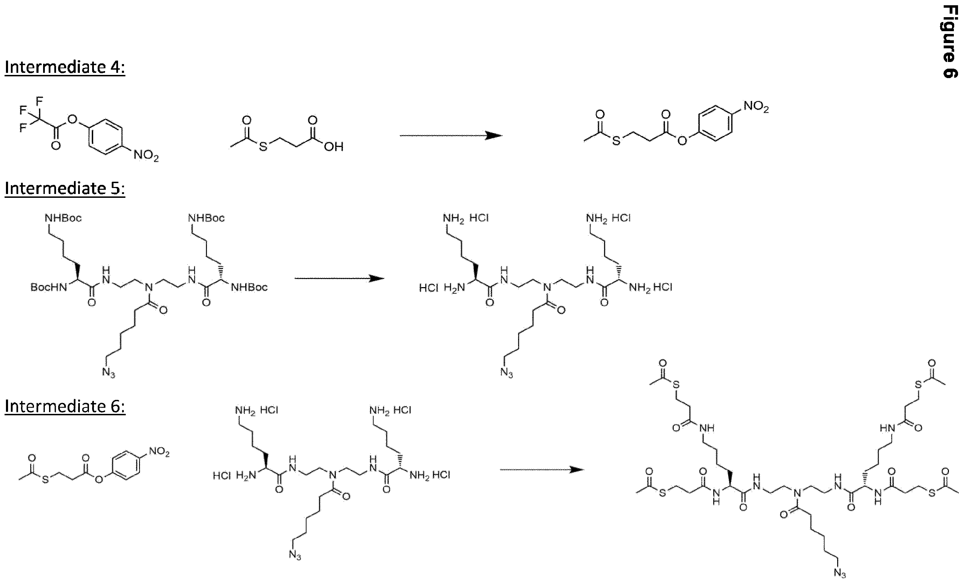

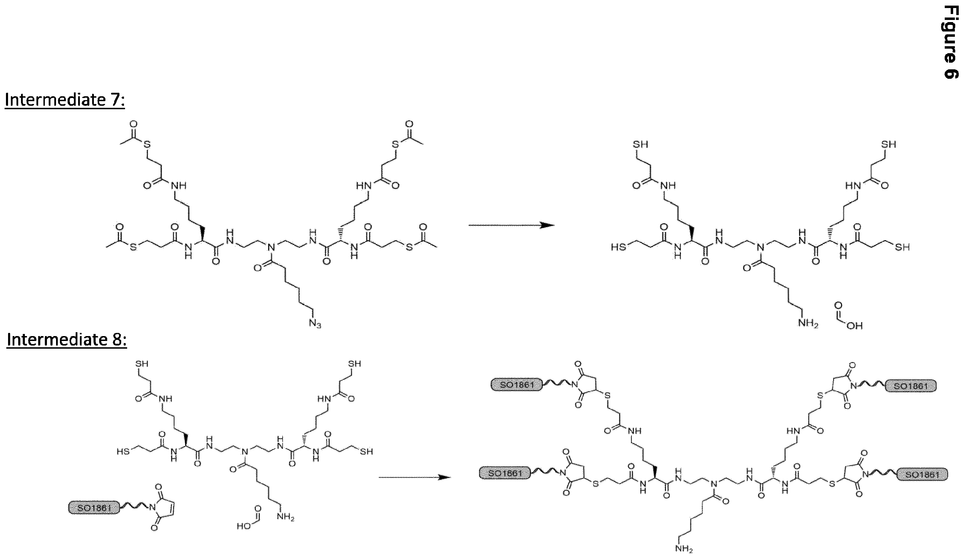

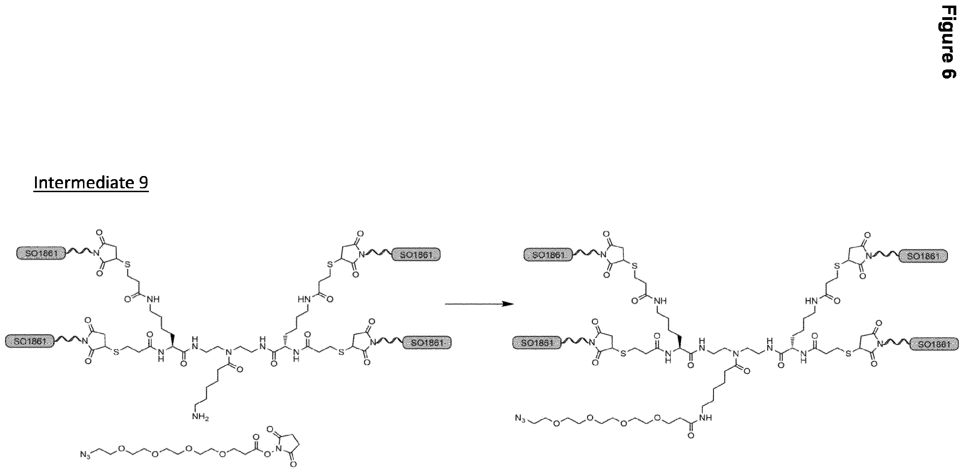

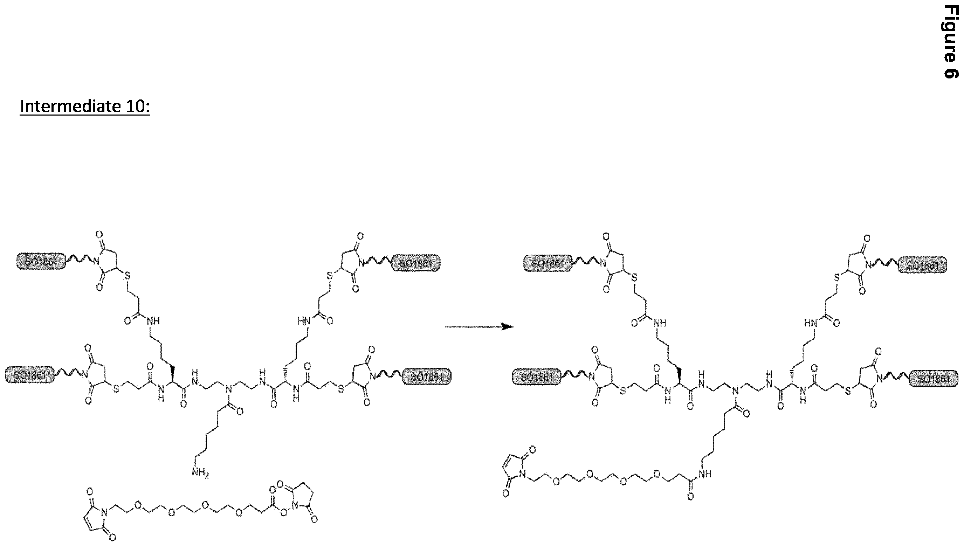

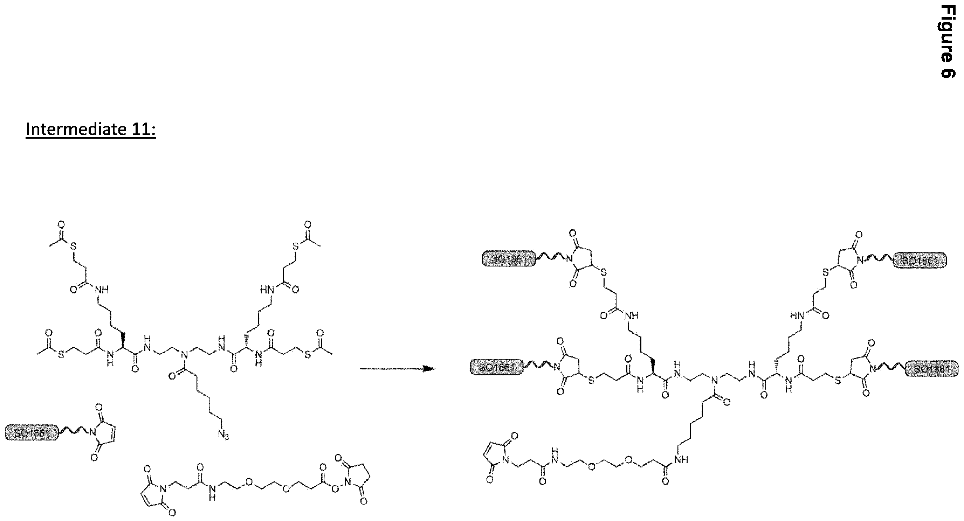

### Dendron(-L-SO1861)⁴synthesis (Figure 6)

### Intermediate 1:

### di-tert-butyl (((6-azidohexanoyl)azanediyl)bis(ethane-2,1-diyl))dicarbamate

6-azidohexanoic acid (0.943 g, 6.00 mmol), EDCl.HCl (1.21 g, 6.30 mmol) and Oxyma Pure (0.938 g, 6.60 mmol) were dissolved in DMF (10.0 mL) and the mixture was stirred for 5 min. Next a solution of di-tert-butyl (azanediylbis(ethane-2,1-diyl))dicarbamate (1.82 g, 6.00 mmol) in DMF (5.00 mL) was added and the reaction mixture was stirred at room temperature. After 5 hours the reaction mixture was evaporated *in vacuo* and the residue was dissolved in ethyl acetate (50 mL). The resulting solution was washed with 1N potassium bisulphate solution (50 mL), saturated sodium bicarbonate solution (2 × 50 mL) and brine (50 mL), dried over Na₂SO₄, filtered and evaporated *in vacuo.* The residue was purified by flash chromatography (ethyl acetate - heptane gradient, 10:90 rising to 100:0) to give the title compound (2.67 g, 100%) as a white solid. Purity based on LC-MS 98%.
LRMS (m/z): 287/343/465 [M-155/M-99/M+23]¹⁺
LC-MS r.t. (min): 2.02^{2A}

### Intermediate 2:

### N,N-bis(2-aminoethyl)-6-azidohexanamide dihydrochloride

To di-tert-butyl (((6-azidohexanoyl)azanediyl)bis(ethane-2,1-diyl))dicarbamate (2.66 g, 6.00 mmol) was added HCl in isopropanol (5-6 N, 20.0 mL, 110 mmol) and the reaction mixture was stirred at room temperature. After 4 hours, the reaction mixture was evaporated *in vacuo* and the resulting crude product was co-evaporated with DCM (3 × 20 mL) to give the crude title product (1.49 g, 79%) as a white solid.
LRMS (m/z): 243 [M+1]¹⁺

### Intermediate 3:

### tetra-tert-butyl ((5S,5'S)-((((6-azidohexanoyl)azanediyl)bis(ethane-2,1-diyl))bis(azanediyl))bis(6-oxohexane-6,1,5-triyl))tetracarbamate

To a solution of N,N-bis(2-aminoethyl)-6-azidohexanamide dihydrochloride (1.19 g, 3.76 mmol) in DMF (30.0 mL) and DIPEA (2.62 mL, 15.1 mmol) was added Boc-Lys(Boc)-ONp (3.69 g, 7.90 mmol) and the mixture was stirred at room temperature overnight. The reaction mixture was evaporated *in vacuo* and the residue was dissolved in ethyl acetate (100 mL). The resulting solution was washed with 1N potassium bisulphate solution (100 mL) and saturated sodium bicarbonate solution (5 × 100 mL), dried over Na₂SO₄, filtered and evaporated *in vacuo.* The residue was purified by flash chromatography (DCM - methanol/DCM (1/9, v/v) gradient 100:0 rising to 0:100) to give the give the title product (3.07 g, 91%) as a slightly yellowish solid. Purity based on LC-MS 94%.
LRMS (m/z): 800/900/922 [M-99/M+1/M+23]¹⁺
LC-MS r.t. (min): 2.17^{2A}

### Intermediate 4:

### 4-nitrophenyl 3-(acetylthio)propanoate

4-Nitrophenyl trifluoroacetate (5.17 g, 22.0 mmol) and 3-(Acetylthio)propionic Acid (2.96 g, 20.0 mmol) were dissolved in DCM (50.0 mL). Next, DIPEA (6.97 mL, 40.0 mmol) was added and the reaction mixture was stirred at room temperature overnight. The reaction mixture was evaporated *in vacuo* and the residue was dissolved in ethyl acetate (50 mL). The resulting solution was washed with 1N potassium bisulphate solution (50 mL), saturated sodium bicarbonate solution (5 × 50 mL) and brine (50 mL), dried over Na₂SO₄, filtered and evaporated *in vacuo.* The residue was purified by flash chromatography (DCM - methanol/DCM (1/9, v/v) gradient 100:0 rising to 0:100) to give the give the title product (4.90 g, 91%) as a slightly yellowish solid. Purity based on LC-MS 99%.
LRMS (m/z): 292 [M+23]¹⁺
LC-MS r.t. (min): 1.94^{2A}

### Intermediate 5:

### (S)-2,6-diamino-N-(2-(6-azido-N-(2-((S)-2,6-diaminohexanamido)ethyl)hexanamido)ethyl)hexanamide tetrahydrochloride

tetra-tert-butyl ((5S,5'S)-((((6-azidohexanoyl)azanediyl)bis(ethane-2,1-diyl))bis(azanediyl))bis(6-oxohexane-6,1,5-triyl))tetracarbamate (1.80 g, 2.00 mmol) was dissolved in HCl in isopropanol (5-6N, 50.0 ml, 275 mmol) and the reaction mixture was stirred at room temperature overnight. The reaction mixture was evaporated *in vacuo* and the resulting crude product was co-evaporated with DCM (3 × 20 mL) to give the crude title product as a white solid.
LRMS (m/z): 250 [M+2]²⁺, 500 [M+1]¹⁺

### Intermediate 6:

### (2S)-2,6-bis[3-(acetylsulfanyl)propanamido]-N-[2-(6-azido-N-{2-[(2S)-2,6-bis[3-(acetylsulfanyl)propanamido]hexanamido]ethyl}hexanamido)ethyl]hexanamide

To a solution of (S)-2,6-diamino-N-(2-(6-azido-N-(2-((S)-2,6- diaminohexanamido)ethyl)hexanamido) ethyl)hexanamide tetrahydrochloride (1.29 g, 2.00 mmol) in DMF (30 mL) and DIPEA (3.48 mL, 20.0 mmol) was added 4-nitrophenyl 3-(acetylthio)propanoate (2.26 g, 8.40 mmol) and the reaction mixture was stirred at room temperature over the weekend. The reaction mixture was evaporated *in vacuo* and the residue was dissolved in DCM/methanol (95:5, v/v, 100 mL). The resulting solution was washed with 1N potassium bisulphate solution (100 mL), 1 N sodium hydroxide solution (3 × 100 mL) and brine (100 mL), dried over Na₂SO₄, filtered and evaporated *in vacuo.* The residue was purified by flash chromatography (DCM - methanol/DCM (1/9, v/v) gradient 100:0 rising to 0:100) to give the title product (1.33 g, 65%) as a white solid. On LC-MS an impurity (15%) was found with m/z values corresponding to the product with one deprotected thioacetate group. The impurity was formed during or after work-up. Purity based on LC-MS 85%.
LRMS (m/z): 510 [M+2]²⁺, 1019/1041 [M+1/M+23]¹⁺
LC-MS r.t. (min): 1.86^{2B}

### Intermediate 7:

### N,N'-((9S,19S)-14-(6-aminohexanoyl)-1 -mercapto-9-(3-mercaptopropanamido)-3,10,18-trioxo-4,11,14,17-tetraazatricosane-19,23-diyl)bis(3-mercaptopropanamide) formate

Scaffold 2 (102 mg, 0.100 mmol) was dissolved in methanol (1.00 ml). Next, a freshly prepared 1 N Sodium hydroxide solution (0.440 ml, 0.440 mmol) was added and the reaction mixture was stirred at room temperature. After 30 min a 1.0 M trimethylphosphine solution in THF (0.500 ml, 0.500 mmol) was added and the resulting mixture was stirred at room temperature. After 30 min the reaction mixture was evaporated *in vacuo* and co-evaporated with methanol (2 × 10 mL). The residue was dissolved in a mixture of methanol/water (9:1, v/v, 1.00 mL) and the resulting solution was subjected to preparative MP-LC.² Fractions corresponding to the product were immediately pooled together, frozen and lyophilized overnight to give the title compound (75.6 mg, 87%) as a colorless sticky oil. Purity based on LC-MS 96%.
LRMS (m/z): 513 [M+2]²⁺, 825 [M+1]¹⁺
LC-MS r.t. (min): 1.42^{2A}

### Intermediate 8:

### dendron(-L-SO1861)⁴-amine

N, N'-((9S,19S)-14-(6-aminohexan oyl)-1-mercapto-9-(3-mercaptopro panamido)-3,10,18-trioxo-4,11,14,17-tetraazatricosane-19,23-diyl)bis(3-mercaptopropanamide) formate (2.73 mg, 3.13 µmol) was dissolved in a mixture of 20 mM NH₄HCO₃ with 0.5 mM TCEP/acetonitrile (3:1, v/v, 3.00 mL). Next, SO1861-EMCH (29.2 mg, 0.014 mmol) was added and the reaction mixture was stirred at room temperature. After 1.5 hours the reaction mixture was subjected to preparative LC-MS.^{3B} Fractions corresponding to the product were immediately pooled together, frozen and lyophilized overnight to give the title compound (12.3 mg, 43%) as a white fluffy solid. Purity based on LC-MS 97%.
LRMS (m/z): 1517 [M-6]⁶⁻, 1821 [M-5]⁵⁻, 2276 [M-4]⁴⁻
LC-MS r.t. (min): 4.39^{5A}

### Intermediate 9:

### dendron(-L-SO1861)⁴-azide

Dendron(SO1861)₄-amine (6.81 mg, 0.748 µmol) and 2,5-dioxopyrrolidin-1-yl 1-azido-3,6,9,12-tetraoxapentadecan-15-oate (2.90 mg, 7.48 µmol) were dissolved in DMF(1.00 mL). Next, DIPEA (1.302 µL, 7.48 µmol) was added and the mixture was shaken for 1 min and left standing at room temperature. After 2 hours the reaction mixture was subjected to preparative LC-MS.^{3C} Fractions corresponding to the product were immediately pooled together, frozen and lyophilized overnight to give the title compound (5.86 mg, 84%) as a white fluffy solid. Purity based on LC-MS 90%.
LRMS (m/z): 2344 [M-4]⁴⁻
LC-MS r.t. (min): 4.78^{5B}

### Intermediate 10:

### dendron(-L-SO1861)⁴-maleimide1

Dendron(SO1861)₄-amine (8.12 mg, 0.891 µmol) and 2,5-dioxopyrrolidin-1-yl 1-(2,5-dioxo-2,5-dihydro-1H-pyrrol-1-yl)-3,6,9,12-tetraoxapentadecan-15-oate (3.94 mg, 8.91 µmol) were dissolved in DMF(1.00 mL). Next, DIPEA (1.55 µL, 8.91 µmol) was added and the mixture was shaken for 1 min and left standing at room temperature. After 3 hours the reaction mixture was subjected to preparative LC-MS.^{3C} Fractions corresponding to the product were immediately pooled together, frozen and lyophilized overnight to give the title compound (6.76 mg, 80%) as a white fluffy solid. Purity based on LC-MS 66%.
LRMS (m/z): 2358 [M-4]⁴⁻
LC-MS r.t. (min): 2.13^{6C}

### Intermediate 11:

### dendron(-L-SO1861)⁴-maleimide2

Scaffold 2 (5.10 mg, 5.00 µmol) was dissolved in methanol (100 µL). Next, a freshly prepared 1 N Sodium hydroxide solution (22.0 µL, 22.0 µmol) was added and the mixture was shaken for 1 min and left standing at room temperature. After 30 min a 1.0 M trimethylphosphine solution in THF (25.0 µL, 25.0 µmol) was added and the resulting mixture was shaken for 1 min and left standing at room temperature. After 30 min the reaction mixture was evaporated *in vacuo* and co-evaporated with methanol (2 × 5 mL). The resulting residue was dissolved in a mixture of 20 mM NH₄HCO₃ with 0.5 mM TCEP/acetonitrile (3:1, v/v, 3.242 mL). From this solution, directly, 1000 µL was added to SO1861-EMCH (14.4 mg, 6.94 pmol, 4.5 equiv. compared to the scaffold) and the mixture was shaken for 1 min and left standing at room temperature. After 10 min the reaction mixture was lyophilized overnight. To the resulting residue 2,5-Dioxopyrrolidin-1-yl 3-(2-(2-(3-(2,5-dioxo-2h-pyrrol-1(5h)-yl)propanamido)ethoxy)ethoxy)propanoate (5.84 mg, 0.014 mmol) and DMF(1.00 mL) were added. Next, DIPEA (2.39 µL, 0.014 mmol) was added and the suspension was shaken for 1 min and left standing at room temperature. After 2 hours the reaction mixture was subjected to preparative LC-MS.^{3C} Fractions corresponding to the product were immediately pooled together, frozen and lyophilized overnight to give the title compound (10.9 mg, 85%) as a white fluffy solid. Purity based on LC-MS 80%.
LRMS (m/z): 2354 [M-4]⁴⁻
LC-MS r.t. (min): 4.16^{5B}

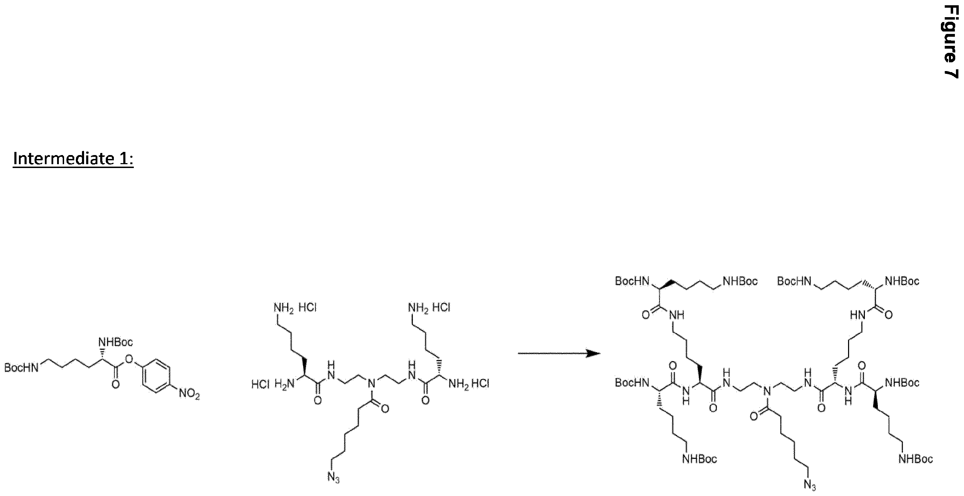

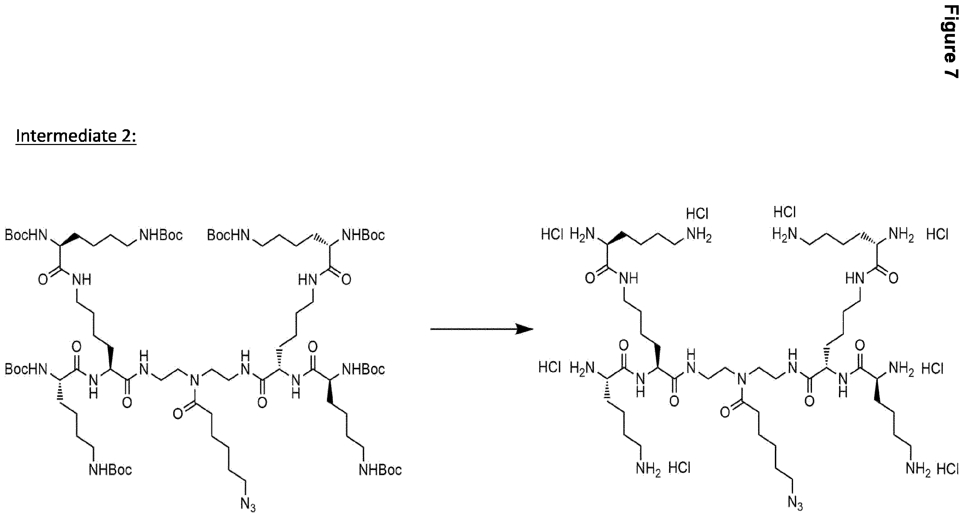

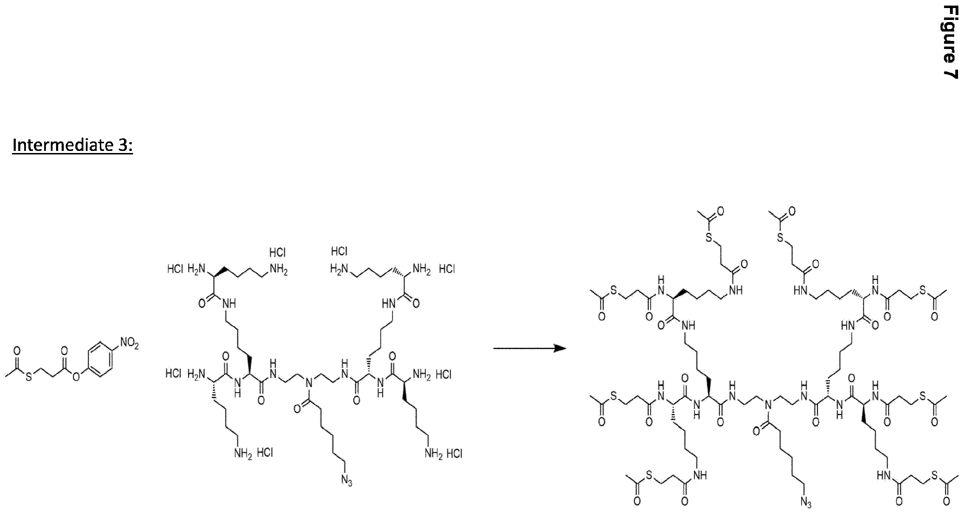

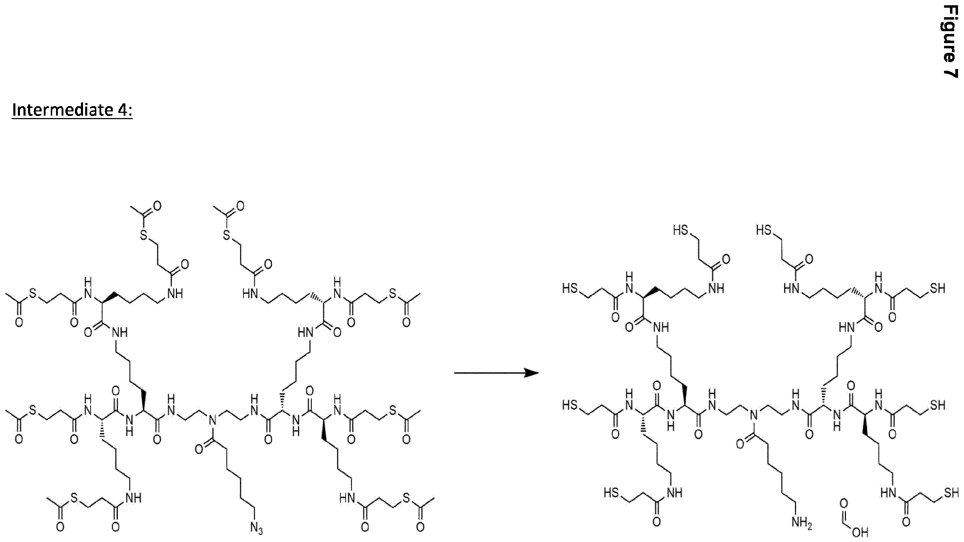

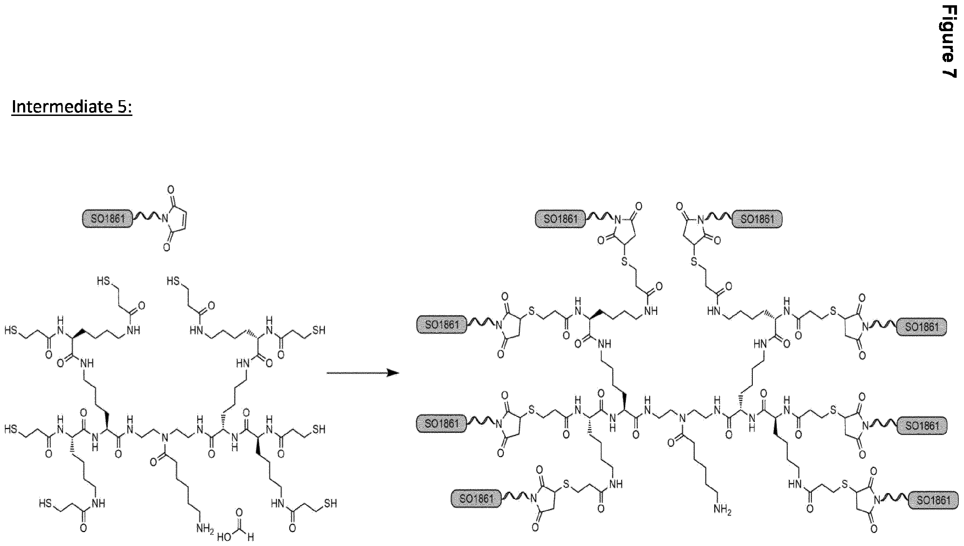

### Dendron(-L-SO1861)⁸synthesis (Figure 7)

### Intermediate 1:

### tert-butyl N-[(1S)-1-{[(1S)-1-{[2-(6-azido-N-{2-[(2S)-2,6-bis[(2S)-2,6-bis({[(tert-butoxy)carbonyl]amino})hexanamido]hexanamido]ethyl}hexanamido)ethyl]carbamoyl}-5-[(2S)-2,6-bis({[(tert-butoxy)carbonyl]amino})hexanamido]pentyl]carbamoyl}-5-{[(tert-butoxy)carbonyl]amino}pentyl]carbamate

(S)-2,6-diamino-N-(2-(6-azido-N-(2-((S)-2,6-diaminohexanamido)ethyl)hexanamido)ethyl)hexanamide tetrahydrochloride (964 mg, 1.50 mmol) was dissolved in DMF (25.0 mL) and triethylamine (2.08 mL, 15.0 mmol). Next, Boc-Lys(Boc)-ONp (3.36 g, 7.18 mmol) was added and the reaction mixture was stirred at room temperature overnight. The reaction mixture was evaporated *in vacuo* and the residue was purified by flash chromatography (DCM - methanol/DCM (1/9, v/v) gradient 100:0 rising to 0:100) to give the title product (2.71 g, 100%) as a white solid. Purity based on LC-MS 97%.
LRMS (m/z): 807 [M-198]²⁺
LC-MS r.t. (min): 2.35^{2B}

### Intermediate 2:

### (2S,2'S)-N,N'-((5S,15S,22S)-22,26-diamino-10-(6-azidohexanoyl)-15-((S)-2,6-diaminohexanamido)-6,14,21-trioxo-7,10,13,20-tetraazahexacosane-1,5-diyl)bis(2,6-diaminohexanamide) octahydrochloride

Intermediate 1 (2.71 g, 1.50 mmol) was dissolved in HCl in isopropanol (5-6N, 25.0 ml, 138 mmol) and the reaction mixture was stirred at room temperature overnight. Next, the reaction mixture was evaporated *in vacuo* and the resulting crude product was co-evaporated with DCM (3 × 20 mL) to give the crude title product as a white solid.
LRMS (m/z): 203/254 [M-200/M+4]⁴⁺, 338 [M+3]³⁺, 507 [M+2]²⁺, 1012 [M+1]¹⁺

### Intermediate 3:

### (2S)-2,6-bis[3-(acetylsulfanyl)propanamido]-N-[(1S)-1-{[2-(6-azido-N-{2-[(2S)-2,6-bis[(2S)-2,6-bis[3-(acetylsulfanyl)propanamido]hexanamido]hexanamido]ethyl}hexanamido)ethyl]carbamoyl}-5-[(2S)-2,6-bis[3-(acetylsulfanyl)propanamido]hexanamido]pentyl]hexanamide

To (2S,2'S)-N,N'-((5S,15S,22S)-22,26-diamino-10-(6-azidohexanoyl)-15-((S)-2,6-diaminohexanamido)-6,14,21-trioxo-7,10,13,20-tetraazahexacosane-1,5-diyl)bis(2,6-diaminohexanamide) octahydrochloride (300 mg, 0.230 mmol) was added DMF (20.0 mL), triethylamine (320 µl, 2.30 mmol) and 4-nitrophenyl 3-(acetylthio)propanoate (595 mg, 2.21 mmol). The resulting suspension was sonicated at 60 °C for 30 min and left stirring at room temperature overnight. The reaction mixture was evaporated *in vacuo* and the residue was purified by first performing flash chromatography (DCM - methanol/DCM (1/9, v/v) gradient 100:0 rising to 0:100), followed by preparative MP-LC² to give the title product (70 mg, 15%) as a white solid. Purity based on LC-MS 100%.
LRMS (m/z): 685 [M+3]³⁺
LC-MS r.t. (min): 1.91^{2A}

### Intermediate 4:

### (2S)-N-[(1S)-1-{[2-(6-amino-N-{2-[(2S)-2,6-bis[(2S)-2,6-bis(3-sulfanylpropanamido)hexanamido]hexanamido]ethyl}hexanamido)ethyl]carbamoyl}-5-[(2S)-2,6-bis(3-sulfanylpropanamido)hexanamido]pentyl]-2,6-bis(3-sulfanylpropanamido)hexanamide formate

Scaffold 4 (10.0 mg, 4.87 µmol) was dissolved methanol (200 µL). Next, a freshly prepared 1 N Sodium hydroxide solution (42.9 µL, 0.043 mmol) was added and the resulting mixture was shaken for 1 min and left standing at room temperature. After 30 min a 1.0 M trimethylphosphine solution in THF (24.4 µL, 0.024 mmol) was added and the resulting mixture was shaken for 1 min and left standing at room temperature. After 30 min the reaction mixture was diluted with water (1 mL) and directly subjected to preparative MP-LC.² Fractions corresponding to the product were immediately pooled together, frozen and lyophilized overnight to give the title compound (4.02 mg, 48%) as a white fluffy solid.
LRMS (m/z): 564 [M+3]³⁺, 846 [M+2]²⁺
LC-MS r.t. (min): 1.54^{2C}

### Intermediate 5:

### dendron(-L-SO1861)⁸-amine

Scaffold 5 (0.52 mg, 0.299 µmol) and SO1861-EMCH (29.2 mg, 0.014 mmol) were dissolved in a mixture of 20 mM NH₄HCO₃ with 0.5 mM TCEP/acetonitrile (3:1, v/v, 1.00 mL) and the resulting mixture was shaken for 1 min and left standing at room temperature. After 30 min TCEP (0.30 mg, 1.05 µmol) was added and the reaction mixture was shaken for 1 min. Next, the mixture was directly subjected to preparative LC-MS.^{3B} Fractions corresponding to the product were immediately pooled together, frozen and lyophilized overnight to give the title compound (2.17 mg, 40%) as a white fluffy solid. Purity based on LC-MS 97%.
LRMS (m/z): 2282 [M-8]⁸⁻, 2607 [M-7]⁷⁻
LC-MS r.t. (min): 4.41^{5A}

### SO1861-BNA oligo conjugation

HSP27 BNA oligo disulfide (1.10 mg, 0.187 µmol) was dissolved in 20 mM NH₄HCO₃ with 1.0 mM TCEP (500 µL) and the mixture was shaken for 1 min and left standing at room temperature. After 1 hour the reaction mixture was filtered by using a centrifugal filter with a molecular weight cut-off of 3000 Da (14000 × g for 30 min). The residue solution was diluted with 20 mM NH₄HCO₃ with 1.0 mM TCEP (500 µL) and the resulting mixture was filtered again under the same conditions described above. The residue solution was diluted with 20 mM NH₄HCO₃/acetonitrile (3:1, v/v, 1.00 mL) and the resulting mixture was added to SO1861-EMCH (3.54 mg, 0.375 µmol). The reaction mixture was shaken for 1 min and left standing at room temperature. After 10 min the reaction mixture was subjected to preparative LC-MS.^{4A} Fractions corresponding to the product were immediately pooled together, frozen and lyophilized overnight to give the title compound (1.25 mg, 85%) as a white fluffy solid. Purity based on LC-MS 100%.
LRMS (m/z): 1561 [M-5]⁵⁻, 1951 [M-4]⁴⁻
LC-MS r.t. (min): 2.46^{6B}

### dendron(SO1861)⁴-BNA oligo conjugation

HSP27 BNA oligo disulfide (1.1 mg, 0.187 µmol) was dissolved in 20 mM NH₄HCO₃ with 1.0 mM TCEP (500 µL) and the mixture was shaken for 1 min and left standing at room temperature. After 1 hour the reaction mixture was filtered by using a centrifugal filter with a molecular weight cut-off of 3000 Da (14000 × g for 30 min). The residue solution was diluted with 20 mM NH₄HCO₃ with 1.0 mM TCEP (500 µL) and the resulting mixture was filtered again under the same conditions described above. The residue solution was diluted with 20 mM NH₄HCO₃/acetonitrile (3:1, v/v, 1.0 mL) and the resulting mixture was added to dendron(SC1861)4-maleimide1 (3.54 mg, 0.375 µmol). The reaction mixture was shaken for 1 min and left standing at room temperature. After 10 min the reaction mixture was subjected to preparative LC-MS.^{4A} Fractions corresponding to the product were immediately pooled together, frozen and lyophilized overnight to give the title compound (1.25 mg, 85%) as a white fluffy solid. Purity based on LC-MS 94%
LRMS (m/z): 1896 [M-8]⁸⁻, 2167 [M-7]⁷⁻
LC-MS r.t. (min): 3.77^{6B}

### Cell culture

Cells were seeded in DMEM (PAN-Biotech GmbH) supplemented with 10% fetal bovine serum (PAN-Biotech GmbH) and 1% penicillin/streptomycin (PAN-Biotech GmbH), in a 96 well plate at 5,000 c/w in 100 µL/well and incubated overnight at 37 °C and 5% CO₂. The next day samples were prepared in DMEM and cells were treated.

### Gene silencing

RNA isolation and Qpcr analysis was performed according to standard procedures and protocols. HSP27 primers: F: R:

### HSP27BNA oligo

HSP27BNA(-thiol) oligos (sequence 5'-GGCacagccagtgGCG-3') (Zhang et al., 2011) were ordered at Bio-synthesis Inc. (Lewisville, Texas)

### Results

BNAoligo, antisense BNA oligo targeting the mRNA transcript of the cancertarget (upregulated in cancer cells), heat shock protein 27 (HSP27BNA) was conjugated to SO1861-EMCH (HSP27BNA-L-SO1861) or dendron(-L-SO1861)⁴ (HSP27BNA-dendron(-L-SO1861)⁴) and co-administrated to an A431 cancer cell line, according to the invention. As readout, gene silencing of HSP27 mRNA in A431 cells was determined. This revealed that HSP27BNA-L-SO1861 treatment resulted in an improvement of HSP27 gene silencing activity compared to the HSP27BNA alone, whereas the activity of HSP27BNA-dendron(-L-SO1861)⁴ (4 SO1861 molecules/BNA) is even stronger (3-fold) compared to the gene silencing activity of HSP27BNA alone (Figure 1). This shows that conjugation of 1 or more SO1861 molecules improves the gene silencing activity of the therapeutic BNA oligo nucleotide due to the enhancement of SO1861-mediated endosomal escape and cytoplasmic delivery of the antisense BNA.

### Example 3

### Materials

The following chemicals were used as purchased: methanol (MeOH, LiChrosolv, Merck), N-ε-maleimidocaproic acid hydrazide (EMCH, 95%, TCI Chemicals), trifluoroacetic acid (TFA, 99.8%, Carl Roth), 2-mercaptoethanol (98%, Sigma-Aldrich), poly(amidoamine) (PAMAM dendrimer, ethylenediamine core, generation 5.0 solution, Sigma-Aldrich), cyanine 3 carboxylic acid (Cy3-COOH, 95%, Lumiprobe), 1-[Bis(dimethylamino)methylene]-1H-1,2,3-triazolo[4,5-b]pyridinium 3-oxid hexafluorophosphate, N-[(Dimethylamino)-1H-1,2,3-triazolo-[4,5-b]pyridin-1-ylmethylene]-N-methylmethanaminium hexafluorophosphate N-oxide (HATU, 97%, Sigma-Aldrich), bovine serum albumin fraction V (BSA, Carl Roth), dimethylsulfoxide (DMSO, 99%, Carl Roth), 2-lminothiolane hydrochloride (98%, Sigma-Aldrich), rhodamine b (RhodB, 95%, Merck), Dulbecco's phosphate buffered saline (PBS, Gibco), hydrochloric acid (HCl, 37%, Merck), NHS-PEG₁₃-DBCO (Click Chemistry Tools), Alexa Fluor^{™} 488 5-TFP (Thermo-Fischer), azido-PEG3-SS-NHS (Conju-Probe), sodium cyanoborohydride (NaCNBH₃, 95 %, Sigma-Aldrich), ammonium persulfate (APS, 98%, Sigma-Aldrich), N,N,N',N'-tetramethylethylenediamine (TMEDA, 99 %, Sigma-Aldrich), customized peptide SESDDAMFCDAMDESDSK (95%, PeptideSynthetics), azido-dPEG₁₂-NHS (95%, Quanta Biodesign), PFd-G4-Azide-NH-BOC Dendron (G4-dendron, 95%, Polymer Factory), Cyanin5-DBCO (Cy5-DBCO, 95%, Lumiprobe), Chloroform (CHCl₃, 99.5 %, Sigma), Amicon Ultra 0.5 mL centrifugal filters (3 kDa MWCO, Sigma), mPEG-SCM (mPEG₂ₖ-NHS, 95.6%, Creative PEG Works), Amicon Ultra 15 mL centrifugal filters (10 kDa MWCO, Sigma).

### Methods

### MALDI-TOF-MS

MALDI-TOF spectra were recorded on a MALDI-Mass Spectrometer (Bruker Ultrafex III). Typically, the sample dissolved in MilliQ water in nanomolar to micromolar range was spotted on the target (MTP 384 target plate polished steel T F, Bruker Daltons) using either super-DHB (99%, Fluka) or sinapinic acid (SA, 99%, Sigma-Aldrich) as the matrix dissolved in acetonitrile (MADLI-TOF-MS tested, Sigma) / 0.1% TFA (7:3 v/v) via the dried-droplet-method. PepMix (Peptide Calibration Standard, Bruker Daltons) or ProteMass (Protein Calibration Standard, Sigma-Aldrich) served as calibration standards. RP mode refers to reflector positive mode. RN mode refers to reflector negative mode. LP mode refers to linear positive mode.

### H-NMR

¹H NMR analysis was performed using a Bruker 400 MHz NMR spectrometer. The sample preparation, in which 2 mg of sample had been dissolved in 0.8 mL of methanol-*D₄* (99%, Deutero), was performed 24 h prior to the measurement.

### UV-Vis

UV-Vis measurements were performed on a NanoDrop ND-1000 spectrophotometer in the spectral range of 200-750 nm.

### Size Exclusion Chromatography

Size exclusion chromatography (SEC) was performed with Sephadex G 25 Superfine from GE Healthcare and on prepacked PD10 columns (GE Healthcare, Sephadex G 25 M). The material was activated by swelling in the respective eluent prior to performing chromatography.

### Dialysis

Regenerated cellulose membranes: MWCO = 1 and 2 kDa (Spectra/Por), and MWCO = 12-14 kDa (Carl Roth) were used to perform dialysis. Typically, dialysis was carried out for 24 h with 1 L of solvent that was exchanged after first 6 h of the process.

### Lyophilization

Freeze-drying was performed on an Alpha 1-2 LD plus (Martin Christ Gefriertrocknungsanlagen GmbH). Typically, samples were frozen with liquid nitrogen and placed into the freeze-dryer at high vacuum.

### SO1861-EMCH synthesis

SO1861 from Saponaria officinalis L (59 mg, 31.7 µmol) and EMCH (301 mg, 888 µmol) were placed in a round flask with stirrer and dissolved in 13 mL methanol. TFA (400 µL, cat.) was added to the solution and the reaction mixture was stirred for 3 h at 800 rpm and room temperature on a RCT B magnetic stirrer (IKA Labortechnik). After stirring for 3 h, the mix was diluted either with MilliQ water or PBS and dialyzed extensively for 24 h against either with MilliQ water or PBS using regenerated cellulose membrane tubes (Spectra/Por 7) with a MWCO of 1 kDa. After dialysis, the solution was lyophilized to obtain a white powder. Yield 62.4 mg (95 %). Dried aliquots were further used for characterization via ¹H NMR and MALDI-TOF-MS.

¹H NMR (400 MHz, methanol-*D*₄) (Figure 3 A, SO1861): *δ* = 0.50-5.50 (m, saponin triterpenoid and sugar backbone protons), 9.43 (1H, s, aldehyde proton of saponin, H^{a}).

¹H NMR (400 MHz, methanol-*D*₄) (Figure 3 B. SO1861-EMCH, PBS workup): *δ* = 0.50-5.50 (m, saponin triterpenoid and sugar backbone protons), 6.79 (2 H, s, maleimide protons, H^{c}), 7.62-7.68 (1 H, m, hydrazone proton, H^{b}).

MALDI-TOF-MS (RP mode) (Figure 4 A): *m*/*z* 2124 Da ([M+K]⁺, saponin-EMCH), *m*/*z* 2109 Da ([M+K]⁺, SO1861-EMCH), *m*/*z* 2094 Da ([M+Na]⁺, SO1861-EMCH)
MALDI-TOF-MS (RN mode) (Figure 5 C): *m*/*z* 2275 Da ([M-H]⁻, saponin-EMCH conjugate), 2244 Da ([M-H]⁻, saponin-EMCH conjugate), 2222 Da ([M-H]⁻, saponin-EMCH conjugate), 2178 Da ([M-H]⁻, saponin-EMCH conjugate), 2144 Da ([M-H]⁻, saponin-EMCH conjugate), 2122 Da ([M-H]⁻, saponin-EMCH conjugate), 2092 Da ([M-H]⁻, saponin-EMCH conjugate), 2070 Da ([M-H]⁻, SO1861-EMCH), 2038 Da ([M-H]⁻, SO1832-EMCH), 1936 Da ([M-H]⁻, SO1730-EMCH), 1861 Da ([M-H]⁻, SO1861).

### Cy3-PAMAM

720 µL PAMAM dissolved in methanol (30 mg, 1.04 µmol) was placed into a 250 mL round flask and methanol was removed via a rotary evaporator (20 mbar, 60 °C). Remaining PAMAM was dissolved in 9 mL DMSO. HATU (7.6 mg, 20 µmol) dissolved in 0.5 mL DMSO was added to a Cy3-COOH (0.6 mg, 1.2 µmol) solution in DMSO and the mix was shaken for 1 h at 800 rpm at room temperature on a ThermoMixer C (Eppendorf). After shaking for 1 h, the HATU-Cy3 solution was added to the stirring PAMAM solution and the reaction mix was stirred for 12 h at room temperature. After stirring for 12 h, the reaction mix was diluted with MilliQ water and dialyzed extensively for 24 h against MilliQ water using regenerated cellulose membrane tubes (Spectra/Por 6) with a MWCO of 2 kDa. After dialysis, the volume of the conjugate solution was reduced via a rotary evaporator (20 mbar, 60 °C) and the concentrated conjugate solution was run through a Sephadex G25 superfine size exclusion column (16 mL column volume). The first fraction was collected and lyophilized to obtain the viscous pink PAMAM-Cy3 conjugate. PAMAM-Cy3 conjugate formation was confirmed by chromatography on thin layer chromatography (methanol/water, v/v 1:1), and the appearance of a faster band on a Sephadex G 25 superfine column. Yield 21.3 mg (63 %). The dye per PAMAM molar ratio determined by UV-Vis spectrophotometry was 0.43.

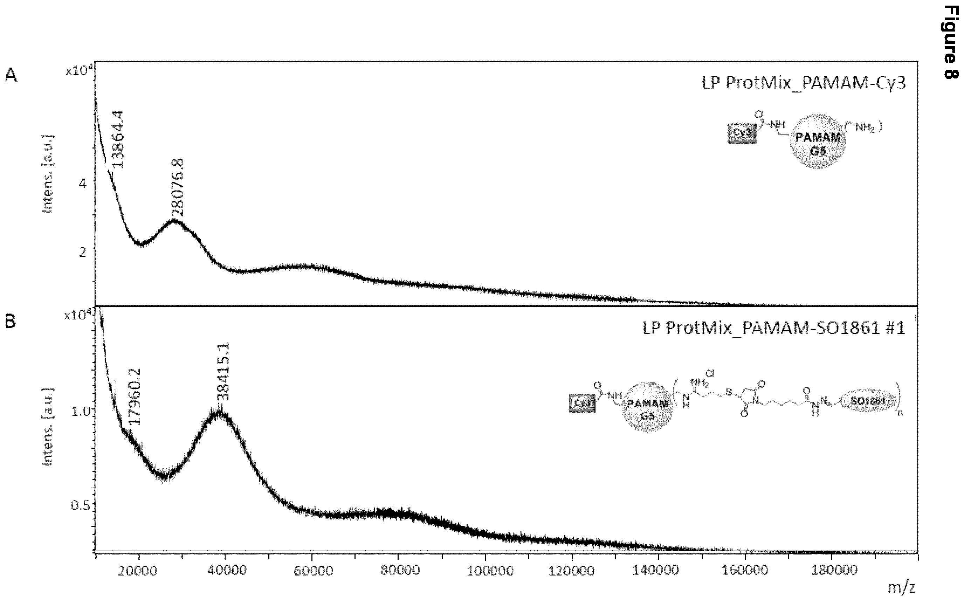

MALDI-TOF-MS (Figure 8 A) (LP mode): *m*/*z* 28.0 kDa ([M+H]⁺, Cy3-PAMAM).

### Cy3-PAMAM-SO1861 synthesis

Procedure is described exemplary for Cy3-PAMAM-(SO1861)s. 2-iminothiolane (1 mg, 6.7 µmol) dissolved in 250 µL MilliQ water was added to a PAMAM-Cy3 solution (0.5 mg, 17 nmol) in 125 µL MilliQ water and the mix was shaken for 40 min at 800 rpm and room temperature on a ThermoMixer C (Eppendorf). After shaking for 40 min, the reaction mix was immediately run through a Sephadex G25 superfine size exclusion column (16 mL column volume) and SO1861-EMCH (176 µg, 85 nmol) dissolved in 40 µL MilliQ water was added to the collected Cy3-PAMAM-SH fraction. The reaction mixture was shaken for 12 h at 800 rpm and room temperature on a ThermoMixer C (Eppendorf). After shaking for 12 h, the reaction mix was diluted with MilliQ water and dialyzed extensively for 24 h against MilliQ water using regenerated cellulose membrane tubes (ZelluTrans, Carl Roth) with a MWCO of 12 - 14 kDa. After dialysis, the Cy3-PAMAM-SO1861 solution was concentrated using centrifugal filtration at 4000 rpm (15 °C) via Amicon Ultra 15 filters with a MWCO of 3 kDa. The conjugate was stored as solution in the fridge and aliquots were taken for analysis. Yield: 0.5 mg (75%).

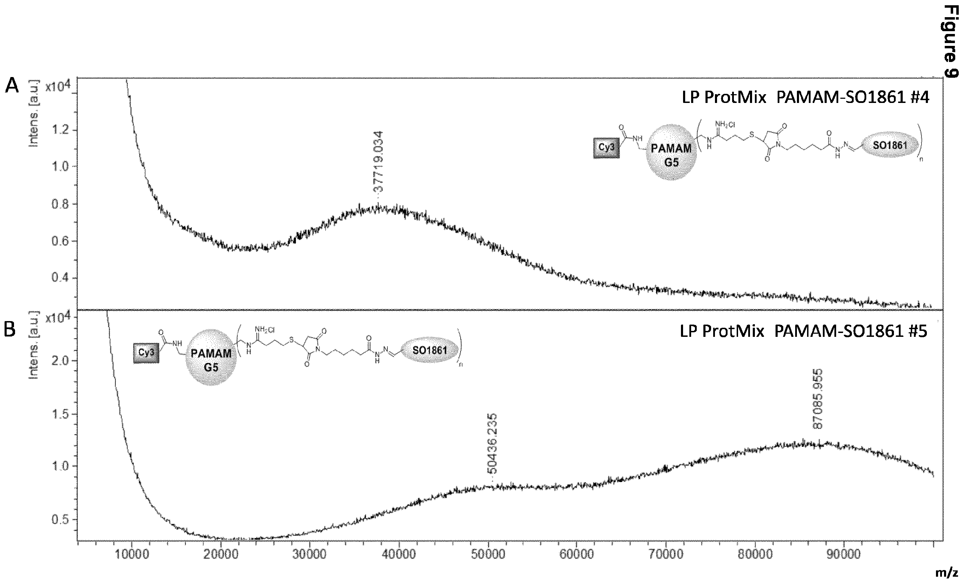

MALDI-TOF-MS spectra are illustrated in Figures 8 B-D, and Figure 9. MALDI-TOF-MS of Cy3-PAMAM-(SO1861)₆ (Figure 8 B) (LP mode): *m*/*z* 38.4 kDa ([M+H]⁺, Cy3-PAMAM-SO1861), 17.9 kDa ([M+H]²⁺, Cy3-PAMAM-SO1861).

The synthesis of Cy3-PAMAM-(SO1861)₅, Cy3-PAMAM-(SO1861)₁₃, Cy3-PAMAM-(SO1861)₅₁, and Cy3-PAMAM-(SO1861)₂₇, has been performed via the above described methodology but differ in the feed equivalents of the starting materials 2-iminothiolane and SO1861-EMCH. The respective feed equivalents of the starting materials and the respective mass of the conjugates are highlighted in Table 1.

**Table 1. Reaction parameter for Cy3-PAMAM-SO1861 synthesis.**

| **2-Iminothiolane feed equivalents to Cy3-PAMAM** | **SO1861-EMCH feed equivalents to Cy3-PAMAM** | **Mass of conjugate via MALDI-TOF-MS** | **SO1861 molecules attached per PAMAM** | **Resulting conjugate** |
|---|---|---|---|---|
| 384 | 6 | 38.7 kDa | ∼5 | Cy3-PAMAM-(SO1861)₆, Figure 8 B |
| 384 | 20 | 53.9 kDa | ∼13 | Cy3-PAMAM-(SO1861)₁₃, Figure 8 C |
| 384 | 57 | 133.9 kDa | ∼51 | Cy3-PAMAM-(SO1861)₅₁, Figure 8 D |
| 8 | 5 | 37.7 kDa | ∼5 | Cy3-PAMAM-(S01861)₅, Figure 9 A |
| 32 | 30 | 87.0 kDa | ∼27 | Cy3-PAMAM-(SO1861)₂₇, Figure 9 B |

### Cy3-PAMAM-NC-SO1861 synthesis

Cy3-PAMAM (0.5 mg, 18 nmol), SO1861 (2.3 mg, 1.24 µmol), and HATU (64.6 mg, 170 µmol) were dissolved separately in 200 µL DMSO. SO1861 and HATU solutions were mixed and shaken for 20 min at 800 rpm and room temperature on a ThermoMixer C (Eppendorf). After shaking for 20 min, Cy3-PAMAM solution was added to the shaking SO1861-HATU solution and the reaction mixture was allowed to shake for 12 h at 800 rpm and room temperature on a ThermoMixer C (Eppendorf). After shaking for 12 h, the reaction mix was diluted with MilliQ water and dialyzed extensively for 24 h against MilliQ water using regenerated cellulose membrane tubes (ZelluTrans, Carl Roth) with a MWCO of 12-14 kDa. After dialysis, the Cy3-PAMAM-NC-SO1861 solution was concentrated using centrifugal filtration at 4,000 rpm (15 °C) via Amicon Ultra 15 filters with a MWCO of 3 kDa. The Cy3-PAMAM-NC-(SO1861)₁₇ conjugate was stored as solution in the fridge and aliquots were taken for analysis. Yield: 0.77 mg (69%).

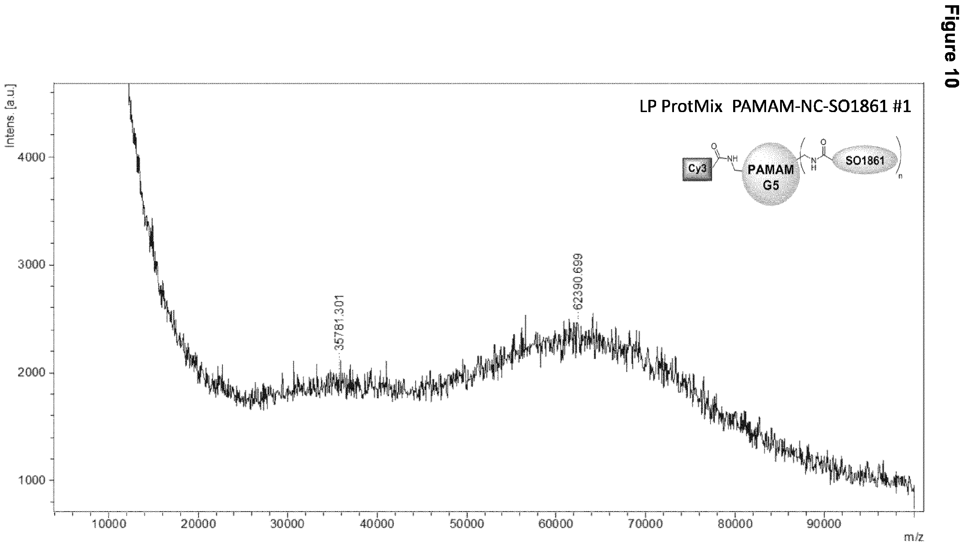

MALDI-TOF-MS (Figure 10) (LP mode): *m*/*z* 62.3 kDa ([M+H]⁺, Cy3-PAMAM-NC-SO1861), 35.7 kDa ([M+H]²⁺, Cy3-PAMAM-NC-SO1861).

### PAMAM thiolation

Procedure is described exemplary for the highest PAMAM to 2-iminothiolane ratio. To a PAMAM (333 µg, 12.8 nmol) solution dissolved in 30 µL methanol 2-iminothiolane (0.53 mg, 3.84 µmol) dissolved in 128 µL MilliQ water was added. The reaction mixture was shaken for 12 h at 800 rpm and room temperature on a ThermoMixer C (Eppendorf). After shaking for 12 h, the reaction mix was washed 4 times with MilliQ water via centrifugal filtration using Amicon Ultra centrifugal filters (3 kDa MWCO) at 15 °C and 13500 rpm. After washing the sample was lyophilized to obtain a white solid. Yield was not determined.

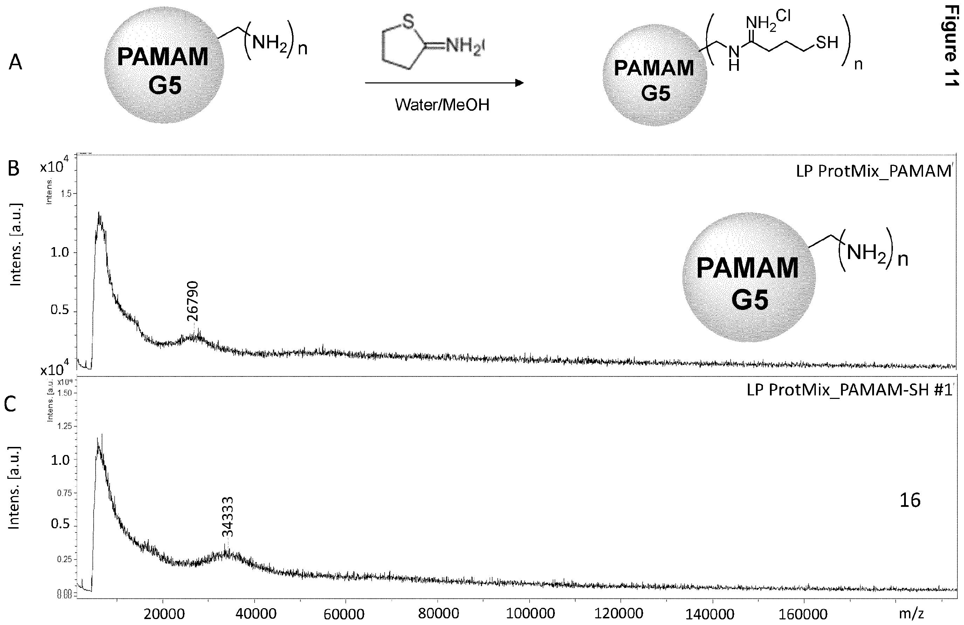

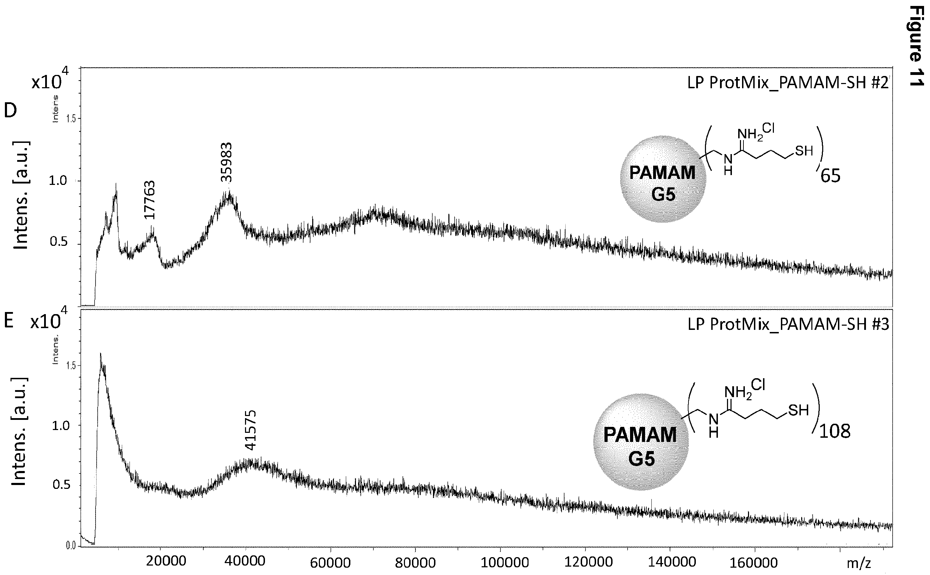

MALDI-TOF-MS spectra are illustrated in Figure 11. MALDI-TOF-MS of PAMAM-(SH)₁₀₈ (Figure 11 C) (LP mode): *m*/*z* 41.5 kDa ([M+H]⁺, PAMAM-[SH]₁₀₈).

The synthesis of other PAMAM-iminothiolane conjugates has been performed via the above described methodology but differs in the feed equivalents of the starting material 2-iminothiolane. For the lowest 2-iminothiolane feed reaction Cy3-PAMAM has been used.

The respective feed equivalents of the starting materials and the respective mass of the conjugates are highlighted in Table 2.

**Table 2. Reaction parameter for PAMAM-SH synthesis.**

| **2-Iminothiolane feed equivalents to PAMAM** | **Mass of conjugates via MALDI-TOF-MS** | **Iminothiolane molecules attached per PAMAM** | **Resulting MS spectrum** |
|---|---|---|---|
| 50 | 34.4 kDa | ∼ 16 | Fig. 11 C |
| 100 | 35.9 kDa | ∼ 65 | Fig. 11 D |
| 300 | 41.5 kDa | ∼108 | Fig. 11 E |

### PAMAM PEGylation

Procedure is described exemplary for the lowest PAMAM to mPEG₂ₖ ratio. To a PAMAM (333 µg, 12.8 nmol) solution dissolved in 10 µL DMSO mPEG₂ₖ-NHS (0.268 mg, 128 nmol) dissolved in 13 µL DMSO was added. The reaction mixture was shaken for 12 h at 800 rpm and room temperature on a ThermoMixer C (Eppendorf). After shaking for 12 h, the reaction mix was diluted with MilliQ water and dialyzed extensively for 24 h against MilliQ water using regenerated cellulose membrane tubes (Spectra/Por 6) with a MWCO of 2 kDa. After dialysis, the batch was concentrated via centrifugal filtration using Amicon Ultra 15 mL centrifugal filters (10 kDa MWCO). The concentrated batch was run through a PD10 size exclusion column followed by lyophilization to obtain a white fluffy powder. Yield was not determined.

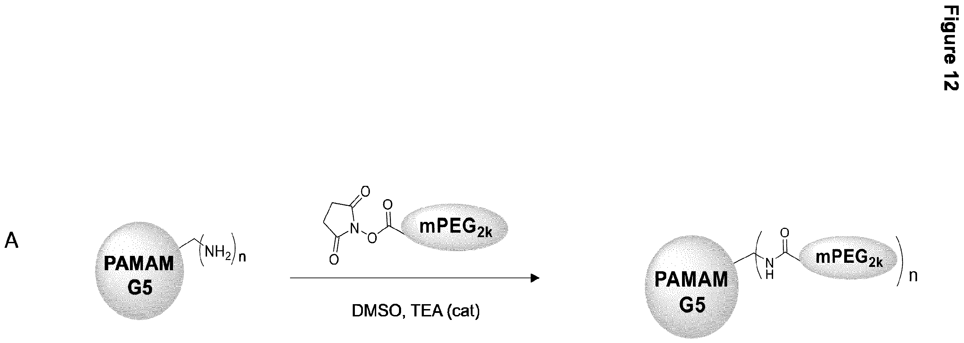

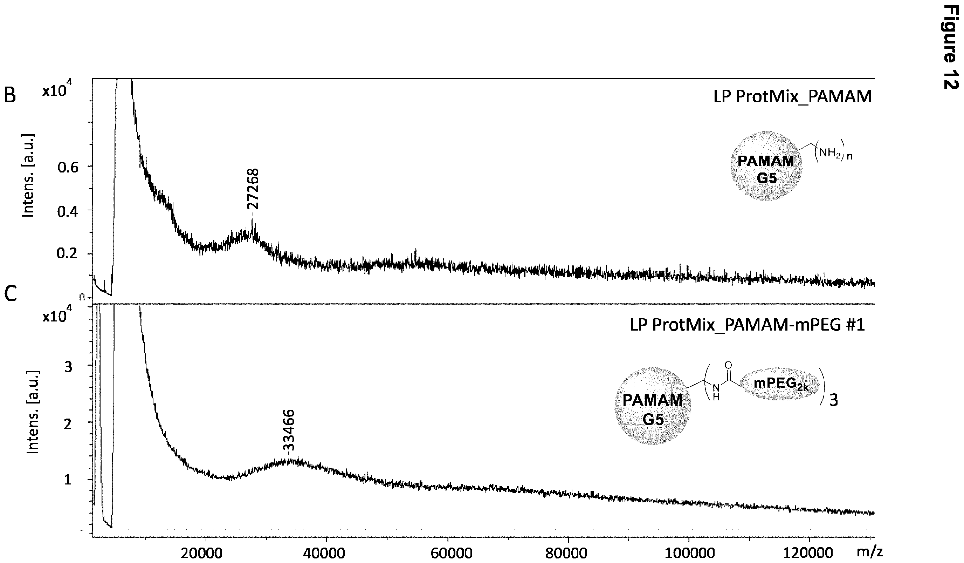

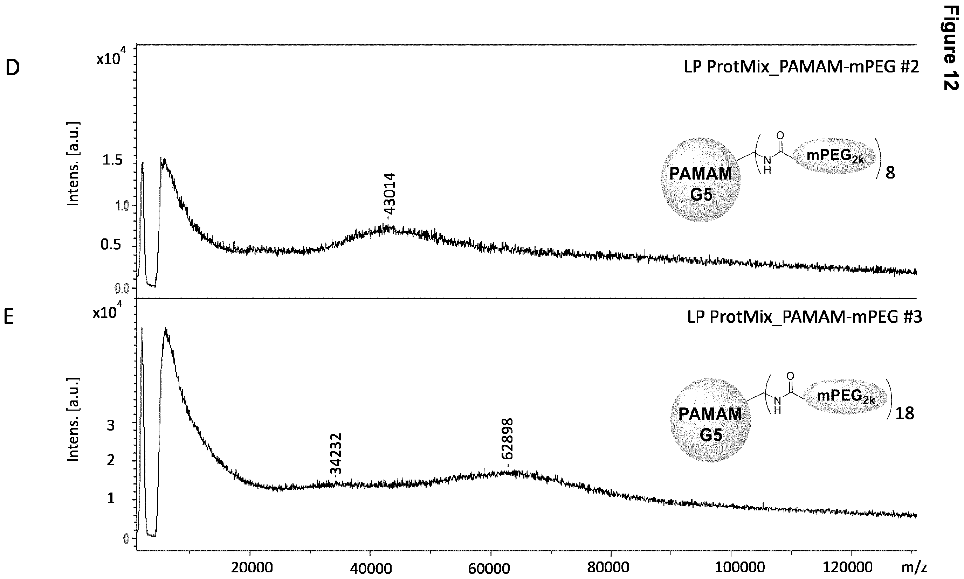

MALDI-TOF-MS spectra are illustrated in Figure 12. MALDI-TOF-MS of PAMAM-(mPEG₂ₖ)₃ (Figure 12 C) (LP mode): *m*/*z* 33.46 kDa ([M+H]⁺, PAMAM-[mPEG₂ₖ]₃).

The synthesis of other PAMAM-mPEG₂ₖ conjugates has been performed via the above described methodology but differs in the feed equivalents of the starting material mPEG₂ₖ-NHS. The respective feed equivalents of the starting materials and the respective mass of the conjugates are highlighted in Table 3.

**Table 3. Reaction parameter for PAMAM-mPEG₂ₖ synthesis.**

| **mPEG₂ₖ-NHS feed equivalents to PAMAM** | **Mass of conjugates via MALDI-TOF-MS** | **mPEG₂ₖ molecules attached per PAMAM** | **Resulting MS spectrum** |
|---|---|---|---|
| 10 | 28.5 kDa | ∼ 3 | Figure 12 C |
| 20 | 43.0 kDa | ∼ 8 | Figure 12 D |
| 100 | 62.8 kDa | ∼ 18 | Figure 12 E |

### Cy3-PAMAM-SO1861-DBCO synthesis

Procedure is described exemplary for Cy3-PAMAM-(SO1861)₂₇-(DBCO)₁₀. Cy3-PAMAM-(SO1861)₂₇ (0.41 mg, 4.71 nmol) was freeze-fried and dissolved in 100 µL DMSO. DBCO-PEG₁₃-NHS ester (0.197 mg, 188 nmol) dissolved in DMSO was added to the Cy3-PAMAM-SO1861 solution and the mixture was shaken at 800 rpm and room temperature on a ThermoMixer C (Eppendorf). After shaking for 3 h, the reaction mix was diluted with MilliQ water and dialyzed extensively for 24 h against MilliQ water using regenerated cellulose membrane tubes (ZelluTrans, Carl Roth) with a MWCO of 12-14 kDa. After dialysis, the Cy3-PAMAM-SO1861-DBCO solution was concentrated using centrifugal filtration at 4,000 rpm (15 °C) via Amicon Ultra 15 filters with a MWCO of 3 kDa. The conjugate was stored as solution in the fridge and aliquots were taken for analysis. Yield: 0.1 mg (22%).

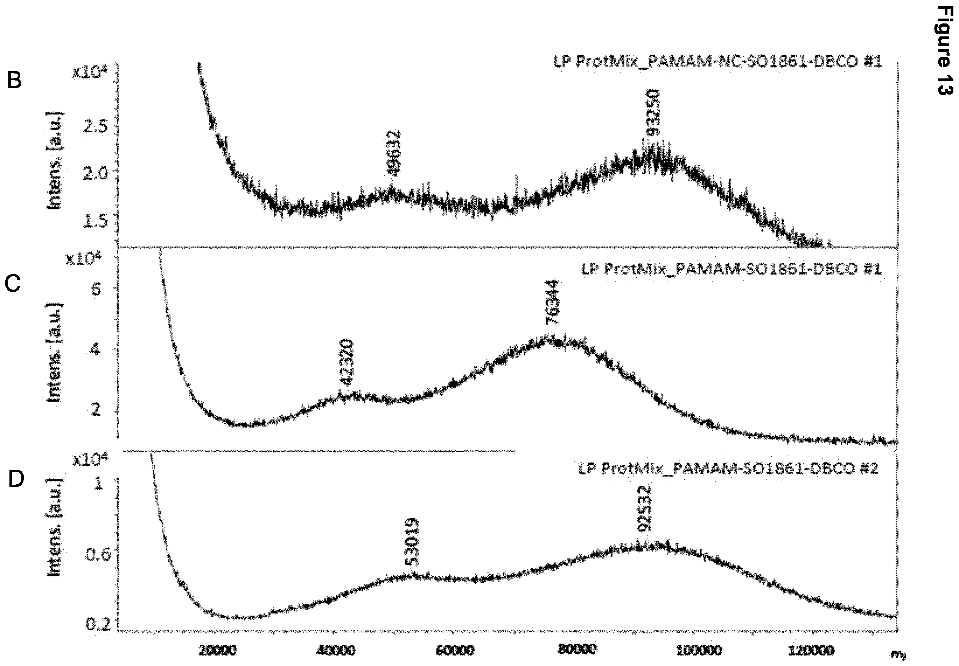

MALDI-TOF-MS (Figure 13 D) (LP mode): *m*/*z* 92.5 kDa ([M+H]⁺, Cy3-PAMAM-SO1861-DBCO), 53.0 kDa ([M+H]²⁺, Cy3-PAMAM-SO1861-DBCO).

The synthesis of Cy3-PAMAM-(SO1861)₅-(DBCO)₃₈, and Cy3-PAMAM-(SO1861)₂₇-(DBCO)₁₀, have been performed via the above described methodology. The respective feed equivalents of the starting material and the respective mass of the conjugates are highlighted in Table 4.

**Table 4. Reaction parameter for Cy3-PAMAM-SO1861-DBCO synthesis.**

| **Used Cy3-PAMAM-saponin batch** | **DBCO-PEG13-NHS feed equivalents** | **Mass via MALDI-TOF-MS** | **DBCO molecules attached per PAMAM** | **Resulting conjugate** |
|---|---|---|---|---|
| Cy3-PAMAM-(SO1861)₅ | 40 | 76.3 kDa | ∼38 | Cy3-PAMAM-(SO1861)₅-(DBCO)₃₈, Figure 13 C |
| Cy3-PAMAM-(SO1861)₂₇ | 40 | 92.5 kDa | ∼10 | Cy3-PAMAM-(SO1861)₂₇-(DBCO)₁₀, Figure 13 D |

### Cy3-PAMAM-NC-SO1861-DBCO synthesis

Cy3-PAMAM-NC-(SO1861)₁₇ (0.3 mg, 4.8 nmol) was freeze-fried and dissolved in 100 µL DMSO. DBCO-PEG₁₃-NHS ester (0.202 mg, 194 nmol) dissolved in DMSO was added to the Cy3-PAMAM-NC-SO1861 solution and the mixture was shaken at 800 rpm and room temperature on a ThermoMixer C (Eppendorf). After shaking for 3 h, the reaction mixwas diluted with MilliQ water and dialyzed extensively for 24 h against MilliQ water using regenerated cellulose membrane tubes (ZelluTrans, Carl Roth) with a MWCO of 12-14 kDa. After dialysis, the Cy3-PAMAM-SO1861-DBCO solution was concentrated using centrifugal filtration at 4,000 rpm (15 °C) via Amicon Ultra 15 filters with a MWCO of 3 kDa. The conjugate was stored as solution in the fridge and aliquots were taken for analysis. Yield: 0.1 mg (22%). Mass spectrometry indicates the conjugation of 30 DBCO moieties per PAMAM molecule.

MALDI-TOF-MS (Figure 13 B) (LP mode): *m*/*z* 93.2 kDa ([M+H]⁺, Cy3-PAMAM-NC-SO1861-DBCO), 49.6 kDa ([M+H]²⁺, Cy3-PAMAM-NC-SO1861-DBCO).

### EGFDianthin and dianthin expression

Plasmid-DNA (His-dianthin-EGF-pET11d or His-dianthin-pET1 1d) [20] was transformed into chemically competent Escherichia coli NiCo21 (DE3) (New England Biolabs^{®}, Inc.) and grown in 3 mL lysogeny broth supplemented with 50 µg/mL ampicillin at 37 °C for 5 h at 200 rpm. These bacteria were used to inoculate 500 mL lysogeny broth supplemented with 50 µg/mL ampicillin for overnight culture at 37 °C. Subsequently, the culture volume was scaled up to 2 L and bacteria were grown until an optical density (A600) of 0.9. Protein expression was induced by the addition of isopropyl β-D-1-thiogalactopyranoside (IPTG) at a final concentration of 1 mM. Cells were further grown for 3 h at 37 °C and 200 rpm. After centrifugation (5 min, 5,000 g, 4 °C) cell pellets were resuspended in 20 mL phosphate buffered saline (Dulbecco's phosphate-buffered saline (PBS) with Ca²⁺ and Mg²⁺, pH 7.4) and stored at -20 °C. After thawing, proteins were released by ultrasound device (Branson Sonifier 250, G. Heinemann). The solution was centrifuged (15,800 × g, 30 min, 4 °C) and adjusted to 20 mM imidazole concentration. The construct contained an N-terminal His-tag and was purified by nickel nitrilotriacetic acid chromatography (Ni-NTA Agarose, Qiagen, Hilden, Germany). After elution with imidazole (20-250 mM) the eluates were analyzed by sodium dodecyl sulfate polyacrylamide gel electrophoresis (SDS-PAGE) (12%). Fractions containing dianthin-EGF or dianthin were dialyzed against 2 L chitin binding domain buffer (20 mM tris(hydroxymethyl)-aminomethane/HCl, 500 mM NaCl, 1 mM EDTA, 0.1% Tween-20, pH 8.0) at 4 °C. Further purification by chitin column affinity chromatography served to remove bacterial proteins with binding activity for Ni-NTA agarose. After elution with chitin binding domain buffer, the fractions were analyzed by SDS-PAGE (12%). Fractions containing dianthin-EGF or dianthin were dialyzed against 5 L PBS at 4 °C. Purified proteins were concentrated by Amicon centrifugal filter devices (10 kDa, Millipore, Eschborn, Germany). The protein concentration was determined by a bicinchoninic acid assay (Pierce, Rockford, USA).

### Dianthin-EGF-Alexa488 synthesis

Dianthin-EGF (240 µg, 6.7 nmol) solution in PBS was placed into an Amicon Ultra 15 filter with a MWCO of 3 kDa and centrifuged at 4,000 rpm and 4 °C for 30 min three times. After each cycle, the Amicon filter was refilled with 0.1 M sodium carbonate buffer at pH 9. After the third centrifugation cycle, the volume was reduced to 0.5 mL via centrifugation. The dianthin-EGF sodium carbonate solution was placed into a 2 mL reaction tube and Alexa Fluor^{™} 488 5-TFP (50 µg, 56 nmol) dissolved in 10 µL DMSO was added to the protein solution. The mix was shaken at 800 rpm and room temperature on a ThermoMixer C (Eppendorf) for 80 min. After shaking, the mix was run through a Sephadex G25 M size exclusion column (GE Healthcare, PD10 column). The dianthin-EGF-Alexa488 conjugate was stored in solution in 0.1 M sodium carbonate buffer at pH 9 in the fridge and aliquots were taken for analysis. Yield: 210 µg (85%).

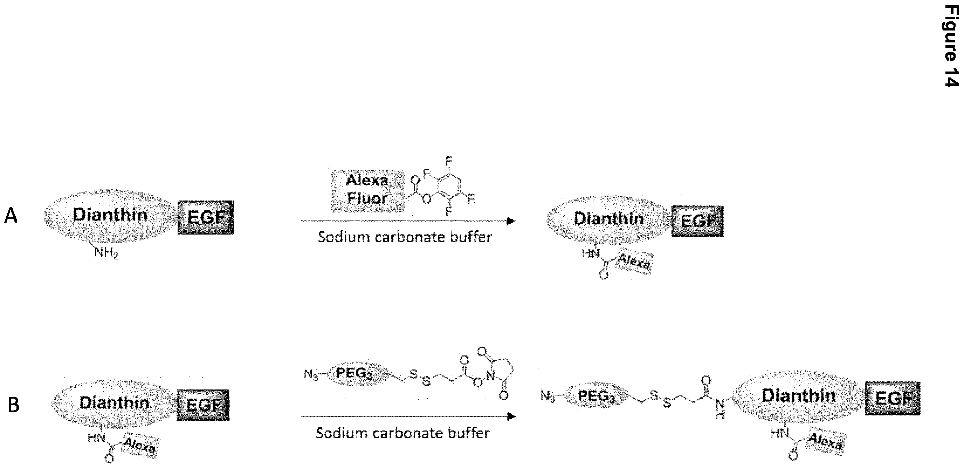

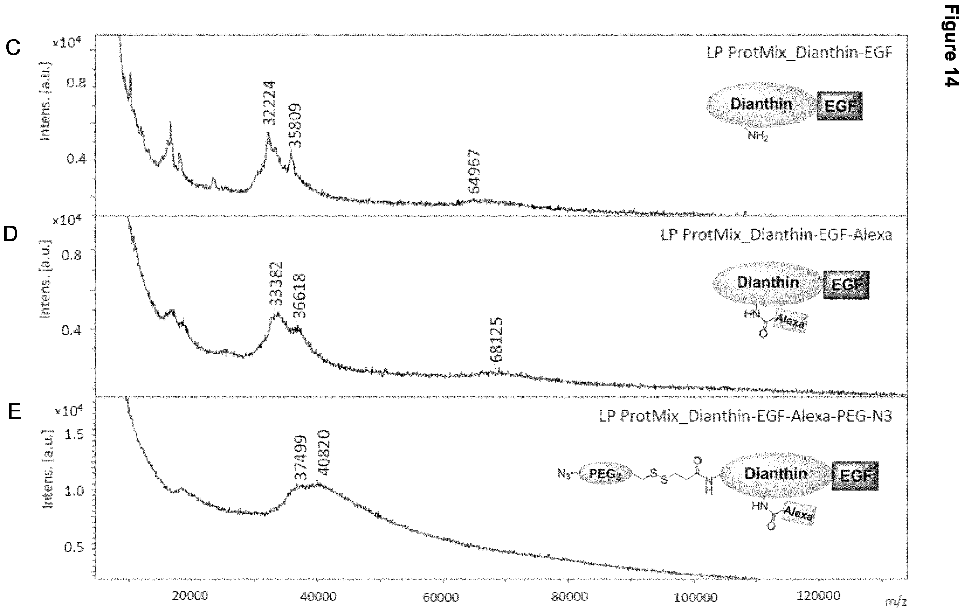

MALDI-TOF-MS (Figure 14 D) (LP mode): *m*/*z* 36.8 kDa ([M+H]⁺, dianthin-EGF-Alexa488), *m*/*z* 33.6 kDa ([M+H]⁺, dianthin-EGF-Alexa488), 18.8 kDa ([M+H]²⁺, dianthin-EGF-Alexa488), 16.6 kDa ([M+H]²⁺, dianthin-EGF-Alexa488).

### Dianthin-Alexa488 synthesis

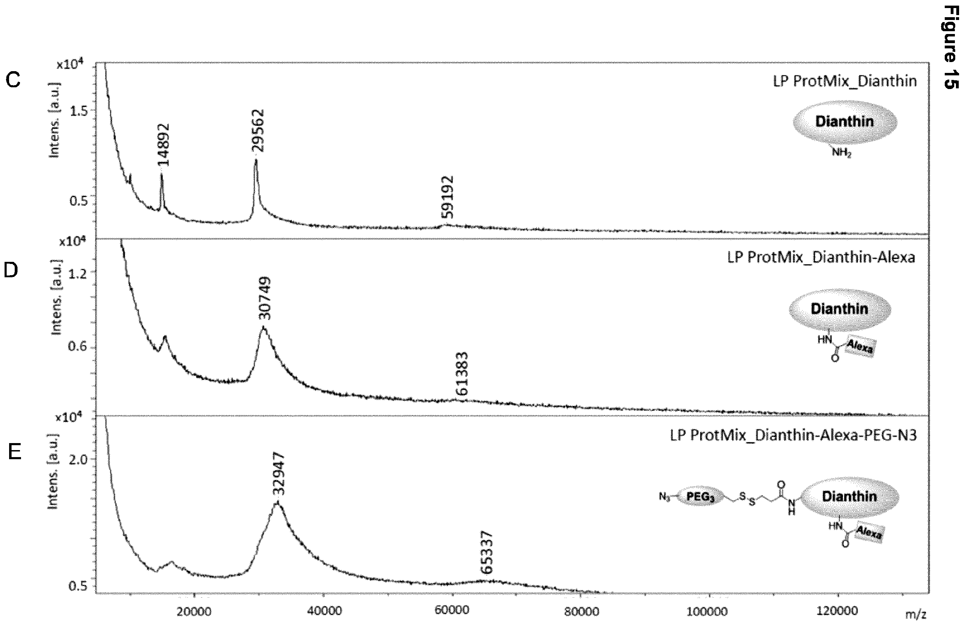

Dianthin (184 µg, 6.2 nmol) solution in PBS was placed into an Amicon Ultra 15 filter with a MWCO of 3 kDa and centrifuged at 4,000 rpm and 4 °C for 30 min three times. After each cycle, the Amicon filter was refilled with 0.1 M sodium carbonate buffer at pH 9. After the third centrifugation cycle, the volume was reduced to 0.5 mL via centrifugation. The dianthin sodium carbonate solution was placed into a 2 mL reaction tube and Alexa Fluor^{™} 488 5-TFP (16.7 µg, 19 nmol) dissolved in 3.5 µL DMSO was added to the protein solution. The mix was shaken at 800 rpm and room temperature on a ThermoMixer C (Eppendorf) for 80 min. After shaking, the mixwas run through a Sephadex G25 M size exclusion column (GE Healthcare, PD 10 column). The dianthin-Alexa488 conjugate was stored in solution in 0.1 M sodium carbonate buffer at pH 9 in the fridge and aliquots were taken for analysis. Yield: not determined MALDI-TOF-MS (Figure 15 D) (LP mode): *m*/*z* 30.7 kDa ([M+H]⁺, dianthin-Alexa488).

### Dianthin-EGF-Alexa488-S-S-PEG-N₃, and Dianthin-EGF-Alexa488-PEG₁₂-N₃ synthesis

Procedure is described exemplary for dianthin-EGF-Alexa488-S-S-PEG-N₃. Dianthin-EGF-Alexa488 (70 µg, 1.9 nmol) sodium carbonate solution was placed into a 2 mL reaction tube and azido-PEG₃-S-S-NHS (120 µg, 272 nmol) dissolved in 9 µL DMSO was added to the protein solution. The mix was shaken at 800 rpm and 15 °C on a ThermoMixer C (Eppendorf) for 12 h. After shaking, the reaction mix was diluted with PBS and was washed with PBS via centrifugal filtration at 4,000 rpm and 4 °C using Amicon Ultra 15 filter with a MWCO of 3 kDa.
Yield: 54 µg (70%).

MALDI-TOF-MS (Figure 14 E) (LP mode): *m*/*z* 40.8 kDa ([M+H]⁺, dianthin-EGF-Alexa488-S-S-PEG-N₃), *m*/*z* 37.5 kDa ([M+H]⁺, dianthin-EGF-Alexa488-S-S-PEG-N₃).

The synthesis of dianthin-EGF-Alexa488-S-S-PEG-N₃, and dianthin-EGF-Alexa488-PEG₁₂-N₃ have been performed via the above described methodology but differed in the used azido-PEG linker. The respective azido-PEG linker, their feed equivalents, and the respective mass of the conjugates are highlighted in Table 5.

**Table 5. Reaction parameter for dianthin-EGF-Alexa488-PEG-N₃ synthesis**

| **Used toxin batch** | **Azido-PEG linker used** | **Azido-PEG linker feed equivalents** | **Mass of conjugate via MALDI-TOF-MS** | **Resulting conjugate** |
|---|---|---|---|---|
| Dianthin-EGF-Alexa488 | Azido-PEG₃-S-S-NHS | 135 | 40.8 kDa | Dianthin-EGF-Alexa488-S-S-PEG₃-N₃ |
| Dianthin-EGF-Alexa488 | Azido-PEG₁₂-NHS | 135 | 43.3 kDa | Dianthin-EGF-Alexa488-PEG₁₂-N₃ |

### Dianthin-Alexa488-S-S-PEG-N₃

Dianthin-Alexa488 (24.5 µg, 0.8 nmol) sodium carbonate solution was placed into a 2 mL reaction tube and azido-PEG₃-S-S-NHS (34 µg, 78 nmol) dissolved in 9 µL DMSO was added to the protein solution. The mix was shaken at 800 rpm and 15 °C on a ThermoMixer C (Eppendorf) for 12 h. After shaking, the reaction mix was diluted with PBS and was washed with PBS via centrifugal filtration at 4,000 rpm and 4 °C using Amicon Ultra 15 filter with a MWCO of 3 kDa.

Yield: 10.3 µg (39%).

MALDI-TOF-MS (Figure 15 E) (LP mode): *m*/*z* 32.9 kDa ([M+H]⁺, dianthin-Alexa488-S-S-PEG-N₃).

### Cy3-PAMAM-Saponin-Toxin conjugate synthesis

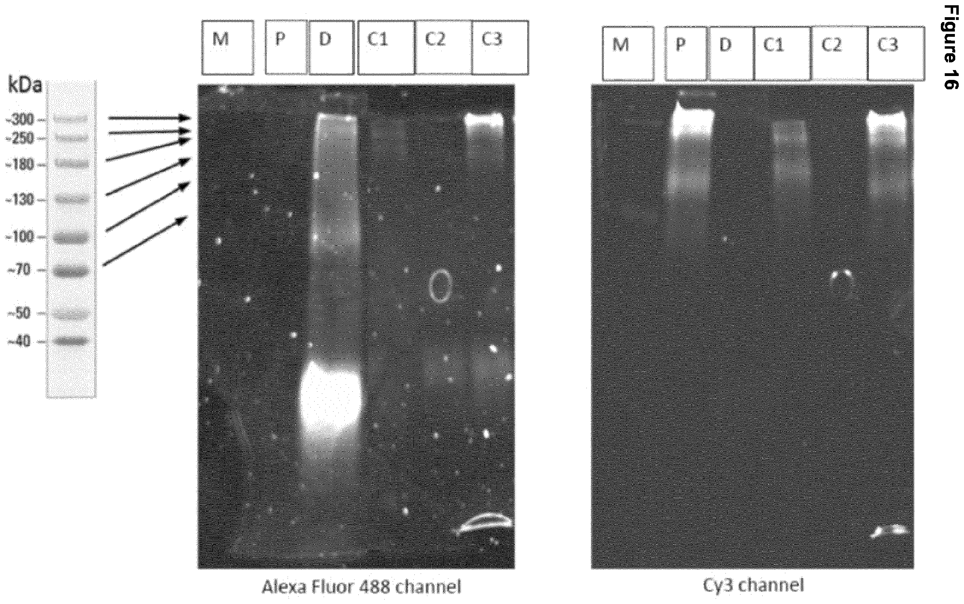

Procedure is described exemplary for Cy3-PAMAM-(SO1861)₂₇-DBCO. Cy3-PAMAM-(SO1861)₂₇-DBCO (17 µg, 0.184 nmol) solution in MilliQ water was mixed with a dianthin-EGF-Alexa488-SS-PEG3-N₃ (3.6 µg, 0.089 nmol) solution in PBS in a 1.5 mL reaction tube and the reaction mix was shaken at 800 rpm and 15 °C on a ThermoMixer C (Eppendorf) for 2 h. After shaking, small aliquots were taken out for analysis via SDS-PAGE and fluorescence imaging on a Molecular Imager^{®} VersaDoc^{™} MP 4000 imaging system (Bio-Rad) (Figure 16).

The synthesis of Cy3-PAMAM-(SO1861)₅-S-S-Dianthin-EGF-Alexa488, Cy3-PAMAM-(SO1861)₂₇-S-S-Dianthin-EGF-Alexa488, Cy3-PAMAM-NC-(SO1861)₁₇-S-S-Dianthin-EGF- Alexa488, Cy3-PAMAM-NC-(SO1861)₁₇-S-S-Dianthin-Alexa488, and Cy3-PAMAM-NC-(SO1861)₁₇-Dianthin-EGF-Alexa488, have been performed via the above described methodology but differ in the used PAMAM-saponin-DBCO batch, the used azido-toxin batch, and their feed equivalents. The respective feed equivalents of the starting materials are highlighted in Table 6.

**Table 6. Reaction parameter for Cy3-PAMAM-saponin-toxin synthesis.**

| **PAMAM-saponin-DBCO batch used** | **PAMAM-saponin-DBCO feed equivalents** | **Azido-toxin batch used** | **Azido-toxin feed equivalents** | **Resulting conjugate** |
|---|---|---|---|---|
| Cy3-PAMAM-(SO1861)₅-(DBCO)₃₈ | 3 | Dianthin-EGF-Alexa488-S-S-PEG₃-N₃ | 1 | Cy3-PAMAM-(SO1861)₅-S-S-Dianthin-EGF-Alexa488 |
| Cy3-PAMAM-(SO1861)₂₇-(DBCO)₁₀ | 2.1 | Dianthin-EGF-Alexa488-S-S-PEG₃-N₃ | 1 | Cy3-PAMAM-(SO1861)₂₇-S-S-Dianthin-EGF-Alexa488 |
| Cy3-PAMAM-NC-(SO1861)₁₇-(DBCO)₃₀ | 2.3 | Dianthin-EGF-Alexa488-S-S-PEG₃-N₃ | 1 | Cy3-PAMAM-NC-(SO1861)₁₇-S-S-Dianthin-EGF-Alexa488 |
| Cy3-PAMAM-NC-(SO1861)₁₇-(DBCO)₃₀ | 7.1 | Dianthin-Alexa488-S-S-PEG₃-N₃ | 1 | Cy3-PAMAM-NC-(SO1861)₁₇-S-S-Dianthin-Alexa488 |
| Cy3-PAMAM-NC-(SO1861)₁₇-(DBCO)₃₀ | 2.3 | Dianthin-EGF-Alexa488-PEG₁₂-N₃ | 1 | Cy3-PAMAM-NC-(SO1861)₁₇-Dianthin-EGF-Alexa488 |

### Cy3-PAMAM-NC-SO1861 synthesis via reductive amination

Cy3-PAMAM (0.19 mg, 13 nmol) and SO1861 (0.73 mg, 0.39 µmol) were dissolved separately in 200 µL 0.1 M acetate buffer at pH 5. SO1861 and Cy3-PAMAM solutions were mixed and shaken for 20 min at 800 rpm and room temperature on a ThermoMixer C (Eppendorf). After shaking for 20 min, NaCNBH₃ (5 mg, 81 µmol) was added to the shaking reaction solution and the reaction mixture was allowed to shake for 12 h at 800 rpm and room temperature on a ThermoMixer C (Eppendorf). After shaking for 12 h, the reaction mix was diluted with MilliQ water and dialyzed extensively for 24 h against MilliQ water using regenerated cellulose membrane tubes (ZelluTrans, Carl Roth) with a MWCO of 12-14 kDa. After dialysis, the Cy3-PAMAM-NC-SO1861 solution was concentrated using centrifugal filtration at 4,000 rpm (15 °C) via Amicon Ultra 15 filters with a MWCO of 3 kDa. The conjugate was stored as solution in the fridge and aliquots were taken for analysis. Yield: not determined

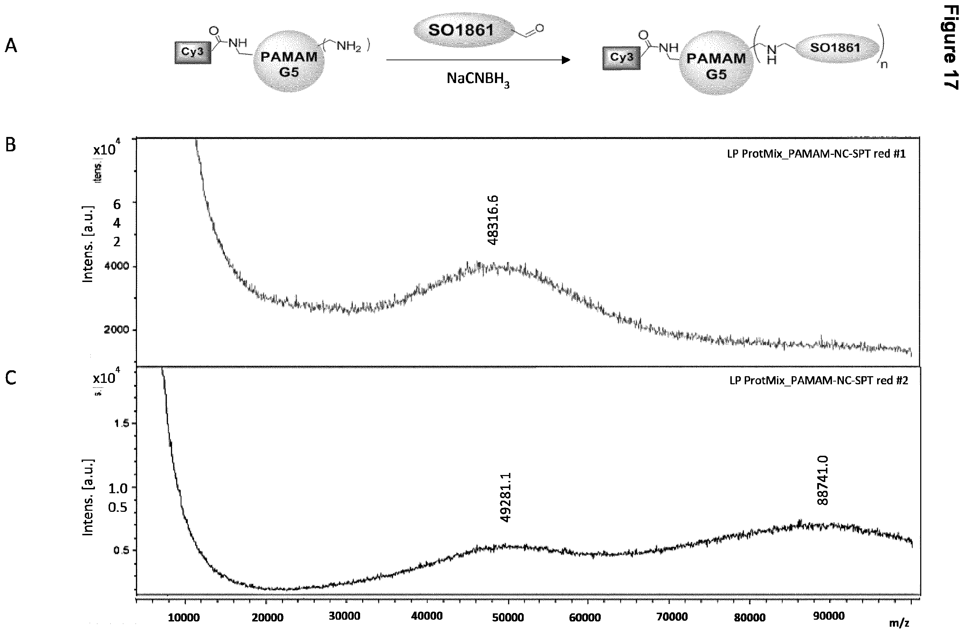

MALDI-TOF-MS (Figure 17 B, C) (LP mode): *m*/*z* 88.7 kDa ([M+H]⁺, Cy3-PAMAM-NC-SO1861), 49.2 kDa ([M+H]²⁺, Cy3-PAMAM-NC-SO1861).

The synthesis of Cy3-PAMAM-NC-(SO1861)₃₀, and Cy3-PAMAM-NC-(SO1861)₁₀, have been performed via the above described methodology but differed in the time after which the reducing agent NaCNBH₃ was added to the reaction batch. The respective time of the NaCNBH₃ addition and the respective mass of the conjugates are highlighted in Table 7.

**Table 7. Reaction parameter Cy3-PAMAM-NC-SO1861 synthesis via reductive amination.**

| **Time of shaking reaction mix before NaCNBH₃ addition** | **Mass via MALDI-TOF-MS** | **SO1861 molecules attached per PAMAM** | **Resulting conjugate** |
|---|---|---|---|
| 20 min | 88.8 kDa | ∼30 | Cy3-PAMAM-NC-(SO1861)₃₀, Figure 17 C |
| 12 h | 48.0 kDa | ∼10 | Cy3-PAMAM-NC-(SO1861)₁₀, |
| | | | Figure 17 B |

### SO1861-EMCH peptide coupling

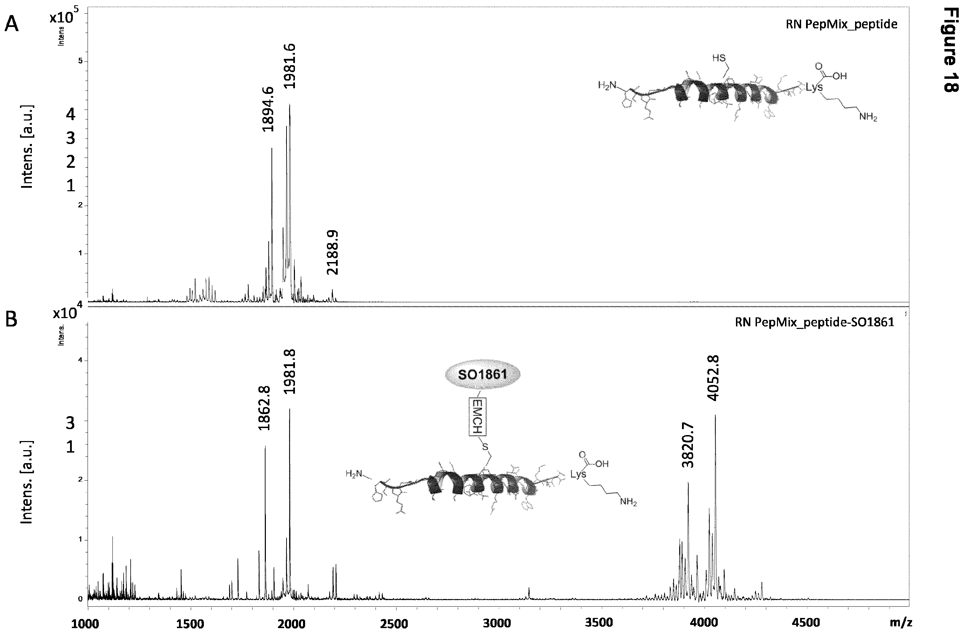

Customized peptide with the sequence SESDDAMFCDAMDESDSK (0.6 mg, 0.3 µmol) and SO1861-EMCH (0.8 mg, 0.39 µmol) were dissolved separately in 200 µL PBS. SO1861-EMCH and peptide solutions were mixed and shaken for 12 h at 800 rpm and room temperature on a ThermoMixer C (Eppendorf). After shaking small aliquots were taken out for analysis. Yield: not determined MALDI-TOF-MS (Figure 18 B) (RN mode): *m*/*z* 4.05 kDa ([M+H]⁻, peptide-SO1861), 3.92 kDa ([M+H]⁻, peptide-SO1730), 1.98 kDa ([M+H]⁻, peptide), 1.86 kDa ([M+H]⁻, SO1861).

### Results

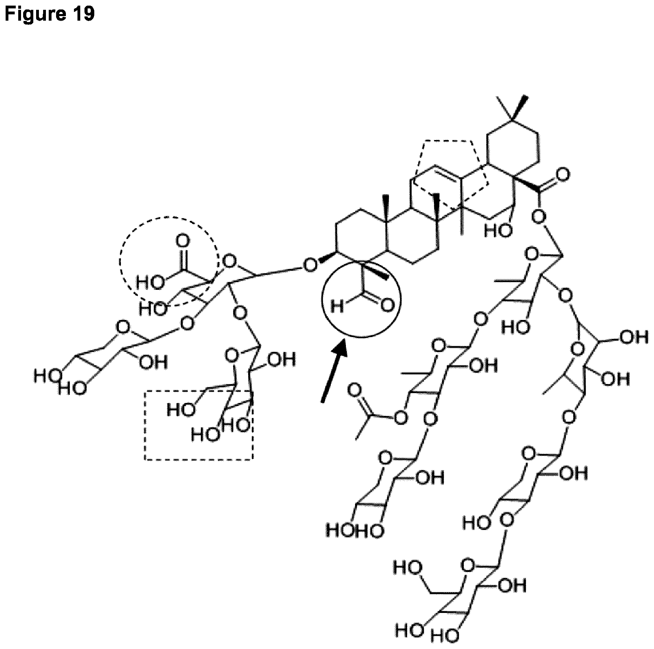

Considering available chemical groups for conjugation reactions to the SO1861 molecule, four chemical groups have been identified. The alcohols and diols of the sugar residues, the aldehyde group on the triterpenoid backbone, the carboxylic acid on one of the sugar residues (glucuronic acid), and the alkene group on the triterpenoid backbone as highlighted in Figure 19.

In view of the pros and cons of each identified chemical group (Table 8), the aldehyde and alcohol groups are best suitable for reversible conjugation reactions, while the alkene and the carboxylic acid (glucuronic acid) are the groups best suitable for irreversible / stable conjugation reactions. The aldehyde group within the molecule structure of SO1861, however, is the most suitable for reversible conjugation reactions over the alcohols. On the one hand, because there is only one aldehyde present in the structure that allows chemoselective reactions. On the other hand, because the aldehyde can perform reversible conjugation reactions with a variety of chemical groups such as amines, hydrazides, and hydroxylamines forming acid-cleavable moieties like imines, hydrazones, and oximes. This factor enables a freedom of choice over the chemical group for the desired reversible conjugation reaction. Contrary, the alcohols are good candidates for reversible conjugation reaction via the formation of acetals and ketals as well, but lack in chemoselectivity since they are present in a large quantity on the glycosidic structure.

For the formation of an irreversible and stable bond the carboxylic acid is the most suitable since it can form amides and esters with the common tools used in peptide chemistry (e.g. reaction with amines via carbodiimide mediated amide formation).

**Table 8. Functional groups that are available for saponin conjugation reactions**

| **Functional Group** | **Pros** | **Cons** |
|---|---|---|
| **Alcohol (Diols)** | - Suitable for reversible acetal/ketal formation | - Acetal/ketal formation without chemoselectivity |
| | - Suitable for ester formations with activated carboxylic acids | - Ester formation without chemoselectivity |
| **Aldehyde** | - Suitable for chemoselective reversible hydrazone formation with hydrazides | |
| | - Suitable for chemoselective reversible imine formation with amines | - Not suitable for acetal formation in the presence of unprotected saponin sugar diols |
| | - Suitable for chemoselective reversible oxime formation with hydroxylamines | |
| **Alkene** | - Suitable for chemoselective irreversible radical reactions | - Not suitable for reversible conjugation reactions |
| | | - Not suitable for reactions involving a hydrogenation step |
| **Carboxylic acid** | - Suitable for chemoselective amide / ester formation with amines and alcohols after activation | - Not suitable for reversible conjugation reactions under mild conditions |

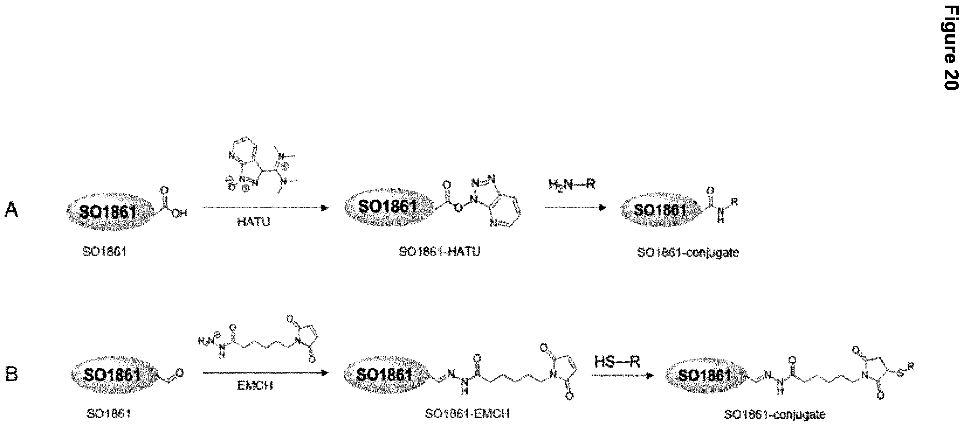

Thus, for the development of an endosomal escape enhancing saponin (such as SO1861) a methodology has been established that allows the generation of a non-cleavable and cleavable 'ready to conjugate' saponins (Figure 20) using the most suitable chemical groups present on SO1861.

For producing non-cleavable `ready to conjugate' saponins the carboxylic group of SO1861 is activated via a reagent used in peptide coupling chemistry to generate an active ester (e.g. 1-[Bis(dimethylamino)methylene]-1H-1,2,3-triazolo[4,5-b]pyridinium 3-oxide hexafluorophosphate, HATU). The resulting active ester of SO1861 is able to react with amines forming stable amide bonded conjugates (Figure 20 A).

For producing cleavable 'ready to conjugate' saponins the aldehyde group of SO1861 is reacted with an EMCH (ε-maleimidocaproic acid hydrazide) linker. The hydrazide group of EMCH forms an acid cleavable hydrazone bond with the aldehyde of SO1861. At the same time the EMCH linker presents a maleimide group that is thiol (sulfhydryl group) reactive and thus can be conjugated to thiols (Figure 20 B).

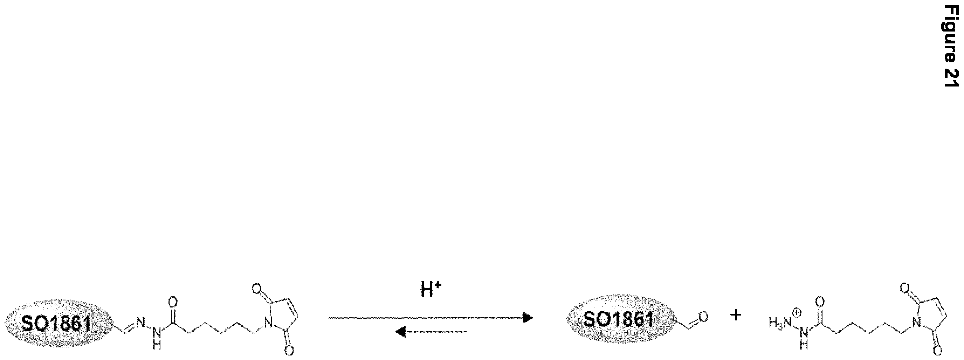

The maleimide group of SO1861-EMCH performs a rapid and specific Michael addition reaction with thiols and thiol bearing polymeric structures when carried out in a pH range of 6.5-7.5 (Figure 20 B). In addition, the acid sensitive hydrazone linkage between the SO1861 and EMCH can be utilized to perform saponin release from a scaffold in acidic environment (Figure 21). Thus, the EMCH linker fulfills both the need for a pH cleavable strategy and a conjugation strategy to a polymeric structure.

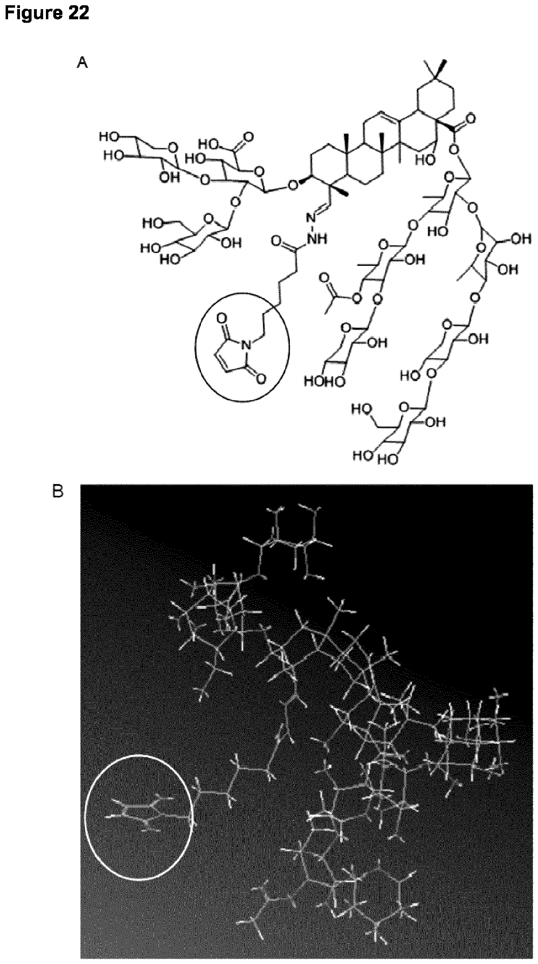

Regarding an ideal EMCH spacer length for conjugation to a polymeric structure, computer simulation (PerkinElmer, ChemBio3D, Ver. 13.0.0.3015) shows that the maleimide group on SO1861-EMCH is located at the periphery of the molecule and thus should be accessible for thiol bearing polymeric structures (Figure 22).

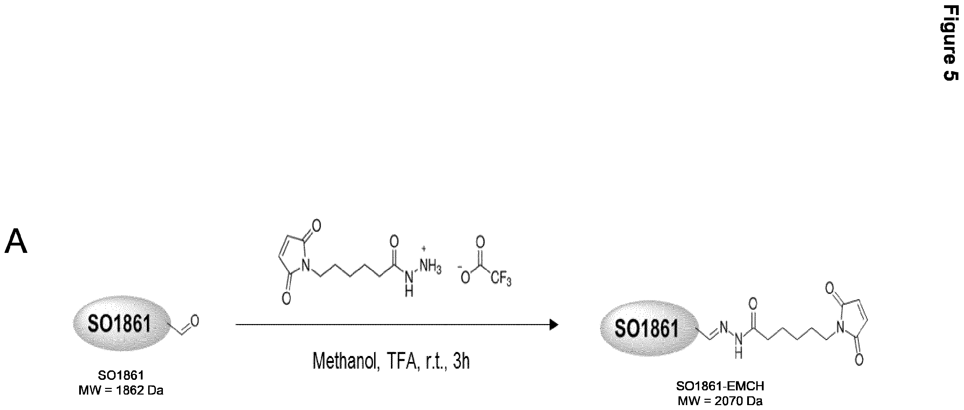

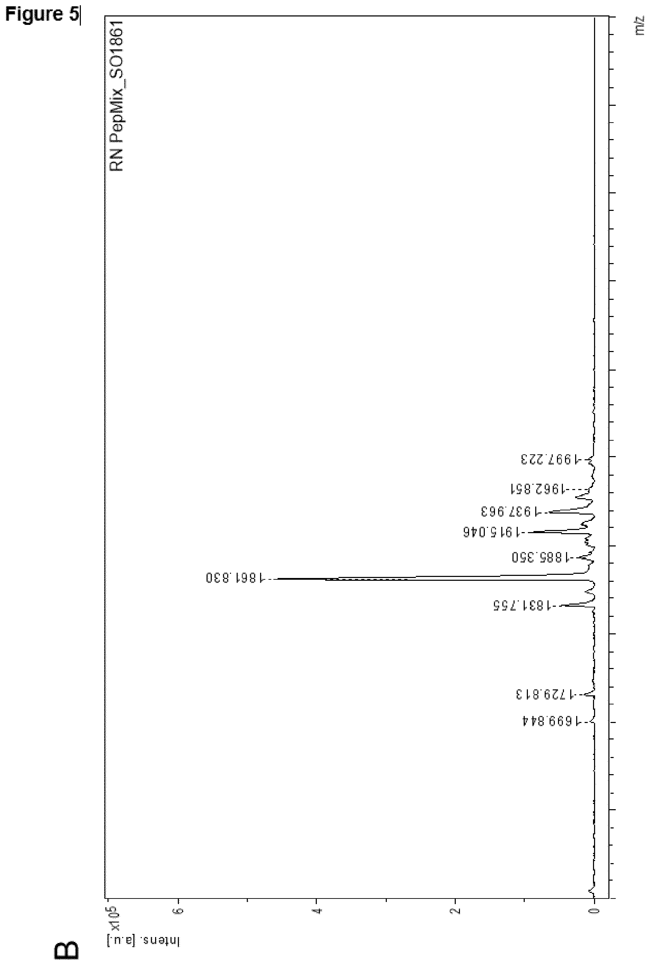

To synthesize the SO1861-EMCH, a strategy has been developed that allows the conversion of the aldehyde group on the SO1861 to EMCH (Figure 5 A). The SO1861-EMCH conjugate was isolated and successfully characterized via nuclear magnetic resonance spectroscopy (see materials and methods section, Figure 3 B) and matrix-assisted laser desorption/ionization time-of-flight mass spectrometry (MALDI-TOF-MS) as shown in Figure 5 B and 5 C, and Figure 4 A.

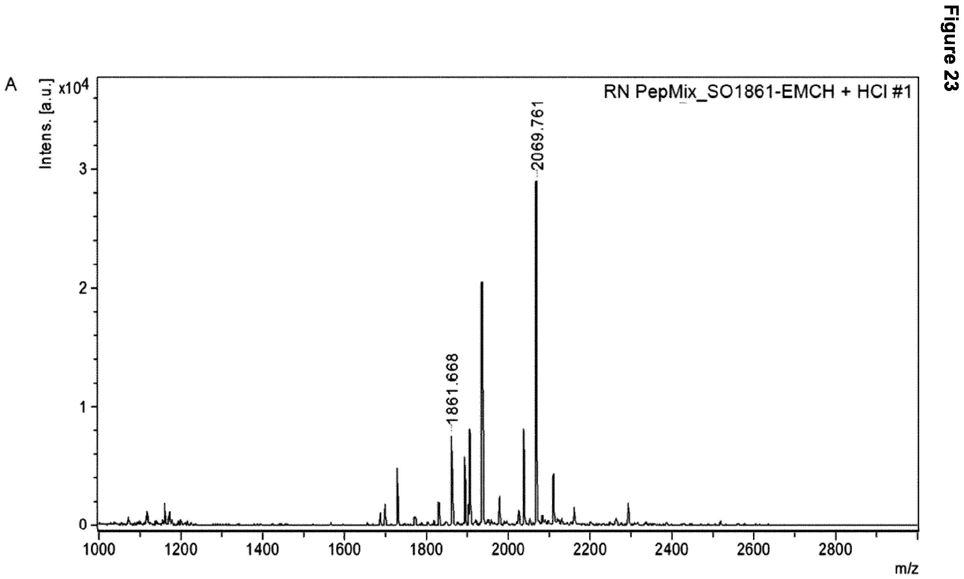

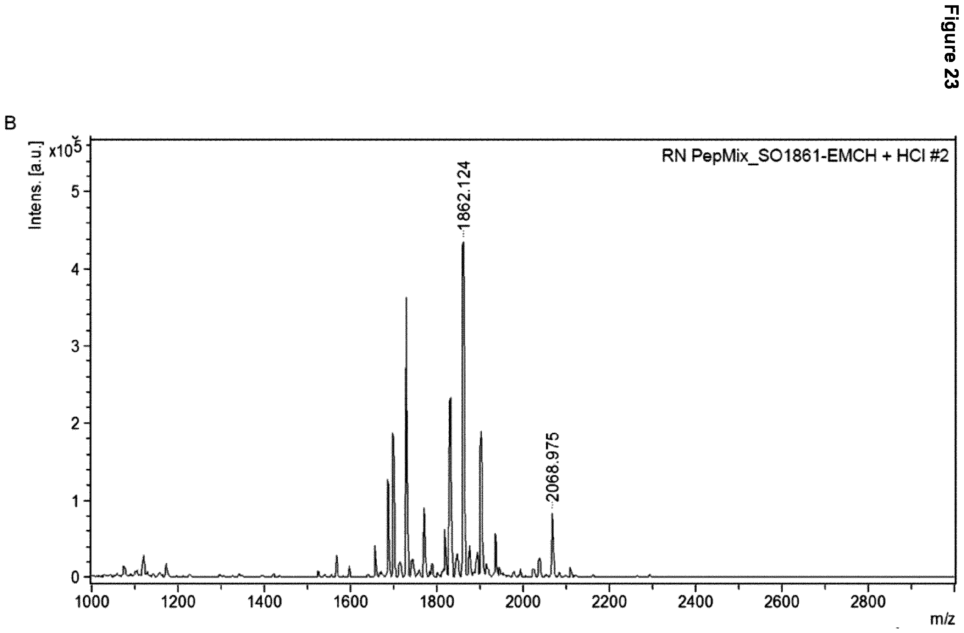

For testing the pH dependent hydrolysis of the hydrazone bond, SO1861-EMCH was dissolved in an HCl solution at pH 3 and MALDI-TOF-MS spectra were recorded at two different points in time (Figure 23). As shown in Figure 23 A and 23 B, a clear decreasing tendency of the peak at *m*/*z* 2070 Da that corresponds to SO1861-EMCH is visible in Figure 23 B. Since SO1861 is generated during hydrolysis, an increase of the peak at *m*/*z* 1861 Da was recorded that accompanied the decreasing tendency at *m*/*z* 2070 Da. These results show that the hydrazone bond is responsive towards hydrolysis and gets cleaved even if it is attached on SO1861.

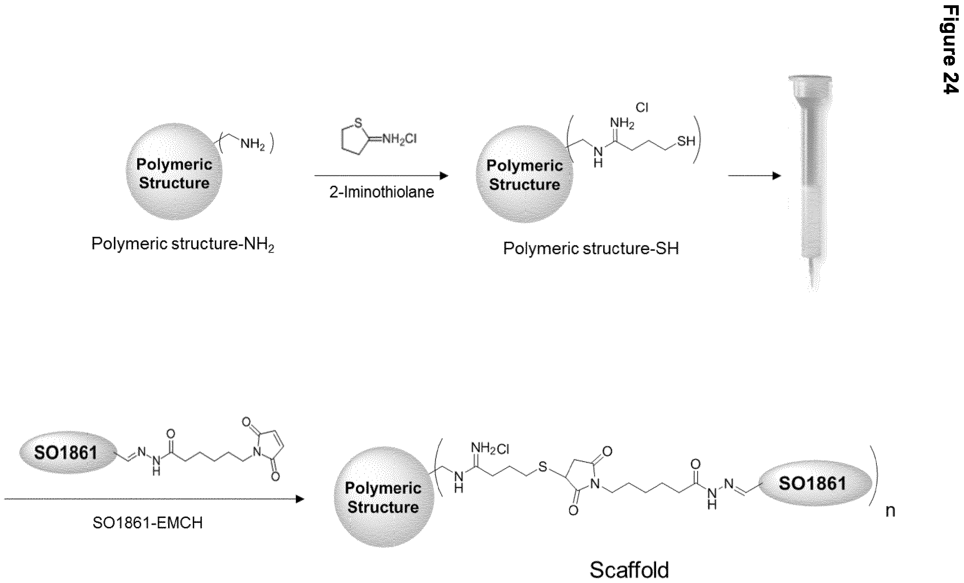

In order to conjugate SO1861-EMCH to a polymeric structure, accessible amines of the polymeric structure are converted into thiols with the aid of the agent 2-iminothiolane. The generated free thiols on the polymeric structure act then as the nucleophile for the thiol-ene Michael-type addition to the maleimide group of SO1861-EMCH (Figure 24). This developed methodology is suitable for the conjugation of SO1861 -EMCH to any available polymeric structure that obtains accessible amine groups and allows furthermore a control over the number of conjugated SO1861 molecules depending on the polymeric structure, respectively.

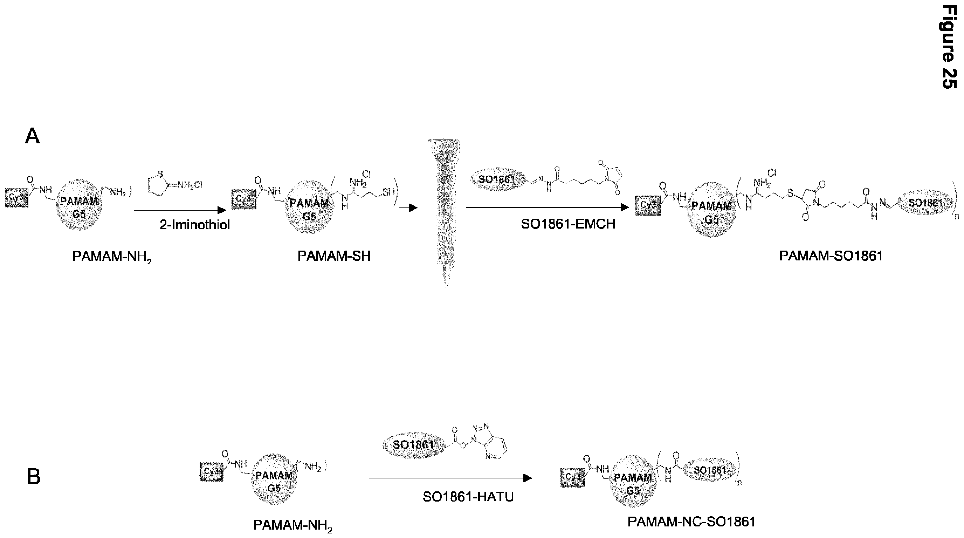

Proof of concept for conjugation of 'ready-to conjugate saponins' to a polymeric structure was obtained by the use of the amine bearing generation 5 (G5) dendrimer poly(amidoamine) (PAMAM with covalently coupled red-fluorescent dye (Cy3)). PAMAM-Cy3 was utilized as the polymeric structure for the conjugation to both SO1861-EMCH and SO1861-HATU and served as a model for conjugation of SO1861 to a polymeric structure (Figure 25).

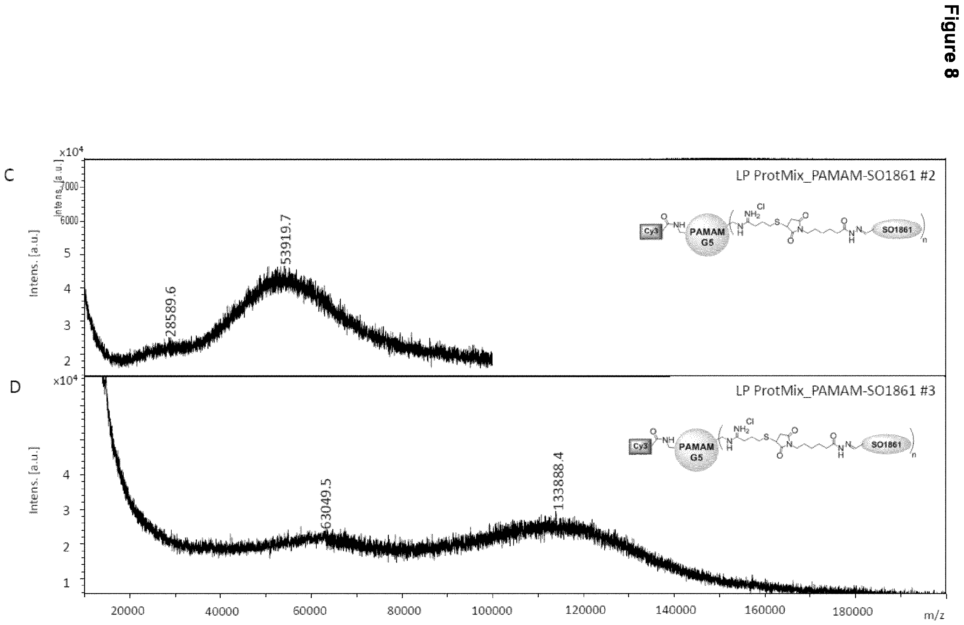

All accessible amine groups of Cy3-PAMAM were converted into thiols using a 3 fold excess of 2-iminothiolane per Cy3-PAMAM amines followed by the reaction with SO1861-EMCH. Three different feed equivalents (5, 20 and 57) of SO1861-EMCH were used for the three reaction batches. After reaction, the recorded masses of the Cy3-PAMAM-SO1861 conjugates at MALDI-TOF-MS show an increment of the corresponding masses with increasing the SO1861-EMCH feed (Figure 8). The three different feeds corresponded to an obtained mass of *m*/*z* 38.4 kDa, *m*/*z* 53.9 kDa and *m*/*z* 133.8 kDa for the Cy3-PAMAM-SO1861 conjugates that correspond to 6, 13 and 51 SO1861 molecules attached per PAMAM dendrimer as shown on Figure 8 B-D.

In another reaction, only a certain number of PAMAM amines were converted into thiols prior to reaction with SO1861-EMCH. Here, two different feed equivalents of 2-Iminothiolane (8 and 32) and two different feed equivalents of SO1861-EMCH (5 and 30) were used, respectively. After reaction, the respective spectra of the Cy3-PAMAM-SO1861 conjugates at MALDI-TOF-MS show peaks at *m*/*z* 37.7 kDa (5 feed equivalents of SO1861-EMCH) and at *m*/*z* 87.0 kDa (30 feed equivalents of SO1861-EMCH) as shown in Figure 9. The obtained masses at *m*/*z* 37.7 kDa and *m*/*z* 87.0 kDa correspond to Cy3-PAMAM-SO1861 conjugates with 5 and 30 SO1861 molecules attached and demonstrate that with this method almost all feed of SO1861-EMCH were conjugated.

For the generation of a non-pH-cleavable saponin conjugate the carboxylic acid of SO1861 was activated with HATU and then reacted with the amines of Cy3-PAMAM forming a pH stable amide bound between Cy3-PAMAM and SO1861. The resulting mass of the conjugate was detected via MALDI-TOF-MS with a mass of *m*/*z* 62.3 kDa that corresponds to Cy3-PAMAM-NC-SO1861 (NC = non-cleavable) conjugate with 17.5 SO1861 molecules attached per PAMAM (Figure 25 B, Figure 10).

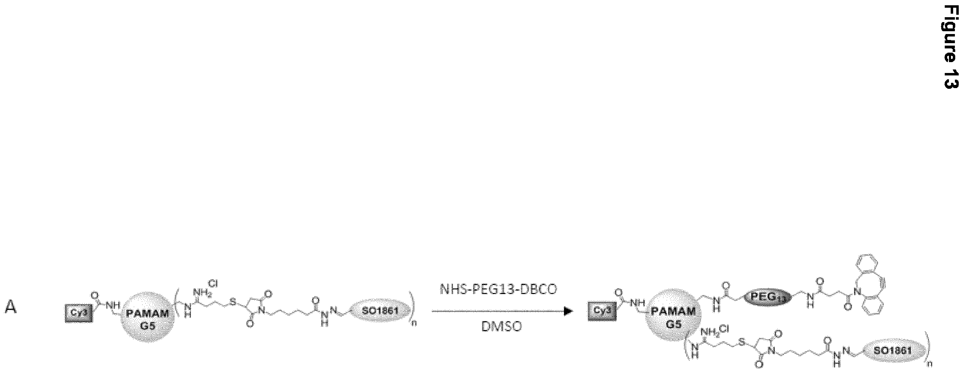

Next, the saponin conjugated scaffolds were conjugated to linking points for a possible conjugation to targeted therapeutics (e.g. targeted toxins) via the so-called strain-promoted alkyne-azide cycloaddition (SPAAC, click chemistry) to obtain a functionalized scaffold. For this reaction, Cy3-PAMAM-SO1861 (Figure 36 C, D) and Cy3-PAMAM-NC-SO1861 (Figure 13 B) were conjugated to a heterobifunctional NHS-PEG₁₃-DBCO linker that generated a strained alkyne on the conjugates' surface (Figure 13 A). The NHS (N Hydroxysuccinimide) moiety of the linker reacted with remaining amines of the PAMAM-saponin conjugates forming an amide bond between the scaffold and the linker. The resulting DBCO (dibenzocyclooctyne) moiety on the conjugates is able to perform SPAAC with corresponding azides on the targeted therapeutics.

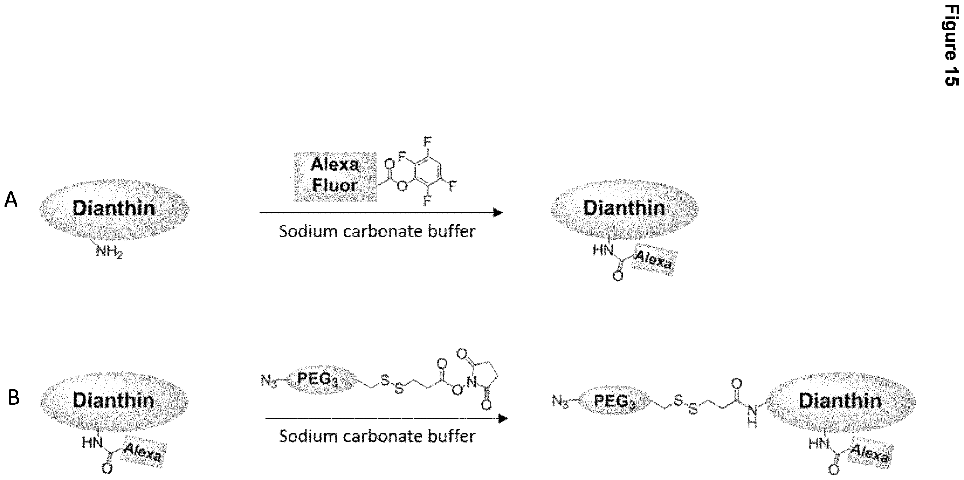

Dianthin-EGF served as a model targeted toxin and dianthin served as a non-targeted toxin. Both toxins were labeled with Alexa Fluor^{™} 488 using the tetrafluorophenyl ester (TFP) derivative of the dye. The dye labeled proteins were then conjugated to a heterobifunctional NHS-SS-PEG₃-azide linker to obtain the corresponding chemical moiety for the SPAAC to the PAMAM-saponin conjugates. Maldi-TOF-MS measurements showed that one Alexa Fluor^{™} 488 dye and 9 NHS-SS-PEG3-azide molecules were attached per dianthin-EGF molecule (Figure 14, Figure 15). Furthermore, Alexa Fluor^{™} 488 labeled dianthin-EGF was conjugated to a heterobifunctional NHS-PEG₁₂-azide linker that lacked the disulfide bond and would thus generate a non-toxin-cleavable construct.

The Cy3-PAMAM-NC-SO1861-DBCO and Cy3-PAMAM-SO1861-DBCO conjugates were reacted with Alexa Fluor^{™} 488 labeled azido-toxins to perform a strain-promoted alkyne-azide cycloaddition. The conjugation between the reacting agents was indicated via gel electrophoresis and the co-localization of the fluorescent signals of Cy3 that is only attached on the PAMAM polymer and Alexa Fluor^{™} 488 that is only attached on the toxins on a polyacrylamide gel after gel electrophoresis (Figure 16).

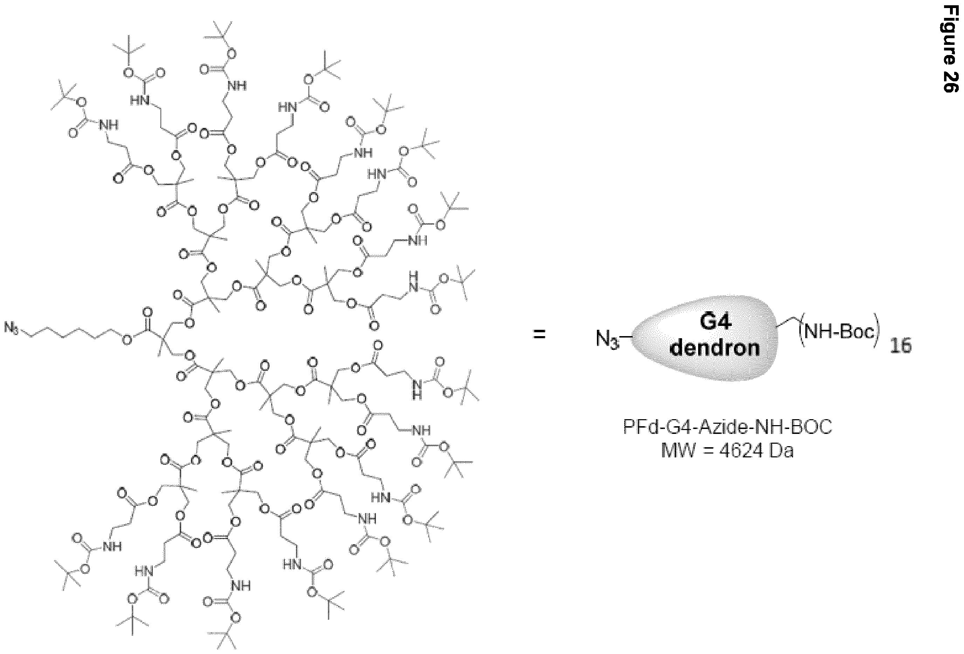

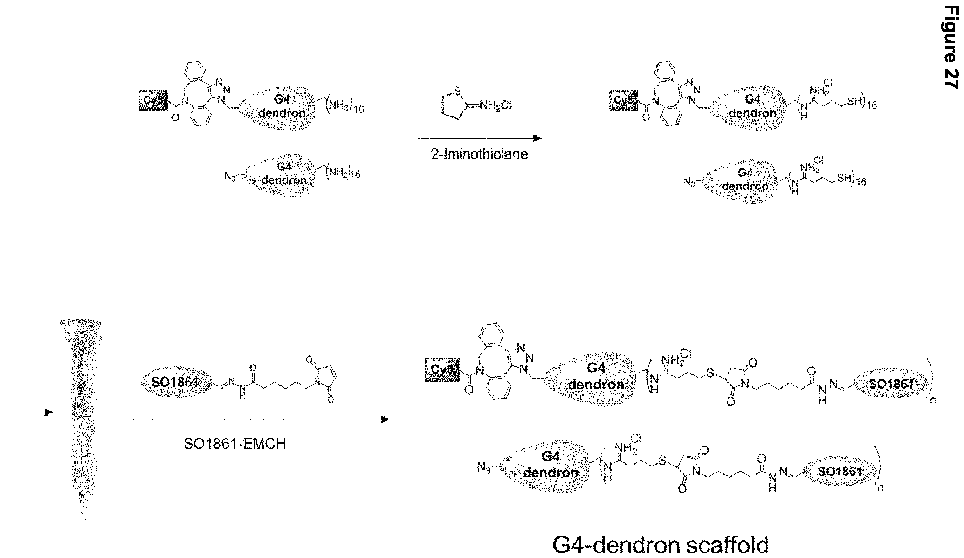

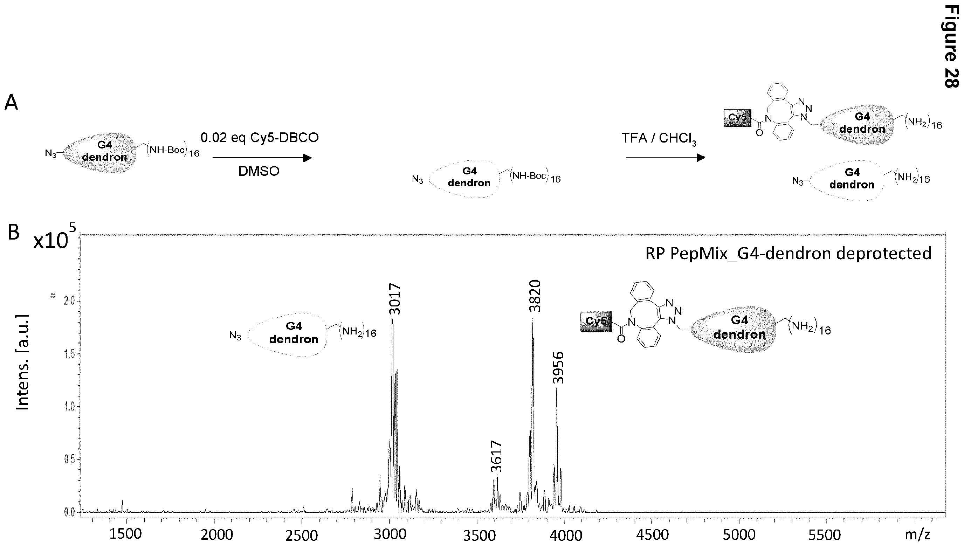

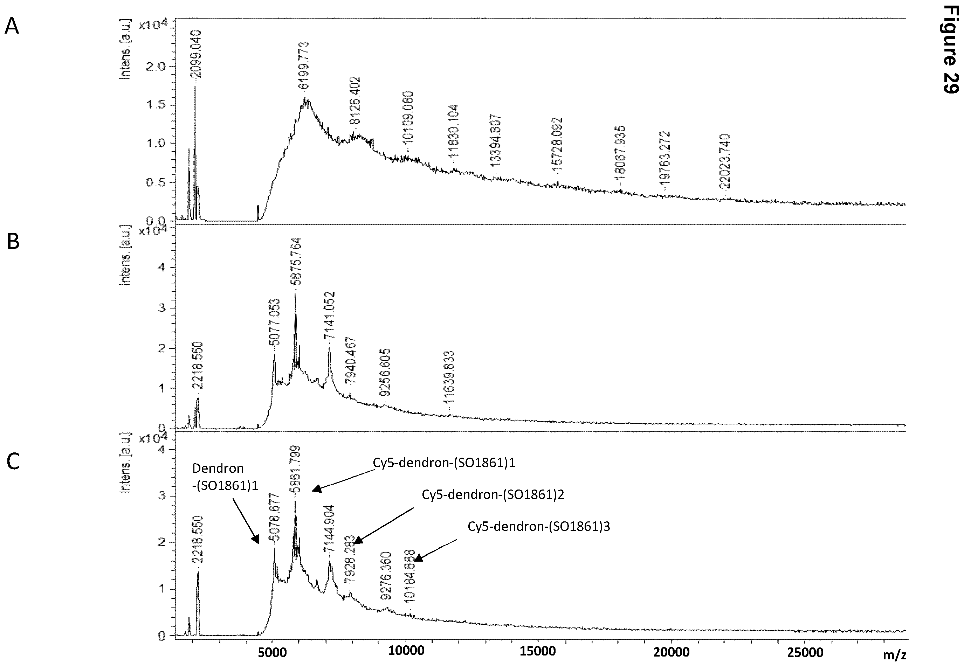

As an alternative polymeric structure to the PAMAM dendrimer, a G4-dendron (PFd-G4-Azide-NH-BOC, Polymer Factory) with 16 functional amino end groups and an azido group at the focal point was utilized for the conjugation to SO1861 (Figure 26). The advantage of using a dendron over a dendrimer is the focal point that the dendron structure is exhibiting. This focal point allows the direct conjugation to a targeted toxin without the need of its post-modification with orthogonal click functions (Figure 27). As shown in Figure 27, the dendron underwent the same methodology as described for the PAMAM dendrimer. Briefly, after partial dye labeling and deprotection (Figure 28), the amino groups of the dendron were converted into thiols using the thiolating reagent 2-iminothiolane followed by conjugation to SO1861-EMCH. For the conjugation to SO1861-EMCH three different feed equivalents of SO1861-EMCH were used. The dendron-SO1861 conjugates were analyzed via MALDI-TOF-MS. As expected, the conjugate species of 1 and 2 SO1861 molecules per dendron molecule were obtained when low SO1861-EMCH feed equivalents of 3 and 10 were used (Figure 29 B, C). Higher dendron-SO1861 conjugate species of up to 9 SO1861 molecules per dendron were obtained (Figure 29 A) when using a feed equivalent of 22 SO1861-EMCH molecules per dendron molecule. In further experiments, the saponin functionalized dendron will be conjugated to targeted toxins over its focal point to yield a functionalized scaffold and will be evaluated biologically.

To further test other conjugation methodologies of SO1861 to a polymeric structure, the reductive amination pathway was used. For this, the aldehyde group of SO1861 was directly conjugated to PAMAM amines forming an imine bound. The imine bond formation was followed a reductive amination step through the addition of the reductive agent sodium cyanoborohydride forming a pH-stable amine bond between SO1861 and PAMAM (Figure 17 A). Similar to the EMCH and HATU approach, this methodology allows a control over the number of conjugated saponins per polymer as shown on Figure 17 B, C. Here, PAMAM-saponin conjugates were produced which obtained a number of 10 (Figure 17 B) and 30 (Figure 17 C) SO1861 molecules per PAMAM.

### Example 4

### Cell viability assay

HeLa cells were seeded in DMEM (PAN-Biotech GmbH) supplemented with 10% fetal bovine serum (PAN-Biotech GmbH) and 1% penicillin/streptomycin (PAN-Biotech GmbH), in a 96 well plate at 5,000 c/w in 100 µL/well and incubated overnight at 37 °C and 5% CO₂. The next day 20x concentrated stocks of the PAMAM, PAMAM-conjugates, G4-dendron (prepared in Example 3) or chloroquine (Sigma Aldrich) samples were prepared in DMEM. The media was removed from the cell culture plate and replaced by 160 µL culture media, followed by the addition of 10 µL sample/well (from the 20x concentrated stocks) and a 45 min incubation at 37 °C. During this incubation the SO1861 concentration curve was prepared. The SO1861 master stock was heated for 10 min at 50 °C, while shaking at 1,250 rpm. Followed by 15 sec sonication and a brief re-heating at 50 °C for 1 min, while shaking at 1,250 rpm. Subsequently a serial dilution of SO1861 was prepared in PBS. The SO1861 concentration curve was prepared as 10x concentrated stock, from which 20 µL was added/well. After a 15 min incubation at 37 °C, 10 µL dianthin-EGF (prepared in Example 2) diluted in DMEM to 30 pM) or DMEM containing an equal amount of PBS was added/well, to obtain a final dianthin-EGF concentration of 1.5 pM as well as the indicated SO1861 and the different polymeric structures or chloroquine concentrations in a final volume of 200 µL/well.

After treatment the cells were incubated for 72 hr at 37 °C before the cell viability was determined by a MTS-assay, performed according to the manufacturer's instruction (CellTiter 96^{®} AQueous One Solution Cell Proliferation Assay, Promega). Briefly, the MTS solution was diluted 20× in DMEM without phenol red (PAN-Biotech GmbH) supplemented with 10% FBS. The cells were washed once with 200 µL/PBS well, after which 100 µL diluted MTS solution was added/well. The plate was incubated for approximately 20-30 minutes at 37 °C. Subsequently, the OD at 492 nm was measured on a Thermo Scientific Multiskan FC plate reader (Thermo Scientific). For quantification the background signal of 'medium only' wells was subtracted from all other wells, before the cell viability percentage of treated/untreated cells was calculated, by dividing the background corrected signal of treated wells over the background corrected signal of the untreated wells (x 100).

### FACS analysis

HeLa cells were seeded in DMEM (PAN-Biotech GmbH) supplemented with 10% fetal calf serum (PAN-Biotech GmbH) and 1% penicillin/streptomycin (PAN-Biotech GmbH), at 500,000 c/plate in 10 cm dishes and incubated for 48 hrs (5% CO₂, 37 °C), until a confluency of 90% was reached. Next, the cells were trypsinized (TryplE Express, Gibco Thermo Scientific) to single cells. 0.75 × 10⁶ Cells were transferred to a 15 mL falcon tube and centrifuged (1,400 rpm, 3 min). The supernatant was discarded while leaving the cell pellet submerged. The pellet was dissociated by gentle tapping the falcon tube on a vortex shaker and the cells were washed with 4 mL cold PBS (Mg²⁺ and Ca²⁺ free, 2% FBS). After washing the cells were resuspended in 3 mL cold PBS (Mg²⁺ and Ca²⁺ free, 2% FBS) and divided equally over 3 round bottom FACS tubes (1 mL/tube). The cells were centrifuged again and resuspended in 200 µL cold PBS (Mg²⁺ and Ca²⁺ free, 2% FBS) or 200 µL antibody solution; containing 5 µL antibody in 195 µL cold PBS (Mg²⁺ and Ca²⁺ free, 2% FBS). APC Mouse IgG1, κ Isotype Ctrl FC (#400122, Biolegend) was used as isotype control, and APC anti-human EGFR (#352906, Biolegend) was used to stain the EGFR receptor. Samples were incubated for 30 min at 4 °C on a tube roller mixer. Afterwards, the cells were washed 3x with cold PBS (Mg²⁺ and Ca²⁺ free, 2% FBS) and fixated for 20 min at room temperature using a 2% PFA solution in PBS. Cells were washed 2x with cold PBS, and resuspended in 250-350 µL cold PBS for FACS analysis. Samples were analyzed with a BD FACSCanto II flow cytometry system (BD Biosciences) and FlowJo software.

### Results

Previously it has been described that the amino groups in amine containing polymeric structures such as PAMAM and PEI (polyethylenimine) are able to block the acidification of the endosomes via the their intrinsic H⁺ buffering capacity. Since the endosomal escape enhancing properties of SO1861 are only exposed at low endosomal pH (< pH5), the scaffold or functionalized scaffold should not contain chemical groups that are able to interfere in acidification of the endosomes and thus block the activity of SO1861.

The amine containing polymeric structures of a G5 PAMAM (128 primary amines as well as approximately 126 tertiary amines) and G4-dendron (16 primary amines) were tested, in order to determine if these molecules inhibit the endosomal escape enhancing capacity of SO1861. Coadministration experiments of PAMAM (native or thiolated) or dendron (native) in combination with dianthin-EGF and various SO1861 concentrations on HeLa cells were performed. As control for the inhibition of endosomal acidification chloroquine was used.

HeLa cells show sufficient EGFR cell surface levels (Figure 30 A). It is observed that both 'native' PAMAM and chloroquine inhibit the SO1861-mediated endosomal escape of the targeted toxin and subsequent cell killing in Hela cells (Figure 30 B). PAMAM at 500 nM inhibits even to an equal extent as the endosomal acidification inhibitor chloroquine, while 667 nM dendron has no effect at all. To further address if the inhibitory activity of the 'native' PAMAM is due to the availability of amino groups in PAMAM, the primary amino groups of PAMAM were partially thiolated through reaction with 2-iminothiolane (Figure 11), resulting in 16 of 128 (Figure 11 C), 65/128 (Figure 11 D), and 108/128 (Figure 11 E) blocked primary amines. It is observed that thiolation of 65 and 108 primary amines overcomes the inhibition of SO1861-mediated endosomal escape, whereas thiolation of up to 16 amines groups still shows the inhibitory effects of SO1861-mediated endosomal escape of the targeted toxin (Figure 30 C). The primary amino groups of PAMAM were also partially PEGylated through a reaction with mPEG2k-NHS (Figure 12), resulting in 3 of 128 (Figure 12 C), 8/128 (Figure 12 D), and 18/128 (Figure 12 E) blocked primary amines. Blocking only 3 primary amines by PEGylation is already sufficient to reverse the inhibition of SO1861-mediated endosomal escape (Figure 11 D). The shielding effect of PEGylation most likely extends beyond the small number of amines that are converted, as PEGylation is known to introduce a hydration layer that can shield off an entire molecule, if a sufficient level is reached.

These results demonstrate that the presence of a certain number of free amino groups on polymeric structures, such as PAMAM, can block endosomal acidification and thus inhibit the endosomal escape activity of SO1861 or other glycosides. When the number of amino groups is lower, as shown for the G4-dendron, or if the amino groups have been shielded, such as thiolation or PEGylation, the inhibitory effect is reversed.

### EXAMPLE A - saponins mixture of Quillaja saponaria comprising QS-21, with endosomal/lysosomal escape enhancing activity

Scheme I displays the common molecular structure of a series of QS-21 saponins (in part adapted from: Conrado Pedebos, Laércio Pol-Fachin, Ramon Pons, Cilaine V. Teixeira Hugo Verli, Atomic Model and Micelle Dynamics of QS-21 Saponin, Molecules 2014, 19, 3744-3760). A mixture of water-soluble saponins obtained from Quillaja saponaria (Sigma-Aldrich, product No. S4521; Roth, Item No. 6857; InvivoGen, product 'Quil-A') may be applied in the effector moiety comprising at least one saponin according to the invention, or in an endosomal/lysosomal escape enhancing conjugate, composition, combination of the invention, based on endosomal/lysosomal escape enhancing properties of at least one individual saponin present in the mixture, e.g. QS-21, or based on a combination of two or more of the saponins comprised by the mixture, such as QS-21 and QS-7.

The inventors demonstrated that the mixture of saponins from Quillaja saponaria at 2,5 microgram/ml dose was capable of enhancing endosomal escape of dianthin, when tested with mammalian tumor cells in a cell-based bioassay. The effector moiety exposed to the cells was dianthin covalently coupled to the ligand EGF: EGF-dianthin. Cells tested were tumor cell lines HeLa for free saponins, and A431, MDA-MB-468, CaSki and A2058 for testing the saponins of Quillaja saponaria when covalently coupled to cetuximab.

### REFERENCES

Weng, A.; Thakur, M.; Beceren-Braun, F.; Bachran, D.; Bachran, C.; Riese, S.B.; Jenett-Siems, K.; Gilabert-Oriol, R.; Melzig, M.F.; Fuchs, H. The toxin component of targeted anti-tumor toxins determines their efficacy increase by saponins. Molecular oncology 2012, 6, 323-332.
saponinum album in a synergistic way. Journal of immunotherapy 2009, 32, 713-725.

Sama, S.; Jerz, G.; Schmieder, P.; Joseph, J.F.; Melzig, M.F.; Weng, A. Plant derived triterpenes from Gypsophila elegans M.Bieb. enable non-toxic delivery of gene loaded nanoplexes. Journal of Biotechnology 2018, 284, 131-139

Kolb, H.C.; Finn, M.G.; Sharpless, K.B. Click Chemistry: Diverse Chemical Function from a Few Good Reactions. Angewandte Chemie 2001, 40, 2004-2021.

Bird, R.E.; Hardmann, K.D.; Jacobson, J.W.; Johnson, S.; Kaufman, B.M.; Lee, S.M.; Lee, T.; Pope, S.H.; Riordan, G.S.; Whitlow, M. Single-chain antigen-binding proteins. Science 1988, 242, 423-426.

Y Zhang, Z Qu, S Kim, V Shi, B Liao1, P Kraft, R Bandaru, Y Wu, LM Greenberger and ID Horak, Down-modulation of cancer targets using locked nucleic acid (LNA)-based antisense oligonucleotides without transfection, Gene Therapy (2011) 18, 326-333

## Claims

1. An effector moiety capable of inducing an intracellular effect when present inside a mammalian cell, the effector moiety conjugated with at least one saponin, wherein the at least one saponin is covalently bound to the effector moiety via at least one linker, or is covalently bound directly to said effector moiety.

2. The effector moiety of claim 1, comprising or consisting of at least one oligonucleotide, a nucleic acid, a xeno nucleic acid, preferably selected from any one or more of a vector, a gene, a cell suicide inducing transgene, deoxyribonucleic acid (DNA), ribonucleic acid (RNA), anti-sense oligonucleotide (ASO, AON), short interfering RNA (siRNA), microRNA (miRNA), DNA aptamer, RNA aptamer, mRNA, mini-circle DNA, peptide nucleic acid (PNA), phosphoramidate morpholino oligomer (PMO), locked nucleic acid (LNA), bridged nucleic acid (BNA), 2'-deoxy-2'-fluoroarabino nucleic acid (FANA), 2'-O-methoxyethyl-RNA (MOE), 2'-O,4'-aminoethylene bridged nucleic acid, 3'-fluoro hexitol nucleic acid (FHNA), a plasmid, glycol nucleic acid (GNA) and threose nucleic acid (TNA), or a derivative thereof, more preferably a BNA, for example a BNA for silencing HSP27 protein expression.

3. The effector moiety of claim 1 or 2, wherein the at least one saponin is a triterpenoid saponin and/or a bisdesmosidic triterpene saponin and optionally comprising a glucuronic acid function in a carbohydrate substituent at the C-3beta-OH group of the saponin, and/or a saponin isolated from a *Gypsophila* species and/or a *Saponaria* species and/or an *Agrostemma* species and/or a *Quillaja* species such as *Quillaja saponaria.*

4. The effector moiety of any one of the claims 1-3, wherein the at least one saponin is a single specific saponin or is a mixture of two or more different saponins.

5. The effector moiety of any one of the claims 1-4, wherein the saponin is one or more of the saponins in Table A1 or Scheme I, NP-005236, AMA-1, AMR, alpha-Hederin, NP-012672, NP-017777, NP-017778, NP-017774, NP-018110, NP-017772, NP-018109, NP-017888, NP-017889, NP-018108, NP-017674, NP-017810, NP-003881, NP-017676, NP-017677, NP-017706, NP-017705, NP-017773, NP-017775, SO1861, SA1657, GE1741, SA1641, AE X55, QS-21, QS-21A, QS-21 A-api, QS-21 A-xyl, QS-21B, QS-21 B-api, QS-21 B-xyl, QS-7-xyl, QS-7-api, QS-17, QS-17-api, QS-17-xyl, QS1861, QS1862, Quillajasaponin, Saponinum album, QS-18, Quil-A, Gyp1, gypsoside A, AG1, AG2, SO1542, SO1584, SO1658, SO1674, SO1832, beta-Aescin, Teaseed saponin I, Teaseedsaponin J, Assamsaponin F, Digitonin, Primula acid 1, AS64R, AS6.2 or any of their stereomers and/or any combinations thereof, preferably the saponin is SO1861 and/or GE1741 and/or SA1641 and/or QS-21 and/or saponin with a quillaic acid aglycon core, a Gal-(1→2)-[Xyl-(1→3)]-GlcA carbohydrate substituent at the C-3beta-OH group and a Glc-(1→3)-Xyl-(1→4)-Rha-(1→2)-[Xyl-(1→3)-4-OAc-Qui-(1→4)]-Fuc carbohydrate substituent at the C-28-OH group, and/or is 3-O-beta-D-galactopyranosyl-(1→2)-[beta-D-xylopyranosyl-(1→3)]-beta-D-glucuronopyranosyl quillaic acid 28-O-beta-D-glucopyranosyl-(1→3)-beta-D-xylopyranosyl-(1→4)- alpha-L-rhamnopyranosyl-(1→2)-[beta-D-xylopyranosyl-(1→3)-4-OAc-beta-D-quinovopyranosyl-(1→4)]-beta-D-fucopyranoside,
more preferably the saponin is SO1861 and/or QS-21.

6. The effector moiety of any one of the claims 1-5, wherein the saponin is a bisdesmosidic saponin having a molecular mass of at least 1.500 Dalton and comprising optionally a hydroxyl group at the C-16 position, with a first branched carbohydrate side chain at the C-3 position which first branched carbohydrate side chain optionally contains glucuronic acid,
wherein the saponin contains an ester group with a second branched carbohydrate side chain at the C-28 position which second branched carbohydrate chain preferably comprises at least four carbohydrate units and optionally contains at least one acetyl residue such as two acetyl residues and/or optionally comprises one or more deoxy carbohydrates and/or quinovose and/or glucose and/or 4-methoxycinnamic acid and/or optionally comprising 5-O-[5-O-Ara/Api-3,5-dihydroxy-6-methyl-octanoyl]-3,5-dihydroxy-6-methyl-octanoic acid and/or optionally comprising 5-O-[5-O-Rha-(1→2)-Ara/Api-3,5-dihydroxy-6-methyl-octanoyl]-3,5-dihydroxy-6-methyl-octanoic acid bound to a carbohydrate via an ester bond, or wherein the at least one saponin is QS-21 or any one or more of QS-21A, QS-21 A-api, QS-21 A-xyl, QS-21B, QS-21 B-api, QS-21 B-xyl, QS-7-xyl, QS-7-api, QS-17-api, QS-17-xyl, QS-18, QS1861, protonated QS1861 (QS1862), Quil-A.

7. The effector moiety of any one of the claims 1-6, wherein the at least one saponin is a bisdesmosidic triterpene saponin and comprising a glucuronic acid function in a carbohydrate substituent at the C-3beta-OH group of the saponin, wherein the at least one saponin is covalently coupled to an amino-acid residue, when present, of the effector moiety via the glucuronic acid function in the carbohydrate substituent at the C-3beta-OH group of the saponin, preferably via at least one linker, wherein the amino-acid residue preferably is selected from cysteine and lysine.

8. The effector moiety of any one of the claims 1-7, wherein the at least one linker comprises at least one non-cleavable linker and/or at least one cleavable linker, wherein optionally said cleavable linker is subject to cleavage under acidic, reductive, enzymatic or light-induced conditions, and preferably the cleavable linker comprises a cleavable bond selected from a hydrazide bond subject to cleavage under acidic conditions, and/or a bond susceptible to proteolysis, for example proteolysis by Cathepsin B, and/or a bond susceptible for cleavage under reductive conditions such as a disulphide bond.

9. The effector moiety of any one of the claims 1-8, wherein the at least one linker comprises at least one cleavable linker which is subject to cleavage *in vivo* under acidic conditions as present in endosomes and/or in lysosomes of mammalian cells, preferably of human cells, preferably at pH 4.0 - 6.5, and more preferably at pH ≤ 5.5.

10. The effector moiety of any one of the claims 1-9, wherein the at least one saponin is covalently bound to a lysine side chain, forming an amide bond, and/or to a cysteine side chain, forming a thio-ether linkage, or a disulphide bond, wherein the lysine and/or cysteine is/are comprised by the effector moiety, and wherein the saponin is bound either directly to the effector moiety, or bound via at least one linker.

11. The effector moiety of any one of the claims 1-10, wherein the at least one saponin is covalently bound to the effector moiety via at least one linker, wherein the linker is or comprises a scaffold comprising a polymeric or oligomeric structure and a chemical group for covalently coupling of the scaffold to the effector moiety,
wherein preferably the at least one saponin is covalently bound to the polymeric or oligomeric structure of the scaffold via a cleavable bond,
wherein more preferably the cleavable bond is subject to cleavage under any of acidic conditions, reductive conditions, enzymatic conditions and light-induced conditions, more preferably the cleavable bond is a hydrazide bond subject to cleavage under acidic conditions, and/or is a bond susceptible to proteolysis, for example proteolysis by Cathepsin B, and/or is a bond susceptible for cleavage under reductive conditions such as a disulphide bond,
wherein more preferably the cleavable bond is subject to cleavage *in vivo* under acidic conditions as present in endosomes and/or in lysosomes of mammalian cells, preferably of human cells, preferably at pH 4.0 - 6.5, and more preferably at pH ≤ 5.5, wherein preferably the at least one saponin is covalently bound to the polymeric or oligomeric structure of the scaffold via an imine bond, a hydrazide bond, an oxime bond, a 1,3-dioxolane bond, a disulphide bond, a thio-ether bond, an amide bond, a peptide bond and/or an ester bond, preferably via at least one linker, preferably an amide bond, a hydrazide bond, a thio-ether bond,
wherein preferably the glucuronic acid function in the carbohydrate substituent at the C-3beta-OH group of the at least one saponin is covalently coupled to linker 1-[Bis(dimethylamino)methylene]-1H-1,2,3-triazolo[4,5-b]pyridinium 3-oxid hexafluorophosphate, which linker is covalently coupled via an amide bond to an amine group in the polymeric or oligomeric structure of the scaffold, such as an amine group of a lysine or an N-terminus of the polymeric or oligomeric structure of the scaffold,
wherein preferably the chemical group of the scaffold, for covalently coupling of the scaffold to the effector moiety, is a click chemistry group, preferably selected from a tetrazine, an azide, an alkene or an alkyne, or a cyclic derivative of these groups, more preferably the click chemistry group is an azide, wherein preferably the polymeric or oligomeric structure of the scaffold comprises a linear, branched and/or cyclic polymer, oligomer, dendrimer, dendron, dendronized polymer, dendronized oligomer, a DNA, a polypeptide, poly-lysine, a poly-ethylene glycol, or an assembly of these polymeric or oligomeric structures which assembly is preferably built up by covalent cross-linking.

12. The effector moiety according to any one of the claims 1-11, wherein the at least one saponin is a defined number of saponins or a defined range of saponins, preferably 1-128 saponins or at least 2, 3, 4, 5, 6, 8, 10, 16, 32, 64 or 128 saponins, or any number of saponins therein between, such as 7, 9, 12 saponins.

13. An antibody-drug conjugate comprising the effector moiety of any one of the claims 1-12, or a ligand-drug conjugate comprising the effector moiety of any one of the claims 1-12, wherein preferably the antibody can bind to any one of CD71, CA125, EpCAM(17-1A), CD52, CEA, CD44v6, FAP, EGF-IR, integrin, syndecan-1, vascular integrin alpha-V beta-3, HER2, EGFR, CD20, CD22, Folate receptor 1, CD146, CD56, CD19, CD138, CD27L receptor, PSMA, CanAg, integrin-alphaV, CA6, CD33, mesothelin, Cripto, CD3, CD30, CD239, CD70, CD123, CD352, DLL3, CD25, ephrinA4, MUC1, Trop2, CEACAM5, CEACAM6, HER3, CD74, PTK7, Notch3, FGF2, C4.4A, FLT3, CD38, FGFR3, CD7, PD-L1, CTLA4, CD52, PDGFRA, VEGFR1, VEGFR2, preferably CD71, HER2, EGFR, and/or wherein the antibody of the antibody-drug conjugate is or comprises any one of cetuximab, daratumumab, gemtuzumab, trastuzumab, panitumumab, brentuximab, inotuzumab, moxetumomab, polatuzumab, obinutuzumab, OKT-9 anti-CD71 monoclonal antibody of the IgG type, pertuzumab, rituximab, ofatumumab, Herceptin, alemtuzumab, pinatuzumab, OKT-10 anti-CD38 monoclonal antibody, an antibody of Table A2 or Table A3 or Table A4, preferably cetuximab or trastuzumab or OKT-9, or at least one tumor-cell specific receptor binding-fragment thereof and/or at least one tumor-cell specific receptor binding-domain thereof, and/or wherein the antibody-drug conjugate comprises any one of Gemtuzumab ozogamicin, Brentuximab vedotin, Trastuzumab emtansine, Inotuzumab ozogamicin, Moxetumomab pasudotox and Polatuzumab vedotin and an antibody-drug conjugate of Table A2 and Table A3, and/or wherein the ligand-drug conjugate comprises or consists of at least one non-proteinaceous ligand and/or at least one proteinaceous ligand for binding to a cell-surface molecule such as EGF or a cytokine.

14. Therapeutic combination comprising:
(a) the effector moiety of any one of the claims 1-12 and optionally a pharmaceutically acceptable excipient; and
(b) an antibody-drug conjugate or a ligand-drug conjugate, and optionally a pharmaceutically acceptable excipient.

15. Pharmaceutical composition comprising the effector moiety of any one of the claims 1-12 or the antibody-drug conjugate of claim 13 or the ligand-drug conjugate of claim 13, and optionally a pharmaceutically acceptable excipient.

16. The effector moiety of any one of the claims 1-12 or the antibody-drug conjugate of claim 13 or the therapeutic combination of claim 14 or the ligand-drug conjugate of claim 13 or the pharmaceutical composition of claim 15, for use as a medicament.

17. The effector moiety of any one of the claims 1-12 or the antibody-drug conjugate of claim 13 or the ligand-drug conjugate of 13 or the therapeutic combination of claim 14 or the pharmaceutical composition of claim 15, for use in the treatment or prevention of a cancer or an autoimmune disease.
